(19) Europäisches Patentamt
European Patent Office
Office européen des brevets

(11) Publication number: **0 180 615 B1**

# (12) EUROPEAN PATENT SPECIFICATION

(45) Date of publication of patent specification: **05.04.95**

(51) Int. Cl.[6]: **C07K 7/00**, C07K 14/00, C12N 1/00, C12N 1/20, C12N 15/00, A61K 38/00

(21) Application number: **85902274.1**

(22) Date of filing: **16.04.85**

(86) International application number: **PCT/US85/00658**

(87) International publication number: **WO 85/04870 (07.11.85 85/24)**

## (54) NOVEL ATRIAL NATRIURETIC/VASODILATOR POLYPEPTIDES.

(30) Priority: **19.04.84 US 602117**
**01.06.84 US 616488**
**20.06.84 US 622639**

(43) Date of publication of application: **14.05.86 Bulletin 86/20**

(45) Publication of the grant of the patent: **05.04.95 Bulletin 95/14**

(84) Designated Contracting States: **AT BE CH DE FR GB IT LI LU NL SE**

(56) References cited:
**EP-A- 0 116 784**
**EP-A- 0 142 487**

**BIOCHEMICAL AND BIOPHYSICAL RESEARCH COMMUNICATIONS, vol. 117, no. 3 28 December 1983, pages 859-865. Academic Press, New York, US; T. GEOFREY FLYNN et al.: "The amino acid sequence of an atrial peptide with potent diuretic and natriuretic properties"**

(73) Proprietor: **SCIOS NOVA INC.**
**2450 Bayshore Parkway**
**Mountain View, CA 94043 (US)**

(72) Inventor: **JOHNSON, Lorin, K.**
**4979 Dolores Drive**
**Pleasanton, CA 94566 (US)**
Inventor: **ATLAS, Steven, A.**
**1161 York Avenue**
**New York, NY 10021 (US)**
Inventor: **McCARTHY, Brian, J.**
**14244 Amherst Court**
**Los Altos Hills, CA 94022 (US)**
Inventor: **LARAGH, John, H.**
**435 East 70th Street, Apt. 22J**
**New York, NY 10021 (US)**
Inventor: **LEWICKI, John, A.**
**954 Michaelangelo Drive**
**Sunnyvale, CA 94087 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 180 615 B1

**BIOLOGICAL ABSTRACTS, vol. 78, 1984, abstract no. 58551, Philadelphia, US; M. CANTIN et al.: "Immunocyto-technical localization of atrial natriuretic factor in the heart and salivary glands"**

**Biochemical and Biophysical Research Communications, 13 January 1984, Kangawa et al., "Purification and Complete Amino Acid Sequence Alpha- Human Atrial Natriuretic Polypeptide" vol. 118 (1) pages 131-139**

**Biochemical and Biophysical Research Communications, 30 March 1984, Kangawa et al., "Purification and Complete Amino Acid Sequence of Beta-Rat Atrial Natriuretic Polypeptide ( -rANP) of 5,000 Daltons" Vol. 119 (3) pages 933-940**

**Biochemical and Biophysical Research Communications, 14 November 1984, Nakao et al "Radio-immunoassay for -Human and Rat Natriuretic Polypeptide" Vol. 124 (3) pages 815-821**

**SCIENCE, vol.225, 16th April 1984, C.E. SEIDMAN et al.: "The structure of rat preproatrial-natriuretic factor as defined by a complementary DNA clone"**

(74) Representative: **Behrens, Dieter, Dr.-Ing. et al**
**Wuesthoff & Wuesthoff**
**Patent- und Rechtsanwälte**
**Schweigerstrasse 2**
**D-81541 München (DE)**

## Description

The present invention relates to polypeptides which are useful as diuretics, natriuretics and vasodilators, methods to produce said polypeptides, compositions of DNA encoding said polypeptides, as well as recombinant expression systems capable of expressing said DNA and recombinant hosts transformed with said expression system.

In the prior art the search continues for a "natriuretic hormone" which might modulate tubular reabsorption.

There are several candidates for such a hormone, among which are included the natriuretic factor(s) recently isolated from atrial muscle cells. A natriuretic effect has been demonstrated by crude extracts of rat atrial tissue but not ventricular tissue. The existence of these atrial natriuretic factors strengthens the long-held suspicion that the heart, aside from its obvious influence on renal perfusion, may play an important role in regulating renal sodium and water excretion. Stretching of the atria is known to induce diuresis and natriuresis, and this is possibly mediated by increased release of these factors.

Diuretic therapy is currently a mainstay in the treatment of hypertension, renal failure and the various edematous states (heart failure, etc.). Currently available pharmacological preparations have, however, several important limitations and undesirable effects. While their use may be directed at a specific abnormality (i.e. volume expansion), their multiple actions are undoubtedly not physiological, leading for instance to potassium depletion, increased retention of uric acid and abnormal glucose and lipid metabolism. In addition, all known diuretics profoundly stimulate the renin-angiotensin-aldosterone system, which counteracts their volume-depleting and blood pressure-lowering effects and leads to other unwanted effects. It would be desirable to provide a pharmacologically effective compound which can regulate blood pressure by providing a complete but controlled range of physiological responses.

However, the isolation of such compounds from atrial tissue is typically a cumbersome process and requires substantial substrate tissue to produce minute quantities of the compounds. While certain of these compounds can be produced by chemical synthesis, it was considered desirable to apply recombinant deoxyribonucleic acid (DNA) and related technologies to the production of larger quantities of such compounds to provide material for clinical and therapeutic applications.

Proceeding from the seminal work of Cohen & Boyer, U.S. Patent No. 4,237,224, recombinant DNA technology has become useful to provide novel DNA sequences and produce large amounts of heterologous proteins in transformed cell cultures. In general, the joining of DNA from different organisms relies on the excision of DNA sequences using restriction endonucleases. These enzymes are used to cut donor DNA at very specific locations, resulting in gene fragments which contain the DNA sequences of interest. These DNA fragments usually contain short single-stranded tails at each end, termed "sticky-ends". These sticky-ended fragments can then be ligated to complementary fragments in expression vehicles which have been prepared, e.g., by digestion with the same restriction endonucleases. Having created an expression vector which contains the structural gene of interest in proper orientation with the control elements, one can use this vector to transform host cells and express the desired gene product with the cellular machinery available. Once expressed, the gene product is generally recovered by lysing the cell culture, if the product is expressed intracellularly, or recovering the product from the medium if it is secreted by the host cell.

Recombinant DNA technology has been used to express entirely heterologous gene products, termed direct expression, or the gene product of interest can be expressed as a fusion protein containing some parts of the amino acid sequence of a homologous protein. This fusion protein is generally processed post translationally to recover the native gene product. Many of the techniques useful in this technology can be found in Maniatis, T., et al., Molecular Cloning: A Laboratory Manual, Cold Spring Harbor Laboratory, New York (1982).

However, while the general methods are easy to summarize, the construction of an expression vector containing a desired structural gene is a difficult process and the successful expression of the desired gene product in significant amounts while retaining its biological activity is not readily predictable. Frequently gene products are not biologically active when expressed in yeast, bacteria or mammalian cell systems. In these cases, post-translational processing is required to produce biological activity.

The present invention provides a polypeptide compound useful as a natriuretic, diuretic, and/or vasodilator in mammals, said polypeptide compound having the formula:

1 2 3 4 5 8 10 11

X-Arg-Ser-Ser-Cys-Phe-$AA_6$-Gly-Arg-$AA_9$-Asp-Arg-Ile-$AA_{13}$-

14 15 16 18 19 20

Ala-Gln-Ser-$AA_{17}$-Leu-Gly-Cys-Y

wherein

X is hydrogen, acetyl, or an oligopeptide of up to 125 amino acid residues including the N-acetyl derivatives thereof;

Y is hydroxyl, amido, Asn-OH, Asn-Ser-OH, Asn-Ser-Phe-OH, Asn-Ser-Phe-Arg-OH, Asn-Ser-Phe-Arg-Tyr-OH, or the C-terminal amide derivatives thereof; or an oligopeptide extension of up to 20 amino acid residues from the C-terminus of any of the foregoing including the C-terminal amide derivatives thereof;

each of $AA_6$, $AA_{13}$, and $AA_{17}$, is independently selected from the group consisting of Gly and Ala; $AA_9$ is selected from Ile, Met, and Val;

wherein 0, 1 or 2 of residues 1-20 of formulas (1) are substituted by the D-isomer thereof, and including such compound containing a disulfide bridge joining the cysteine residues and the pharmacologically acceptable salts thereof, with the proviso that when $AA_6$, $AA_7$, $AA_{13}$, and $AA_{17}$ are Gly, $AA_9$ is Ile or Met and Y is Asn-OH, Asn-Ser-OH, Asn-Ser-Phe-OH, Asn-Ser-Phe-Arg-OH, Asn-Ser-Phe-Arg-Tyr-OH, then X is H or acetyl, or at least one amino acid residue is a D-isomer.

Further embodiments of the invention are set forth in the attached claims 2 to 9.

Disclosure of the Invention

Compounds of the present invention useful as natriuretics, diuretics, vasodilators and modulators of the renin-angiotensin-aldosterone system include atrial natriuretic/vasodilator polypeptides (ANVPs) substantially free of unrelated atrial tissue or products. Also included are polypeptide compounds identified by the formula:

$$X\text{-}aa_{1-3}\text{-}\overline{Cys\text{-}aa_{5-n}\text{-}Cys}\text{-}Y$$

where n = 19, and

wherein $aa_n$ is of the general formula

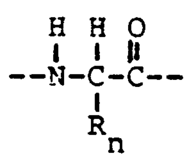

including any of the D-isomer, L-isomer and DL-isomer (racemic mixture); and

wherein

$R_n$ is hydrogen or an aliphatic, aromatic or alkaryl group of from one to ten, usually one to six carbon atoms, including groups having substitutions of three or less nitrogen, oxygen or sulfur atoms as amido, thio or oxy, including hydroxy, thiol and ethers, wherein the ether is usually an alkyl ether, generally of one carbon atom, e.g. methyl;

X is hydrogen, amido, acetyl or additionally includes an oligopeptide of up to 125 amino acid residues, including N-acetyl derivatives thereof;

Y is hydroxyl, amido or an oligopeptide of up to 20 amino acid residues, including C-terminal amide derivatives thereof.

Also provided are methods for producing compounds of the present invention using recombinant DNA techniques for expressing DNA encoding ANVPs, proANVPs or pre-proANVPs comprising:

a) preparing an expression vector capable of expressing the DNA sequence in a host cell;

b) transforming a host cell culture with said expression vector;
c) culturing said transformed host cells under conditions permitting expression of the DNA sequence to produce polypeptides comprising ANVPs proANVPs or pre-proANVP; and
d) recovering the polypeptides.

Methods for using these compounds as diagnostic and therapeutic agents are also provided.

## Brief Description of the Drawings

Figure 1 A provides the deoxyribonucleic acid (DNA) sequence of one embodiment of the present invention, namely the gene encoding human pre-proANVP together with the amino acid sequence of the polypeptide synthesis directed by this DNA;
Figure 1 B provides the complementary deoxyribonucleic acid (cDNA) sequence of one embodiment of the present invention, namely cDNA encoding human pre-proANVP, together with the amino acid sequence of the polypeptide synthesis directed by this DNA;
Figure 2 provides the deoxyribonucleic acid (DNA) sequence of one embodiment of the present invention, namely DNA encoding rat pre-proANVP, together with the amino acid sequence of the polypeptide synthesis directed by this DNA;
Figures 3 A-D are graphic representations of the purification of atrial natriuretic/vasodilator (ANVP) compounds of the present invention from atrial tissue, in which:
Figure 3 A displays the results of G-50 gel filtration of crude extract;
Figure 3 B displays the results of HPLC ($C_{18}$ column) purification of refined extract;
Figure 3 C displays the re-chromatography of the product of Figure 1B; and
Figure 3 D displays the results of HPLC (CN column) purification of the purified active fractions of Figure 1C;
Figure 4 portrays the sequences of oligonucleotide probes used to identify complementary DNA (cDNA) clones containing nucleic acid compositions of the present invention;
Figure 5 depicts the sites at which specific restriction endonucleases cleaved the deoxyribonucleic acid (DNA) encoding rat pre-proANVP to provide DNA fragments for dideoxynucleotide sequence analysis;
Figure 6 (a) shows the results of Northern blot analysis of atrial and ventricular mRNA in which lane 1 depicts RNA isolated from rat atrial tissue and lane 2 depicts RNA isolated from rat ventricular tissue;
Figures 6 (b), (c), (d) and (e) show the results of two dimensional gel fractionation of cell-free translation products encoded by poly $A^+$ RNA where (b) shows $^{35}S$ proteins encoded by atrial poly $A^+$ RNA and (c) shows $^{35}S$ proteins encoded by ventricular poly $A^+$ RNA. In vitro translations of poly $A^+$ RNA specifically hybridizing to and eluting from DNA encoding rat pre-proANVP is shown, where (d) depicts poly $A^+$ RNA derived from atrial tissue and (e) depicts poly $A^+$ RNA derived from ventricular tissue;
Figure 7 shows sites at which specific restriction endonucleases cleaved human genomic deoxyribonucleic acid (DNA) encoding human pre-proANVP to provide DNA fragments for dideoxynucleotide sequence analysis;
Figure 8 is a comparison of the vasorelaxant activity of purified and synthetic compounds of the present invention;
Figures 9A and B portray the binding of $^{125}I$-rANVP(126-150) to cultured bovine aortic smooth muscle cells (panel A) and aortic endothelial cells (panel B). The solid line (—) represents specific binding and the dashed line (---) represents non-specific binding. Analysis of the data by Scatchard plots is shown in the inset;
Figures 10A and B depict cyclic GMP levels in cultured bovine vascular smooth muscle (panel A) and vascular endothelial cells (panel B) in response to various doses of rANVP(126-150) and hANVP(127-151), respectively;
Figures 11 A and B are dose-response curves depicting the ability of various ANVPs to increase cGMP in cultured bovine aortic smooth muscle cells (panel A) and cultured bovine aortic endothelial cells (panel B). Data are expressed as the percent maximal response as a function of dose;
Figure 12 is a schematic representation of the bacterial expression plasmids used in the expression of rat and human pro-ANVP and derived fragments, in which Figure 12A shows plasmid expression vector pKT-52; Figure 12B shows a segment of DNA derived from the rat pre-proANVP cDNA shown in Figure 12C, encoding amino acids 87-152 cloned in plasmid pRNF-6852; Figure 12D shows a segment of DNA derived from the rat pre-proANVP cDNA of Figure 12C, encoding amino acids 25-152 cloned in plasmid pRNF-12852; Figure 12F shows a synthetic DNA sequence containing the tryptophan operon promoter/operator and Shine-Delgarno sequence (SD) used to construct pTRP-233 shown in Figure 12F; and Figure 12G shows a segment of DNA derived from the human pre-proANVP cDNA encoding amino

acids 26-151 cloned in plasmid phNF-233;

Figure 13 is a photographic representation of an SDS-polyacrylamide gel showing proteins labeled with L-[$^{35}$S]-cysteine in which E. coli in lane A contained the pKT52 expression vector, lane C, contained pRNF-6852 (pKT52 modified to contain DNA encoding amino acids 87-152 of pre-proANVP), lane E contained pRNF-12852 (pKT52 modified to contain DNA encoding amino acids 25-152 of pre-proANVP), lanes B, D and F, the products from lanes A, C and E, respectively, that were immunoprecipitated with a specific antiANVP antiserum, lane G, protein molecular weight standards with their corresponding molecular sizes. Arrows indicate unique polypeptides derived from pRNF-6852 and pRNF-12852;

Figure 14 is a photographic representation of an SDS polyacrylamide gel showing proteins labelled with [$^{35}$S]-cysteine in which E. coli in lane A contained the pTRP-233 expression vector, lane B contained phNF-233 (pTRP-233 modified to contain DNA encoding human pre-proANVP(26-151)) expression product; lanes C and D, the products from lanes A and B, respectively that were immunoprecipitated with specific anti-ANVP antiserum. Sizes of molecular weight standards are shown adjacent to lane A;

Figure 15 depicts a construction for expressing rat pre-proANVP and related polypeptide fragments in Saccharomyces cerevisiae using a specific vector and the yeast $\alpha$-factor secretion signal;

Figure 16 is a photographic representation of an SDS polyacyrylamide gel showing S. cerevisiae secreted proteins labeled with $^{35}$S-methionine(A) or $^{35}$S-cysteine and $^{35}$S-methionine(B). In A, S. cerevisiae in lanes 1 and 2 contained the YEp-$\alpha$-8 shuttle vector, lanes 3 and 4 contained YEp-$\alpha$-NF-9, lanes 5 and 6 contained YEp-$\alpha$-NF-12. In B, the secreted proteins were acetone and methanol extracted where lanes 1 and 2 represent S. cerevisiae containing YEp-$\alpha$-NF-7 and YFp-$\alpha$-NF-5 lanes 3 and 4 represent the proteins from these preparations after immunoprecipitation with specific anti-ANVP IgG, lanes 5 and 6 contained YEp-$\alpha$-NF-12 and YEp-$\alpha$-NF-9, respectively, and show the proteins specifically immunoprecipitated by anti-ANVP IgG;

Figure 17 depicts a schematic diagram of the yeast expression plasmid and synthetic gene sequence encoding human proatrial natriuretic/vasodilator fragment 128-151. Details are located in Section IV. A) Sequences of the relevant regions within plasmids JJ-1 and JC1-5. Representations are: ⊢—⊣, pBR322 sequences, ▬ DNA fragment containing the B form of 2$\mu$; ▭ DNA fragment encoding the $\alpha$-factor precursor/peptide; ▨ DNA fragment containing the LEU2 gene. B) Synthetic DNA gene sequence encoding human proatrial natriuretic vasodilator fragment 128-151. Component oligodeoxynucleotides are numbered 1-8. Amino acid numbering above the nucleotide sequence for $\alpha$-factor corresponds to the gene sequence described by Kurjan J. and Herskowitz, supra.

Figure 18 A depicts an expression vector construction for expressing rat and human pre-proANVP in Chinese hamster ovary cells; and

Figure 18 B is a photographic representation of an SDS-polyacrylamide gel of $^{35}$S-Methionine labeled protein from Chinese hamster ovary cell media in which lane 1 show $^{35}$S-Methionine labeled proteins from a CHO cell pool containing sequences directing the synthesis of prohANVP(26-151), lane 2 shows $^{35}$S-Methionine labeled proteins from control CHO cells, lanes 3-6 show $^{35}$S-Methionine labeled proteins from CHO cells containing sequences directing the synthesis of prohANVP(26-151) where lanes 3-6 represent CHO-8/2-93, CHO-8/2-81, CHO-8/2-55 and CHO-8/2-6 respectively.

## Best Mode for Practicing the Invention

In accordance with the present invention novel atrial natriuretic/vasodilator polypeptide compounds (ANVPs) are provided for the regulation of fluid volume and blood pressure in host organisms, in which one aspect of the invention provides ANVPs substantially free of unrelated atrial tissue or products.

Generally preferred embodiments of the present invention include compounds identified by the formula:

X-Arg-Ser-Ser-Cys-Phe-Gly(Ala)-Gly-Arg-Ile(Met)
(Val)-Asp-Arg-Ile-Gly(Ala)-Ala-Gln-Ser-Gly(Ala)-
Leu-Gly-Cys-Y

where X and Y are as previously defined, the cysteine residues are desirably bound by a disulfide bond and amino acid residues in parenthesis are alternatives to the residue immediately preceeding.

Certain presently preferred embodiments included in the above formulae include:

X'-Arg-Ser-Ser-Cys-Phe-Gly(Ala)-Gly-Arg-Ile(Val)-
Asp-Arg-Ile-Gly(Ala)-Ala-Gln-Ser-Gly(Ala)-Leu-
Gly-Cys-Y

or

X'-Arg-Ser-Ser-Cys-Phe-Gly(Ala)-Gly-Arg-Met
(Val)-Asp-Arg-Ile-Gly(Ala)-Ala-Gln-Ser-Gly(Ala)-
Leu-Gly-Cys-Y

wherein amino acid residues in parenthesis are alternatives to the residue immediately preceeding, the cysteine residues are bound by a disulfide bond and

X' is selected from the group consisting of COOH, H or OH; and

Y is selected from the group consisting of Asn-Y', Asn-Ser-Y', Asn-Ser-Phe-Y', Asn-Ser-Phe-Arg-Y' and Asn-Ser-Phe-Arg-Tyr-Y', and where Y' is OH, $NH_2$ or an oligopeptide; for example, without showing the disulfide bond, such compounds include:

H-Arg-Ser-Ser-Cys-Phe-Gly-Gly-Arg-Ile-Asp-Arg-Ile-
Gly-Ala-Gln-Ser-Gly-Leu-Gly-Cys-Asn-Ser-Phe-Arg-
Tyr-OH;

H-Arg-Ser-Ser-Cys-Phe-Gly-Gly-Arg-Ile-Asp-Arg-Ile-
Gly-Ala-Gln-Ser-Gly-Leu-Gly-Cys-Asn-Ser-Phe-Arg-
OH;

H-Arg-Ser-Ser-Cys-Phe-Gly-Gly-Arg-Met-Asp-Arg-Ile-
Gly-Ala-Gln-Ser-Gly-Leu-Gly-Cys-Asn-Ser-Phe-Arg-
Tyr-OH;

H-Arg-Ser-Ser-Cys-Phe-Gly-Gly-Arg-Met-Asp-Arg-Ile-
Gly-Ala-Gln-Ser-Gly-Leu-Gly-Cys-Asn-Ser-Phe-Arg-
OH;

H-Arg-Ser-Ser-Cys-Phe-Gly-Gly-Arg-Ile-Asp-Arg-Ile-Gly-Ala-Gln-Ser-Gly-Leu-Gly-Cys-Asn-Ser-Phe-OH;

H-Arg-Ser-Ser-Cys-Phe-Gly-Gly-Arg-Met-Asp-Arg-Ile-Gly-Ala-Gln-Ser-Gly-Leu-Gly-Cys-OH;

H-Arg-Ser-Ser-Cys-Phe-Gly-Gly-Arg-Ile-Asp-Arg-Ile-Gly-Ala-Gln-Ser-D-Ala-Leu-Gly-Cys-Asn-Ser-Phe-Arg-Tyr-OH;

H-Arg-Ser-Ser-Cys-Phe-Gly-Gly-Arg-Ile-Asp-Arg-Ile-Gly-Ala-Gln-D-Ser-Gly-Leu-Gly-Cys-Asn-Ser-Phe-Arg-Tyr-OH;

H-Arg-Ser-Ser-Cys-Phe-Gly-Gly-Arg-Ile-Asp-Arg-Ile-Gly-Ala-Gln-Ser-Gly-D-Leu-Gly-Cys-Asn-Ser-Phe-Arg-Tyr-OH;

H-Arg-Ser-Ser-Cys-Phe-Gly-Gly-Arg-Ile-Asp-Arg-Ile-Gly-Ala-D-Gln-Ser-Gly-Leu-Gly-Cys-Asn-Ser-Phe-Arg-Tyr-OH;

H-Arg-Ser-Ser-Cys-Phe-Gly-Gly-Arg-Ile-Asp-Arg-Ile-D-Ala-Ala-Gln-Ser-Gly-Leu-Gly-Cys-Asn-Ser-Phe-Arg-Tyr-OH;

H-Arg-Ser-Ser-Cys-Phe-Gly-Gly-Arg-Ile-Asp-Arg-Ile-Gly-D-Ala-Gln-Ser-Gly-Leu-Gly-Cys-Asn-Ser-Phe-Arg-Tyr-OH;

H-Arg-Ser-Ser-Cys-Phe-D-Ala-Gly-Arg-Ile-Asp-Arg-Ile-Gly-Ala-Gln-Ser-Gly-Leu-Gly-Cys-Asn-Ser-Phe-Arg-Tyr-OH;

H-Arg-Ser-Ser-Cys-Phe-Gly-Gly-D-Arg-Ile-Asp-Arg-Ile-Gly-Ala-Gln-Ser-Gly-Leu-Gly-Cys-Asn-Ser-Phe-Arg-Tyr-OH;

H-Arg-Ser-Ser-Cys-Phe-Gly-Gly-Arg-D-Val-Asp-Arg-Ile-Gly-Ala-Gln-Ser-Gly-Leu-Gly-Cys-Asn-Ser-Phe-Arg-Tyr-OH;

H-Arg-Ser-Ser-Cys-D-Phe-Gly-Gly-Arg-Ile-Asp-Arg-Ile-Gly-Ala-Gln-Ser-Gly-Leu-Gly-Cys-Asn-Ser-Phe-Arg-Tyr-OH;

H-Arg-Ser-Ser-D-Cys-Phe-Gly-Gly-Arg-Ile-Asp-Arg-Ile-Gly-Ala-Gln-Ser-Gly-Leu-Gly-Cys-Asn-Ser-Phe-Arg-Tyr-OH;

H-Arg-Ser-Ser-Cys-Phe-Gly-D-Ala-Arg-Ile-Asp-Arg-Ile-Gly-Ala-Gln-Ser-Gly-Leu-Gly-Cys-Asn-Ser-Phe-Arg-Tyr-OH;

H-Arg-Ser-Ser-Cys-Phe-Gly-Gly-Arg-D-Met-Asp-Arg-Ile-Gly-Ala-Gln-Ser-Gly-Leu-Gly-Cys-Asn-Ser-Phe-Arg-Tyr-OH;

H-Arg-Ser-Ser-Cys-Phe-Gly-Gly-Arg-Ile-Asp-D-Arg-Ile-Gly-Ala-Gln-Ser-Gly-Leu-Gly-Cys-Asn-Ser-Phe-Arg-Tyr-OH;

H-Arg-Ser-Ser-Cys-Phe-Gly-Gly-Arg-Ile-Asp-Arg-D-Val-Gly-Ala-Gln-Ser-Gly-Leu-Gly-Cys-Asn-Ser-Phe-Arg-Tyr-OH;

A further aspect of the invention provides nucleic acid sequences capable of directing the synthesis of ANVPs, proANVPs (the precursor forms of the mature ANVP expression product compounds) and pre-proANVPs (proANVPs with intact signal peptides), and fragments derived therefrom, such nucleic acid sequences comprising the DNA sequences of Figures 1 and 2, including oligonucleotide sequences contained therein, and allowing for the replacement of codons with other codons capable of directing the synthesis of the same or functionally equivalent amino acid sequences, such equivalent amino acids including the alternative residues disclosed in the examples provided.

The nomenclature used to describe ANVP compounds of the present invention follows the conventional practice of using the first three letters of the trivial name of the amino acid and wherein the L form of any

amino acid having an optical isomer is intended unless otherwise expressly indicated.

Compounds within the scope of the present invention can also be obtained by modifying the above recited formulae in numerous ways while preserving the activity of the ANVP compounds thus obtained. For example, while the amino acids of these compounds are normally in the natural L form, one or more, usually two or less and preferably one amino acid may be replaced with the optical isomer D form, or a DL-racemic mixture, as demonstrated in certain of the illustrative examples included in the present application. Amino acid residues contained within the compounds can also be modified by amidation, acetylation or substituted with other chemical groups which can, for example, change the solubility of the compounds without effecting their activity.

In addition, one or more amino acid residues can be replaced by functionally equivalent residues; for example basic polar amino acids can be replaced with other basic polar amino acids and acidic polar amino acids can be replaced with other acidic polar amino acids. However, the replacement of certain nonpolar hydrophobic amino acids, particularly cysteine, are considered less desirable due to the likelihood of interfering with the cystine disulfide bridge.

The ANVP compounds of the present invention can also be modified by extending, decreasing or substituting in the compounds' amino acid sequence, e.g., by the addition or deletion of amino acids or oligopeptides on either the N-terminal or C-terminal end, or both, of the sequences disclosed above. Particularly, Y' can be amide or an amino acid or oligopeptide of not more than about 20, more usually 8, and desirably 5 or less amino acids and X' can be N-acetyl or an amino acid or oligopeptide of not more than about 125, and desirably about 101 amino acids, provided the modifications do not adversely effect all of the natriuretic, diuretic and vasorelaxant activities of the subject compounds.

Furthermore, compounds of the present invention can be mixed with, bonded to or conjugated with compounds having the same or a complementary range of biologic activities to obtain the benefits of the present invention.

Certain presently preferred ANVP compounds of the present invention have been isolated from atrial tissue substantially free of unrelated atrial tissue or products. Generally, acetic acid extracts of atrial tissue are subjected to gel filtration, and reversed phase high performance liquid chromatography (using $C_{18}$ and CN columns), while assaying for the natriuretic and vasorelaxant activity of the fractions.

Compounds within the scope of the present invention can be isolated and purified from biological tissue sources, notably mammalian atrial tissue sources, or can be synthesized chemically by means well-known in the art such as, e.g., solid phase synthesis. The synthesis is commenced from the C-terminal end of the peptide using an alpha-amino protected amino acid. t-Butyloxycarbonyl (Boc) protective groups can be used for all amino groups even though other protective groups are suitable. For example, Boc-Arg-OH or Boc-Tyr-OH (i.e., selected C-terminal amino acids) can be esterified to chloromethylated polystyrene resin supports. The polystyrene resin support is preferably a copolymer of styrene with about 0.5 to 2% divinyl benzene as a cross-linking agent which causes the polystyrene polymer to be completely insoluble in certain organic solvents. See Stewart et al., Solid-Phase Peptide Synthesis, W. H. Freeman Co., San Francisco (1969) and Merrifield, J. Am. Chem. Soc. 85:2149-2154 (1963).

Conveniently, ANVP compounds may be synthesized using manual techniques or automatically employing, for example, a Biosearch SAM II automatic peptide synthesizer (Biosearch, Inc. San Rafael, California) as described in the instruction manual.

Alternatively, compounds of the present invention can be produced by expression of recombinant DNA constructs. Such production can be desirable to provide large quantities or alternative embodiments of such compounds.

More particularly, modifications in the amino acid sequence of the various forms of pre-proANVP, proANVP and ANVP compounds can be effected by various changes in the nucleotide sequence of the cloned structural gene used to direct the synthesis of the compounds. Included within such modification of the DNA sequence are the replacement of various codons with other codons which, due to the degeneracy of the genetic code, direct the synthesis of the same amino acid.

In addition, by codon substitution, one or more amino acid residues can be replaced by functionally equivalent residues, as disclosed above.

Compounds of the present invention are shown to have natriuretic and diuretic activity in the intact mammal and in the kidney isolated from a mammal. Furthermore, compounds of the present invention including synthetic compounds, possess vasorelaxant activity, and inhibit the release of aldosterone that has been shown to be enhanced by oxidation and diminished by reduction, which indicates the presence of a disulfide bridge between the cysteine residues contained in the general formulae disclosed herein is required for the biological activity described above.

Compounds of the present invention which are shown to have the above recited physiological effects can find use in numerous therapeutical applications such as, e.g., inducing natriuresis, diuresis, and vasodilatation. Thus these compounds can find use as therapeutic agents in the treatment of various edematous states such as, for example, congestive heart failure, nephrotic syndrome and hepatic cirrhosis, in addition to hypertension and renal failure due to ineffective renal perfusion or reduced glomerular filtration rate.

These compounds can be administered to mammals for veterinary use such as with domestic animals, and clinical use in humans in a manner similar to other therapeutic agents, that is in a physiologically acceptable carrier. In general the dosage will range from about 0.01 to 100 µg/kg, more usually 0.1 to 10 µg/kg of the host body weight. Alternatively, dosages within these ranges can be administered by constant infusion over an extended period of time, usually exceeding 24 hours, until the desired therapeutic benefits have been obtained.

These compounds can be administered neat, as mixtures with other physiologically acceptable active or inactive materials, or with physiologically suitable carriers such as, for example, water or normal saline. The compounds can be administered orally or parenterally, for example, by injection. Injection can be subcutaneous, intravenous, or by intramuscular injection.

These compounds are desirably administered in pharmaceutically effective amounts and often as pharmacologically acceptable salts such as acid addition salts. Such salts can include, e.g., hydrochloride, hydrobromide, phosphate, sulphate, acetate, benzoate, malate, among others.

Compounds of the present invention can also be used for preparing antisera for use in immunoassays employing labelled reagents, usually antibodies. Conveniently, the polypeptides can be conjugated to an antigen by means of dialdehydes, particularly from 4 to 6 carbon atoms and aliphatic, or carbodiimide. These compounds and immunologic reagents may be labelled with a variety of labels such as chromophores, fluorophores such as, e.g., fluorescein or rhodamine, radioisotopes such as $^{125}I$, $^{35}S$, $^{14}C$, or $^{3}H$, or magnetized particles, by means well known in the art.

These labeled compounds and reagents, or labeled reagents capable of recognizing and specifically binding to them, can find use as, e.g., diagnostic reagents. Samples derived from biological specimens can be assayed for the presence or amount of substances having a common antigenic determinant with compounds of the present invention. In addition, monoclonal antibodies can be prepared by methods known in the art, which antibodies can find therapeutic use, e.g., to neutralize overproduction of immunologically related compounds in vivo.

The following examples are provided by way of illustration, rather than implying any limitation of the subject invention.

## EXPERIMENTAL

In the experimental disclosure which follows, pre-proANVPs, proANVPs and ANVPs derived from rat and human DNA sequences have amino acid residues numbered 1-152 and 1-151 respectively to indicate differences in the disclosed amino acid sequences. The amino acid sequence of chemically synthesized ANVPs are numbered from the arginine residue found at position 126 in the rat-derived sequence and position 127 in the human-derived sequence (see Figures 1 & 2).

### I. Isolation and Purification of Atrial Natriuretic/Vasodilator Polypeptide Compounds

Compounds within the scope of the present invention have been isolated from atrial tissue in accordance with the following protocol. These compounds and their synthetic polypeptide analogs are included in the collective term ANVPs.

A polypeptide compound was isolated from an acetic acid extract of atria, substantially free from unrelated tissue and products. Atria from 1400 male Wistar rats were homogenized in 8 volumes of 1 N acetic acid containing 1 mM phenylmethylsulfonyl fluoride (PMSF Sigma Chemical Co., St. Louis, Mo.), 3 mM ethylenediaminetetraacetic acid (EDTA) and 5 uM pepstatin A (pepsin and renin inhibitor, Sigma Chemical Co., St. Louis, MO). This homogenate was centrifuged at 10,800 x g for 30 minutes and the pellet was rehomogenized in 4 volumes of the original buffer. The supernants from the extracts were pooled and neutralized with ammonium hydroxide. The neutralized supernatants were then centrifuged at 10,000 x g for 20 minutes and lyophilized.

The lyophilized atrial extract was reconstituted in 6 ml buffer, centrifuged and loaded on a 2.5 X 45 cm gel filtration column of Sephadex® G-50 (fine, Pharmacia Fine Chemicals, Piscataway, NJ) previously equilibrated with 1 N acetic acid. Aliquots from each fraction were dried (Savant Speed-Vac concentrator),

reconstituted in phosphate buffered saline (PBS) and assayed for natriuretic activity in intact rat and for vasorelaxant activity using rabbit aortic rings.

The results of this chromatographic step were as shown in Figure 3A, and the regions contained in horizontal bracket were lyophilized, reconstituted with 0.1% aqueous trifluoroacetic acid (TFA), pooled and centrifuged.

The pooled material was adjusted to 15% acetonitrile ($CH_3CN$) and applied to a 0.39 X 30.0 centimeter μ-Bondapak $C_{18}$ column (Waters, Inc., Milford, MA), using a Waters U6K injector and solvent delivery system (Waters, Inc., Milford, MA). Bound material was eluted with a linear gradient of solvents A (0.1% TFA): B ($CH_3CN$) from 85:15 to 45:55 over 40 minutes.

Aliquots of the fractions were assayed for natriuresis in the isolated kidney and vasorelaxant activity as described subsequently. A broad region of coincident natriuretic and vasorelaxant activity was eluted and these fractions were pooled and dried.

The material obtained and dried was reconstituted in A:B, 78:22, and rechromatographed (in 12 separate applications) at 1.0 ml/min. using a gradient of 22 to 34% B over 48 minutes. Aliquots of the fractions were tested for natriuretic and vasorelaxant activities as described. The results were as displayed in Figure 3B. Fractions from the three active peaks were pooled and dried overnight.

The combined fractions from the second peak (indicated by bracketed area in Figure 3B) were reconstituted in A:B, 77:23, applied to a $C_{18}$ column and eluted using a gradient of 23 to 29% B over 90 minutes. The results of this rechromatography were as shown in Figure 3C, where the bracketed area indicates fractions with vasorelaxant activity. Active fractions from 6 applications were pooled.

The material thus obtained was applied to a 0.39 X 30 cm μ-Bondapak CN column (waters, Inc., Milford, MA). The solvent system used was A (0.1% TFA in water) and B (0.055% TFA in $CH_3CN$). The sample was reconstituted in A:B, 90:10, and chromatographed in three separate applications at 0.6 ml/min. using a gradient of 10 to 30% B over 60 minutes. Vasorelaxant activity was determined by the reduction in tension produced in histamine-contracted aortic rings as described subsequently.

The most active peak, indicated by the bracket in Figure 3D, was dried and sequenced. The sequence was determined from one nanomole of protein using the Applied Biosystems 470A gas-phase sequencer (Applied Biosystems Inc., Foster City, CA) in accordance with the instructions of the manufacturer. PTH amino acids were identified with a Beckman 334 T HPLC, using a 0.46 X 25 cm IBM CN-column. The gradient applied was as indicated in Hunkapiller, N. W. and L. F. Hood, Methods in Enzymology, 91:486-492 (Academic Press, New York) (1983), with the following modifications: The binary gradient system was replaced by a ternary gradient system in which acetonitrile and methanol were pumped by separate pumps and the ratio of the two varied with time over the course of the gradient, with appropriate modification of the gradient program; the Permaphase ETH® guard column was replaced with a 5 X 0.46 centimeter IBM CN analytical "mini-column", and the analytical column was heated to 28°C.

The following compound, isolated substantially free from unrelated rat atrial tissue and products has the sequence

H-Arg-Ser-Ser-Cys-Phe-Gly-Gly-Arg-Ile-Asp-Arg-Ile-Gly-
Ala-Gln-Ser-Gly-Leu-Gly-Cys-Asn-Ser-Phe-Arg-X

where X = OH or Tyr-OH

To provide comparison with the amino acid sequences of rat and human pre-proANVPs and proANVPs deduced from nucleotide sequences described below, the compounds are referred to as

rANVP (126-149) where X = OH and

rANVP (126-150) where X = Tyr-OH

The human equivalents of these ANVPs, derived from the corresponding human DNA sequences described below, are referred to as hANVP (127-150) and hANVP (127-151), respectively. ANVP compounds of the present invention are numbered with the $NH_2$ terminal arginine residue numbered as described (126-rat and 127-human).

Because of the methods used to assay the products of the purification procedure, natriuretic and vasorelaxant activity is an inherent property of the isolated and purified ANVP material.

II. Recombinant DNA Cloning of Atrial Natriuretic/Vasodilator Polypeptides

In the examples that follow, deoxyribonucleic acid (DNA) sequences encoding rat and human derived pre-proANVPs and proANVPs are described. It is required that numerous alternative sequences can be constructed which will direct the expression of polypeptide embodiments of the present invention.

A. Cloning of Rat Pre-proatrial natriuretic/vasodilator polypeptide cDNA.

1. Isolation of rat atrial mRNA

Total RNA was isolated from rat atria by the method of Chirgwin, J.M. et al. Biochemistry 18:5294-5299 (1979). The tissue was homogenized in a solution of 6 M guanidine thiocyanate, 0.005 M sodium citrate, pH 7.0, 0.1 M 2-Mercaptoethanol, 0.5% Sarcrosyl. This homogenate was made 2.0 M in CsCl and layered over a 5.7 M CsCl cushion in 0.1 M EDTA. The RNA was pelleted through this cushion by centrifugation at 115,000 x g for 16 hours. The RNA was then dissolved in 0.01 M Tris, pH 7.4, 0.005 M EDTA, 1.0% sodium dodecylsulfate (SDS), extracted with a 4:1 mixture of chloroform and 1-butanol, and precipitated from 70% ethanol.

The polyadenylated RNA (poly $A^+$ RNA) fraction was obtained by affinity chromatography using oligo (dT) cellulose as described by Aviv, H. and P. Leder, Proc. Natl. Acad. Sci. USA 69:1408-1412 (1972). The poly$A^+$ RNA was bound to the cellulose matrix in a solution of 0.02 M Tris, pH 7.6, 0.001 M EDTA, 0.1% SDS, containing 0.5 M NaCl. The non-polyadenylated RNA was removed by washing the column with this solution. The poly$A^+$ RNA was then eluted in the same solution minus NaCl, and precipitated from 70% ethanol. With these techniques, 100 µg of polyadenylated RNA was isolated from 10 gm of atrial tissue.

2. Generation of rat atrial cDNA library

Double-stranded cDNA was synthesized and prepared for insertion into the plasmid vector pUC8 (Vieira, J. and J. Messing, Gene 19:259-268, 1982) using the sequential addition of EcoRI and SalI oligonucleotide linkers as described by Helfman, D.M. et al., Proc. Natl. Acad. Sci. USA 80:31-35 (1983).

First strand cDNA was synthesized by the RNA-dependent DNA polymerase from Avian Myeloblastosis Virus primed with oligo$(dT)_{12-18}$. The RNA template was then removed by base hydrolysis. Second strand DNA was synthesized by RNA-dependent DNA polymerase, relying on self-priming at the 3'-end of the first strand molecule, thereby forming a double-stranded hairpin DNA. These molecules were blunt-ended at the open-ended termini using the large fragment of DNA polymerase I of E. coli to fill in single-stranded regions. EcoRI oligonucleotide linkers were added to the open-end using T4-DNA ligase. The hairpin loop was cleaved open with $S_1$ nuclease from Aspergillus oryzae and the termini of the molecules were again blunt-ended as before. SalI oligonucleotide linkers were then added, using T4-DNA ligase. SalI and EcoRI "sticky ends" were released by cleavage with these restriction endonucleases. These double-stranded double-linkered cDNA molecules were then ligated into EcoRI and SalI-digested pUC8 and transferred into E. coli MC1061 by the $CaCl_2$-treatment described by Casabaden, M. and S. Cohen, J. Mol. Biol. 138:179-207 (1980).

Five µg of rat atrial poly $A^+$ RNA yielded about 25 ng of cDNA, size selected to greater than 300 base pairs, and gave a library of about 200,000 independent recombinants. These recombinants were plated on nitrocellulose filters, replica plated and the library stored frozen on glycerol impregnated filters at -70°C with the protocol of Hanahan, D. and M. Meselson, Gene 10:63-67 (1980) and Hanahan, D. and M. Meselson, Methods in Enzymology, Academic Press, New York, pp. 333-342.

3. Screening of the rat atrial cDNA library

Amino acid sequences for native rat ANVPs, as determined in Section I, were used to design oligonucleotide probes to screen the rat atrial cDNA library, as described in Wallace, R.B. et al., Nucleic Acids Res. 9:879-894 (1981)). Due to the degeneracy of the genetic code, two oligonucleotide pools were synthesized for each region. Region 1 was covered by two tetradecamer oligonucleotide pools, probe a and probe b, each consisting of 64 base sequences. Region 2 was covered by another two tetradecamer pools, probe c and probe d, each consisting of 72 sequences. The sequence and location of these oligonucleotide probes are shown in Figure 4. The sequence of amino acids 4-13 of native rat ANVP is shown along with the sequence of the four oligonucleotide mixtures, probes a and b for region 1, and probes c and d for region 2, wherein R = A or G, Y = T or C, N = A,G,T or C. Each oligonucleotide mixture was synthesized on a

Biosearch SAM I oligonucleotide synthesizer (Biosearch, Inc., San Rafael, Cal.) by a modification of the standard phosphotriester method using mesitylenesulfonyl chloride in the presence of N-methylimidazole as condensing reagents as described by Efimov, V.A. et al., Nuc. Acids Res. 10:6875-6894 (1982) and purified by polyacrylamide gel electrophoresis.

The cDNA library was then screened by colony hybridization using these probes. Four replica filters were prepared from each filter, so that each colony could be screened with each oligonucleotide probe pool.

The filters were baked for 2 hrs. at 80 °C under vacuum and then washed overnight at 68 °C with shaking in a large volume of 3X SSC (where 1X SSC is 0.15 M NaCl, 0.15 M sodium citrate, pH 7.5) 0.1% SDS. The filters were prehybridized in 6X SSC, 0.1% SDS, 1 mM EDTA, 5x Denhardt's solution, (0.1% Ficoll, 0.1% polyvinylpyrrolidone, 0.1% bovine serum albumin) 0.05% sodium pyrophosphate at 50 °C for a minimum of 2 hrs.

Filters were then hybridized with 2.5 X $10^6$ cpm $^{32}P$-labeled oligonucleotide probe mixture (phosphorylated in accordance with Maniatis, T. et al., Molecular Cloning, Cold Spring Harbor Laboratories, 1982, pp. 122-123) per filter in 10 ml hybridization solution containing 100 µg/ml tRNA at 45 °C in a shaking water bath. After 1 hr., the thermostat was lowered to 25 °C and the bath allowed to equilibrate for 12 hrs. The filters were washed twice in 6X SSC, 0.1% SDS at room temperature for 15 mins., then washed in 6X SSC, 0.1% SDS at 35 °C (for probes c and d) or 39 °C (for probes a and b) for 1-2 mins. The final washing temperature was obtained from the empirical formula of Suggs, S.V. et al., Developmental Biology Using Purified Genes (ed. D.D. Brown and C.F. Fox) Academic Press, New York pp. 683-693, that is $T_d = 4(G + C) + 2(A + T)$. The hybridized filters were then dried and autoradiographed on Kodak® XAR film with Dupont® Cronex intensifying screens until complete exposures were obtained.

A colony was considered positive if it hybridized with one probe from region number 1 and one probe from region number 2. One colony was chosen which hybridized strongly to the oligonucleotide probes (pools a and c) and hybridized to a random primed atrial cDNA probe but not a ventricular cDNA probe. Sequencing of this clone demonstrated that it encoded rat pre-proANVPs. This clone is referred to as pNFl.

4. Complete sequencing of the rat pre-proatrial natriuretic/vasodilator polypeptides cDNA.

The purified DNA insert, obtained from pNFl, was prepared using small miniprep methods (Maniatis et al., supra at p. 366) and isolated on acrylamide gels. The intact DNA insert was then subcloned into bacteriophage M13 (a single stranded phage designed specifically for DNA sequencing using the dideoxynucleotide method as described by Messing J. and J. Vieira, Gene 19:259-268 (1982)), via the EcoRI and SalI sites on the 5' and 3' ends, respectively (Figure 5). An initial reading of the entire sequence was then obtained from these clones using the Sanger dideoxynucleotide sequencing technique, Sanger, F. et al., Proc. Nat. Acad. Sci. USA 74:5463-5469 (1977). In order to confirm this initial sequence, a separate reading of the other DNA strand was necessary. For this, the HincII site at base 340 was used. The prepared insert was cleaved with endonuclease HincII, and the resulting digest was cloned into M13 mp9 cleaved with SmaI plus EcoRI (arrow 5) and M13 mp8 digested with SalI plus SmaI (arrow 6). A similar approach was taken using the PstI site at base 647 to obtain additional confirmation (arrows 3 and 4). Although the initial clone used for sequencing (pNFl) terminated at base 784 of the sequence (see figure 2), another clone (pNF4) extended further 3', containing the final 22 bases plus the 3' poly A tail. The sequence of the 3' end of this clone was obtained using M13 clones containing the PstI to SalI portion of the insert (arrow 7) and is shown in Figure 2 as bases 785-806. Finally, the very 5'-terminal nucleotides of the DNA were determined by Maxam and Gilbert sequencing (Maxam, A. and W. Gilbert, Proc. Nat. Acad. Sci. USA 74:560-564 (1977)) of a $^{32}P$-labelled single stranded DNA made complementary to the 5' region using the BglII fragment spanning bases 1-186. The sequence determined thereby was included in Figure 2 as bases 1-22. Thus, nucleotide sequence analysis confirmed that clone pNFl, which includes bases 23-784 of Figure 2, encodes an ANVP precursor, pre-proANVP. When the atrial cDNA library was re-screened with the cDNA insert, approximately 0.5% of the colonies hybridized. This indicates that pre-proANVP mRNA is a major species in rat atrial mRNA population.

The amino acid sequence of native rat pre-proANVP was determined from the cDNA nucleotide sequence. A single open reading frame encoding a 152 amino acid sequence was disclosed, extending from the initiation codon ATG at base 85 to the termination codon TAA at position 541. Biologically active ANVPs (see Figure 5) can be identified in the amino acid sequences of human and rat pre-proANVPs (see Figure 1 and Figure 2, respectively)).

5. Determination of atrial specificity

Atrial and ventricular poly $A^+$ RNA was subject to Northern blot analysis after fractionation by electrophoresis on a 1.4% agarose gel containing methylmercuric hydroxide by the method of Bailey, J.M. and N. Davidson, Anal. Biochem. 70:75-85 (1976). Northern blot analysis results, using nick translated pNFI DNA, are shown in Figure 6a where lane 1 contains atrial poly $A^+$ RNA and lane 2 ventricular poly $A^+$ RNA. As indicated in Figure 6a, pNFI hybridizes to an atrial mRNA of approximately 800-900 nucleotides in length. It does not hybridize with ventricular mRNA.

The cDNA sequence for pre-proANVP determined above indicates that pre-proANVP has a molecular weight of approximately 16,500 daltons. To determine the actual precursor size, atrial mRNA encoding pre-proANVP was purified by hybrid selection (Goldberg, M.L. et al., Methods in Enzymology 68:206-220, Academic Press, New York), by immobilizing 5 ug pNFI DNA on 1 $cm^2$ nitrocellulose discs and hybridizing with 5 ug of poly $A^+$ RNA for 3 hrs. at 50 °C in 20 mM PIPES, pH 6.4, 1mM EDTA, 65% formamide, 5X SSC, 0.1% SDS. The filters were washed extensively with 10 mM Tris-HCl, pH 7.5, 0.15 M NaCl, 1mM EDTA, 0.1% SDS at 70 °C. Thereafter filters were washed in the same buffer but without SDS. Hybridized RNA was eluted in $H_20$ at 100 °C in the presence of 50 ug yeast tRNA for 1 min. and quickly frozen at -70 °C. After thawing, the RNA was ethanol precipitated using 2 volumes of absolute ethanol.

Hybrid selected RNA and total poly $A^+$ RNA was translated using a rabbit reticulocyte lysate system (Bethesda Research Labs, Gaithersburg, Maryland) in the presence of 250 uCi/ml [$^{35}$ S]-methionine. Translation products were fractionated by 2-dimensional gel electrophoresis by loading 1 X $10^6$ cpm of acid-precipitable radioactivity per sample. The first dimension was an isoelectric focusing gel using a gradient from pH3.5-10, O'Farrell, P.Z. et al., Cell 12:113-1142 (1977). The results of the isoelectric focusing were subject to electrophoresis in an SDS-PAGE using a 15% gel. Following sodium salicylate equilibration, the gel was dried and then fluorgraphed at-70 °C for 24 hrs.

The results were as indicated in Figures 6b and 6c, where the position of several atrial-specific translation products having molecular weights between 12,000 and 30,000 daltons are marked by arrows. Translation products encoded by pNFI hybrid selected atrial RNA are indicated in Figure 6d which shows at least 3 related protein species having molecular weight between 18,000 and 20,000 daltons which are major atrial-specific species. Figure 6e shows that hybrid selection does not recognize any ventricular-specific proteins. Because the proteins in Figure 6d were hybrid selected, are atrial specific and are of the correct molecular weight range, they represent pre-proANVPs.

B. Cloning of the Human Gene Encoding Pre-proatrial natriuretic/vasodilator polypeptides

1. Isolation of the human native pre-proatrial natriuretic/vasodilator polypeptide gene

The cDNA (isolated from pNFI) encoding rat pre-proANVP provided a probe for identifying the human gene. A human genomic clone library in bacteriophage Charon 4A (Lawn, R.M. et al., Cell 15:1157-1174 (1978)) was obtained from Dr. T. Maniatis, Harvard University. Approximately $10^6$ phage were grown on E. coli K803, and plaque lysates were transferred to nitrocellulose filters as described by Benton, W.D. and R. W. Davis, Science 196:180-182 (1977). These filters were hybridized with the rat cDNA which had been radioactively labeled with $^{32}P$ by the nick-translation method of Rigby, P. W J. et al., J. Mol. Biol. 113:237-251 (1977). Filters were pre-washed in hybridization buffer (0.75 M NaCl, 0.75 M sodium nitrate, 40% formamide, 0.05% SDS, 0.02% bovine serum albumin, 0.02% Ficoll - 400,000, 0.02% polyvinyl pyrollidone, 0.1% sodium pyrophosphate, 20 $\mu$g/ml denatured sheared salmon sperm DNA) at 42 °C for 1 hr. 5 x $10^5$ cpm of $^{32}P$-labelled boiled rat pre-proANVPs cDNA was added per ml of fresh hybridization buffer and the filters were incubated in this buffer at 42 °C for 16 hrs. Filters were then washed in 0.3 M NaCl and 0.3 M sodium nitrate and .05% SDS three times at 50 °C, and exposed for autoradiography overnight. Six clones containing sequences hybridizing to rat native pre-proANVP cDNA were purified.

The size of the native human pre-proANVP gene was determined to permit identification of a full length clone. Two mg of high-molecular weight DNA was prepared from 20 g of rat liver by the method of Blin, N. and D Stafford, (Nuc. Acid Res. 3:2303-2308 (1976)). This DNA was digested with the restriction endonucleases BamHI, BglII, KpnI, and SacI, alone and in combination with EcoRI, electrophoresed on 1% agarose gels, and transferred to nitrocellulose filters by the method of Southern, E. M., J. Mol. Biol. 98:503-517 (1975). These filters were probed for sequences homologous to native rat pre-proANVPs by the same conditions used to identify the clones. In this manner a unique 2,600 base pair EcoRI - BamH1 DNA fragment was identified which appeared to span the entire gene.

The six human genomic clones that hybridized to rat pre-proANVPs cDNA were then analyzed for the presence of a similarly sized fragment and one of them, designated λHG6, contained such a fragment.

λHG6 DNA was then digested with EcoRI and BamHI and DNA fragments were ligated into pBR322 previously digested with the same endonucleases. Ligation products were transfered into E. coli MC1061 cells as previously described. Plasmid pHGRBI was thusly generated among the clones to the other fragments, and identified by the colony hybridization procedure of Grunstein, M. and D. Hogness, Proc. Natl. Acad. Sci. USA 72:3961-3965 (1975). Hybridizations were performed as described above. pHGRBI was then sequenced and shown to contain the entire gene sequence for native human pre-proANVP.

2. Sequencing of the human native pre-proatrial natriuretic/vasodilator polypeptide gene.

For the human gene, the 2589 base pair fragment shown to hybridize with the rat cDNA was prepared from a large-scale plasmid prep by 4% polyacrylamide gel electrophoresis. Before sequencing could proceed, the large size of the DNA segment dictated that several useful restriction endonuclease cleavage sites be determined which would break the sequence into smaller fragments. Particularly useful sites were found at positions 586 (SstI), 984 and 1839 (AvaI), and 1581 and 2374 (PstI). These sites are shown in Figure 7 which portrays the human gene sequencing strategy consistent with methods described for rat cDNA in Section II.A.4. Several M13 subclones were prepared spanning the DNA segments between these sites in order to cover these regions on both DNA strands. The DNA fragments generated by restriction endonuclease cleavage and M13 subcloning are indicated in Figure 7 by arrows 1-10. The resulting sequence is shown in Figure 1A. The sequence information obtained was analyzed using various Intelligenetics (Palo Alto, California) computer programs in accordance with the instructions of the manufacturer. The regions containing the signal peptide, precursor sequence and mature peptide were identified by comparison to the rat native pre-proANVP cDNA. The entire coding region is contained within the BamHI to EcoRI fragment, and the coding region for the gene contains 2 introns of 122 and 1095 bases, and 3 exons spanning approximately bases 577-696, 819-1145 and 2241-2536. Putative control signals for both transcriptional initiation (bases 347-354 and 446-452) and termination (bases 2515-2520) were also localized within the fragment. The human equivalence of the rANVPs isolated in Section I can be deduced within the second and third exons of the human gene.

C. Cloning of human Pre-proatrial natriuretic/vasodilator polypeptide cDNA

1. Isolation of human fetal heart in RNA

A human fetal heart, obtained at 26 weeks of gestation, was used to prepare poly $A^+$ mRNA, as described in section II. From a total of 1.7 gm of heart tissue 60 μg of poly $A^+$ mRNA was isolated.

2. Generation of a human fetal heart cDNA library

Double stranded cDNA was prepared as described in Manniatis, T. et al., Molecular Cloning, Cold Spring Harbor Laboratories, 1982, pp. 212-246. Ten μg of the template RNA was copied into the first strand cDNA using AMV reverse transcriptase, primed with oligo dT 12-18. The RNA template was then removed by base hydrolysis and double stranded. DNA was synthesized by AMV reverse transcriptase relying on self priming by the hairpin loop found naturally at the 3'-end of the first strand cDNA. The resulting double stranded hairpin DNA was then treated with the $S_1$-nuclease from Aspergillus oryzae to remove the hairpin loop, and the resulting molecules were treated with the large fragment of E. coli DNA polymerase I to make them blunt ended. EcoRI oligonucleotide linkers were added to the cDNA molecules using $T_4$-DNA ligase and the cohesive EcoRI ends were released by cleavage with the restriction enzyme EcoRI. The resulting double stranded, EcoRI linkered cDNA was then size fractionated on a Biogel A-50m column (BioRad, Richmond, ) and 10 ng of cDNA greater than 500 bp long was recovered.

The size fractionated cDNA was then cloned into the bacteriophage λ-vector, λgt10, as described by Huynh, T.V. et al., cDNA Cloning Techniques: A Practical Approach, ed. D. Glover (IRL, Oxford) 1984 in press. DNA was prepared from λgt10 and digested with EcoRI. This DNA was ligated to the EcoRI linkered human fetal heart cDNA and packaged in vitro using the packaging kit obtained from Amersham. The resulting phage were then plated on the E. coli strain BNN102 described by Huynh, T.V. et al., supra. In this way, a human fetal heart library of about 200,000 individual members, was obtained and amplified for storage and subsequent screening.

3. Screening of the human fetal heart cDNA library

The cDNA library was screened by plague hybridization as described by Maniatis, T. et al., pp. 320-321. The hybridization probe was the EcoRI - SalI insert from the rat pre-proANVP, cDNA clone pNF1 (see Section II.A.3 of this application). This purified DNA fragment was $^{32}P$ labelled by nick translation using the kit available from Bethesda Research Laboratories.

Using the amplified human fetal heart cDNA library, phage were plated on plates using the host strain BNN102. Nitrocellulose filters were lifted from these plates, baked for 2 hours at 80 ° C under vacuum, and hybridized to 5 X $10^5$ cpm of [$^{32}P$] labelled rat pre-proANVP cDNA, pNF1 insert. Hybridization was in 40% formamide, 50mM Na phosphate pH 6.5, 5x Denhardts solution (0.1% Ficoll, 0.14 M polyvinylpyridine, 0.1 % bovine serum albumin), 5xSSC, 50 µg/ml salmon sperm DNA and 50 µg/ml yeast RNA for 16 hours at 42 ° C. The filters were washed twice for 30 minutes in 1xSSC 0.1% SDS at 50 ° C and autoradiographed. A total of twenty hybridizing phage corresponding to human pre-proANVP cDNA clones were then identified.

4. Sequence analysis of the human pre-proANVP cDNA clone

Twelve of the positively hybridizing phage were chosen and were purified to homogeneity by replating. Phage DNA preps were made from these human pre-proANVP cDNA clones and the DNA was digested with EcoRI to determine the size of the cDNA insert. One clone, number 6, was thus shown to have an insert of about 700 base pairs and was chosen for DNA sequence analysis.

The EcoRI insert of clone number 6 was subcloned into phage M13 vector (Messing J. & Vieira, J., Gene 19 259-268 (1982)) and sequenced by the dideoxynucleotide chain termination method as described by Sanger, F. et al., supra.

The nucleotide sequence of the human pre-proANVP cDNA clone number 6 is shown in Figure 1B. The clone was compared to the rat pre-proANVP cDNA to confirm that it corresponds to human ANF pre-proANVP. This cDNA clone extends from the region corresponding to amino acid 15 through the human pre-proANVP coding region and contains all of the 3'-untranslated region. It therefore contains all of the sequences coding for the biologically active components of human proANVP and is suitable for expression in foreign systems.

III. Chemical Synthesis of Atrial Natriuretic/Vasodilatory Polypeptides

A. Synthesis Procedures

Compounds of the present invention were synthesized by solid-phase techniques. Synthesis were performed manually or, alternatively, on a Biosearch SAM II automated peptide synthesizer (Biosearch, San Rafael, California) using t-Boc amino acids in accordance with the instructions of the manufacturer.

In accordance with the above description, the following procedures were used for the chemical synthesis of novel ANVPs.

Procedure A

Preparation of Boc-$AA_n$-$AA_{n-1}$ ... $AA_1$ Hydroxymethyl Polystyrene Ester

One g of Boc-$AA_n$-O-Polystyrene-Resin (0.2-0.6 mmole/g subs) (Peninsula Labs, Inc.) was treated according to schedule A for incorporation of the Boc-$AA_1$-OH ... Boc-$AA_{n-1}$-OH.

Schedule A

1) Wash 3x with dichloromethane ($CH_2Cl_2$).
2) Treat for 1 min. with TFA:$CH_2Cl_2$:EDT (45:50:5 by volume)
3) Treat for 20 min. with TFA:$CH_2Cl_2$:EDT (45:50:5) by volume
4) Wash 3x with $CH_2Cl_2$
5) Treat 2x for 1 min. 10% (V/V) Diisopropylethylamine (DIPEA) in $CH_2Cl_2$
6) Wash 2x with $CH_2Cl_2$
7) Wash 2x with MeOH
8) Repeat (5-7) once
9) Wash 3x with $CH_2Cl_2$

10) Add 1-6 equivalents of preformed symmetrical anhydride of the suitably protected Boc-amino acid dissolved in $CH_2Cl_2$ or DMF/$CH_2Cl_2$ (50:50 volume). Boc-Asn-OH and Boc-Ala-OH were coupled with active esters of N-hydroxybenzotriazole.
11) Wash 2x with $CH_2Cl_2$
12) Wash 2x with 10% DIPEA
13) Wash 2x with $CH_2Cl_2$
14) Wash 2x with MeOH
15) Wash 2x with $CH_2Cl_2$
16) Repeat steps (11-15) once
17) Test by ninhydrin reaction according to Kaiser et al., Annal. Biochem. 34:595 (1970). If the reaction was incomplete, repeats steps (10-16) or, alternatively, cap synthesis using N-acetyl imidazole (0.30 M in DMF) or an excess of acetic anhydride in $CH_2Cl_2$.

Procedure B

Preparation of Boc-$AA_n$-p-Methylbenzhydrylamine resin

Boc-$AA_n$-OH is attached to a p-Methylbenzhydrylamine (pMBHA) resin via dicyclohexylcarbodiimide, as described below.

Schedule B

1) Wash the p-MBHA•HCl resin
2) Wash the resin 2x with 10% (V/V) DIPEA in $CH_2Cl_2$
3) Wash 2x with $CH_2Cl_2$
4) Wash 2x with MeOH
5) Wash 2x with $CH_2Cl_2$
6) Add 1-6 equivalents of preformed symmetrical anhydride of the suitably protected Boc-amino acid dissolved in $CH_2Cl_2$, with reaction time of 0.5 - 24 hrs.

Unreacted amino groups are acetylated with 0.30M N-acetylimidazole:-DMF, or acetic anhydride:$CH_2Cl_2$.

The first two examples demonstrate the chemical synthesis of the natural peptides that were isolated from rat atria (see Section I).

Example III.A.1: rANVP (126-150)

H-Arg-Ser-Ser-Cys-Phe-Gly-Gly-Arg-Ile-Asp-Arg-Ile-Gly-
Ala-Gln-Ser-Gly-Leu-Gly-Cys-Asn-Ser-Phe-Arg-Tyr-OH

One g of Boc L-Tyr(BrZ)O-Resin (Peninsula Labs Inc., 0.54 meq/g, Belmont, CA) was subjected to procedure A with the required sequence of amino acids (introduced in order as Loc-Arg(Tos)OH, Boc-PheOH, Boc-Ser(Bzl)OH, Boc-AsnOH, Boc-Cys(4-$CH_3$Bzl)OH, Boc-GlyOH, Boc-LeuOH•$H_2O$, Boc-GlyOH, Boc-Ser(Bzl)OH, Boc-GlnOH, BocAlaOH, Boc-GlyOH, Boc-IleOH•$\frac{1}{2}$ $H_2O$, Boc-Arg(Tos)OH, Boc-Asp(OBzl)-OH, Boc-IleOH•$\frac{1}{2}$ $H_2O$, Boc-Arg(Tos)OH, Boc-GlyOH, Boc-GlyOH, Boc-PheOH, Boc-Cys(4$CH_3$Bzl)OH, Boc-Ser(Bzl)OH, Boc-Ser(Bzl)OH, Boc-Arg(Tos)OH). The protected peptidyl resin was treated with TFA:$CH_2Cl_2$:EDT (45:50:5 v/v/v) for 1 min., then 20 min. and washed 3 times with $CH_2Cl_2$, 2 times with MeOH to give the TFA salt of the peptidyl resin and dried in vacuo.

The peptidyl resin was then suspended in anhydrous hydrogen fluoride (HF) containing 10% anisole, 2% Ethyl methyl sulfide for 30 min. at -10 °C, and 30 min. at 0 °C. The HF was removed by evaporation under vacuum and the peptide/resin mixture was suspended in diethyl ether. The peptide/resin mixture was washed twice with diethyl ether, once with chloroform, once with diethyl ether, once with chloroform and once with diethylether. The peptide was extracted from the mixture with 2.0 M acetic acid, diluted with $H_2O$ and lyophilized, to give the unoxidized dihydro peptide.

The crude peptide was dissolved in deoxygenated 0.01 M ammonium acetate ($NH_4OAc$), pH 8, to 0.5 mg/ml and then oxidized by dropwise addition of a slight excess of 0.01 M potassium ferricyanide (KCN)

solution stirred 20 minutes and pH adjusted to 5 with acetic acid. The peptide solution was treated with DOWEX AG3x4 anion exchange resin, filtered, diluted with $H_2O$ and lyophilized to give the crude cyclized peptide.

Purification of the peptide was achieved by desalting on Sephadex G-25F using 0.5 M AcOH as eluant, follwed by ion exchange chromatography on CM-Sepharose (Pharmacia Fine Chemicals) or CM-cellulose (Whatman) using a gradient elution generated by addition of 300 mM $NH_4OAc$, to a solution of 0.01 M $NH_4OAc$, pH 4.5. Fractions collected having a minimum 97% purity as judged by reversed phase HPLC were pooled and lyophilized from $H_2O$ several times to yield the purified rANVP (126-150) acetate salt.

Example III.A.2: rANVP (126-149)

H-Arg-Ser-Ser-Cys-Phe-Gly-Gly-Arg-Ile-Asp-Arg-Ile-Gly-
Ala-Gln-Ser-Gly-Leu-Gly-Cys-Asn-Ser-Phe-Arg-OH

1.2 g of Boc-Arg(Tos)O-Resin (Biosearch, Inc., San Rafael, CA) was subjected to procedure A with the required sequence of amino acids (introduced in order as Boc-PheOH, Boc-Ser(Bzl)OH, Boc-AsnOH, Boc-Cys(4-$CH_3$Bzl)OH, Boc-GlyOH, Boc-LeuOH•$H_2O$, Boc-GlyOH, Boc-Ser(Bzl)OH, Boc-GlnOH, BocAlaOH, Boc-GlyOH, Boc-IleOH•$\frac{1}{2}$ $H_2O$, Boc-Arg(Tos)OH, Boc-Asp(OBzl)OH, Boc-IleOH•$\frac{1}{2}$ $H_2O$, Boc-Arg(Tos)OH, Boc-GlyOH, Boc-GlyOH, Boc-PheOH, Boc-Cys(4$CH_3$Bzl)OH, Boc-Ser(Bzl)OH, Boc-Ser(Bzl)OH, Boc-Arg-(Tos)OH). The protected peptidyl resin was treated with TFA:$CH_2Cl_2$:EDT (45:50:5 v/v/v) for 1 min., then 20 min. and washed 3 times with $CH_2Cl_2$, 2 times with MeOH and dried in vacuo to give the TFA salt of peptidyl resin.

The peptidyl resin was then suspended in anhydrous HF containing 10% anisole, 2% Ethyl methyl sulfide for 30 min. at -10°C, and 30 min. at 0°C. The HF was removed by evaporation under vacuum and the peptide/resin mixture was suspended in diethyl ether. The peptide/resin mixture was washed twice with diethyl ether, twice with chloroform, and twice with diethyl ether. The peptide was extracted with 2.0 M acetic acid and lyophilized, to give the unoxidized dihydro peptide.

The crude peptide was dissolved in 2.0 M acetic acid, 10 mM 2-mercaptoethanol and was chromatographed on a Sephadex G-25SF column in the same solution. The peptide was then lyophillized and resuspended in a solution of 0.1 M $NH_4HCO_3$, pH 8.0 at a peptide concentration of 100 $\mu$g/ml. The suspended peptide was exposed to air for 48 hours to promote slow reoxidation. The peptide was then lyophillized.

The following example demonstrates the chemical synthesis of a human atrial natriuretic/vasodilator polypeptide.

Example III.A.3: hANVP (127-151)

H-Arg-Ser-Ser-Cys-Phe-Gly-Gly-Arg-Met-Asp-Arg-Ile-Gly-
Ala-Gln-Ser-Gly-Leu-Gly-Cys-Asn-Ser-Phe-Arg-Tyr-OH

One g of Boc L-Tyr(BrZ)O-Resin (Peninsula Labs Inc., 0.54 meq/g, Belmont, CA) was subjected to procedure A with the required sequence of amino acids (introduced in order as Boc-Arg(Tos)OH, Boc-PheOH, Boc-Ser(Bzl)OH, Boc-AsnOH, Boc-Cys(4-$CH_3$Bzl)OH, Boc-GlyOH, Boc-LeuOH•$H_2O$, Boc-GlyOH, Boc-Ser(Bzl)OH, Boc-GlnOH, BocAlaOH, Boc-GlyOH, Boc-IleOH•$\frac{1}{2}$ $H_2O$, Boc-Arg(Tos)OH, Boc-Asp(OBzl)-OH, Boc-MetOH•$\frac{1}{2}$ $H_2O$, Boc-Arg(Tos)OH, Boc-GlyOH, Boc-GlyOH, Boc-PheOH, Boc-Cys(4$CH_3$Bzl)OH, Boc-Ser(Bzl)OH, Boc-Ser(Bzl)OH, Boc-Arg(Tos)OH). The protected peptidyl resin was treated with TFA:$CH_2Cl_2$:EDT (45:50:5 v/v/v) for 1 min., then 20 min. and washed 3 times with $CH_2Cl_2$, and twice with MeOH to give the TFA salt of the peptidyl resin and dried in vacuo.

The peptidyl resin was then suspended in anhydrous hydrogen fluoride (HF) containing 10% anisole, 2% Ethyl methyl sulfide for 30 min. at -10°C, and 30 min. at 0°C. The HF was removed by evaporation under vacuum and the peptide/resin mixture was suspended in diethyl ether. The peptide/resin mixture was washed twice with diethyl ether, once with chloroform, once with diethyl ether, once with chloroform and

once with diethylether. The peptide was extracted from the mixture with 2.0 M acetic acid, diluted with $H_2O$ and lyophilized, to give the unoxidized dihydro peptide.

The crude peptide was dissolved in deoxygenated 0.01 M ammonium acetate ($NH_4OAc$), pH 8, to 0.5 mg/ml and then oxidized by dropwise addition of a slight excess of 0.01 M potassium ferricyanide (KCN) solution stirred 20 minutes and pH adjusted to 5 with acetic acid. The peptide solution was treated with DOWEX AG3X4 anion exchange resin, filtered, diluted with $H_2O$ and lyophilized to give the crude cyclized peptide.

Purification of the peptide was achieved by desalting on Sephadex G-25F using 0.5 M AcOH as eluant, followed by ion exchange chromatography on CM-Sepharose (Pharmacia Fine Chemicals) or CM-cellulose (Whatman) using a gradient elution generated by addition of 300 mM $NH_4OAc$, to a solution of 0.01 M $NH_4OAc$, pH 4.5. Fractions collected having a minimum 97% purity as judged by reversed phase HPLC were pooled and lyophilized from $H_2O$ several times to yield the purified hANVP (127-151) acetate salt.

Example III.A.4: rANVP (126-148) $NH_2$

H-Arg-Ser-Ser-Cys-Phe-Gly-Gly-Arg-Ile-Asp-Arg-Ile-Gly-
Ala-Gln-Ser-Gly-Leu-Gly-Cys-Asn-Ser-Phe-$NH_2$

One g of Boc-Phe-pMBHA-Resin was obtained using schedule B, with the required sequence of amino acids (introduced in order as Boc-Ser(Bzl)OH, Boc-AsnOH, Boc-Cys(4-$CH_3$Bzl)OH, Boc-GlyOH, Boc-LeuOH•$H_2O$, Boc-GlyOH, Boc-Ser(Bzl)OH, Boc-GlnOH, BocAlaOH, Boc-GlyOH, Boc-IleOH•$\frac{1}{2}$ $H_2O$, Boc-Arg-(Tos)OH, Boc-Asp(OBzl)OH, Boc-IleOH•$\frac{1}{2}$ $H_2O$, Boc-Arg(Tos)OH, Boc-GlyOH, Boc-GlyOH, Boc-PheOH, Boc-Cys(4-$CH_3$Bzl)OH Boc-Ser(Bzl)OH, Boc-Ser(Bzl)OH, Boc-Arg(Tos)OH).

The peptidyl resin was then suspended in anhydrous hydrogen fluoride (HF) containing 10% anisole, 2% Ethyl methyl sulfide for 30 min. at -10°C, and 30 min. at 0°C. The HF was removed by evaporation under vacuum and the peptide/resin mixture was suspended in diethyl ether. The peptide/resin mixture was washed twice with diethyl ether, once with chloroform, once with diethyl ether, once with chloroform and once with diethylether. The peptide was extracted from the mixture with 2.0 M acetic acid, diluted with $H_2O$ and lyophilized, to give the unoxidized dihydro peptide.

The crude peptide was dissolved in deoxygenated 0.01 M ammonium acetate ($NH_4OAc$), pH 8, to 0.5 mg/ml and then oxidized by dropwise addition of a slight excess of 0.01 M potassium ferricyanide (KCN) solution stirred 20 minutes and pH adjusted to 5 with acetic acid. The peptide solution was treated with DOWEX AG3X4 anion exchange resin, filtered, diluted with $H_2O$ and lyophilized to give the crude cyclized peptide.

Purification of the peptide was achieved by desalting on Sephadex G-25F using 0.5 M AcOH as eluant, followed by ion exchange chromatography on CM-Sepharose (Pharmacia Fine Chemicals) or CM-cellulose (Whatman) using a gradient elution generated by addition of 300 mM $NH_4OAc$, to a solution of 0.01 M $NH_4OAc$, pH 4.5. Fractions collected having a minimum 97% purity as judged by reversed phase HPLC were pooled and lyophilized from $H_2O$ several times to yield the purified rANVP (126-148) acetate salt.

Following the procedures outlined in Examples III.A.1-4, with appropriate modification, the following ANVPs were synthesized:

Example III.A.5: hANVP(127-146)

H-Arg-Ser-Ser-Cys-Phe-Gly-Gly-Arg-Met-Asp-Arg-Ile-Gly-
Ala-Gln-Ser-Gly-Leu-Gly-Cys-OH

Example III.A.6: [D-Cys129]rANVP(126-150)

H-Arg-Ser-Ser-[D-Cys]-Phe-Gly-Gly-Arg-Ile-Asp-Arg-Ile-
Gly-Ala-Gln-Ser-Gly-Leu-Gly-Cys-Asn-Ser-Phe-Arg-Tyr-OH

Example III.A.7: [D-Phe130]rANVP(126-150)

H-Arg-Ser-Ser-Cys-[D-Phe]-Gly-Gly-Arg-Ile-Asp-Arg-Ile-
Gly-Ala-Gln-Ser-Gly-Leu-Gly-Cys-Asn-Ser-Phe-Arg-Tyr-OH

Example III.A.8: [D-Ala131]rANVP(126-150)

H-Arg-Ser-Ser-Cys-Phe-[D-Ala]-Gly-Arg-Ile-Asp-Arg-Ile-
Gly-Ala-Gln-Ser-Gly-Leu-Gly-Cys-Asn-Ser-Phe-Arg-Tyr-OH

Example III.A.9: [D-Ala132]rANVP(126-150)

H-Arg-Ser-Ser-Cys-Phe-Gly-[D-Ala]-Arg-Ile-Asp-Arg-Ile-
Gly-Ala-Gln-Ser-Gly-Leu-Gly-Cys-Asn-Ser-Phe-Arg-Tyr-OH

Example III.A.10: [D-Arg133]rANVP(126-150)

H-Arg-Ser-Ser-Cys-Phe-Gly-Gly-[D-Arg]-Ile-Asp-Arg-Ile-
Gly-Ala-Gln-Ser-Gly-Leu-Gly-Cys-Asn-Ser-Phe-Arg-Tyr-OH

Example III.A.11: [D-Met135]hANVP(127-151)

H-Arg-Ser-Ser-Cys-Phe-Gly-Gly-Arg-[D-Met]-Asp-Arg-Ile-
Gly-Ala-Gln-Ser-Gly-Leu-Gly-Cys-Asn-Ser-Phe-Arg-Tyr-OH

Example III.A.12: [D-Val$^{134}$]rANVP(126-150)

H-Arg-Ser-Ser-Cys-Phe-Gly-Gly-Arg-[D-Val]-Asp-Arg-Ile-Gly-Ala-Gln-Ser-Gly-Leu-Gly-Cys-Asn-Ser-Phe-Arg-Tyr-OH

Example III.A.13: [D-Arg$^{136}$]rANVP(126-150)

H-Arg-Ser-Ser-Cys-Phe-Gly-Gly-Arg-Ile-Asp-[D-Arg]-Ile-Gly-Ala-Gln-Ser-Gly-Leu-Gly-Cys-Asn-Ser-Phe-Arg-Tyr-OH

Example III.A.14: [D-Val$^{137}$]rANVP(126-150)

H-Arg-Ser-Ser-Cys-Phe-Gly-Gly-Arg-Ile-Asp-Arg-[D-Val]-Gly-Ala-Gln-Ser-Gly-Leu-Gly-Cys-Asn-Ser-Phe-Arg-Tyr-OH

Example III.A.15: [D-Ala$^{138}$]rANVP(126-150)

H-Arg-Ser-Ser-Cys-Phe-Gly-Gly-Arg-Ile-Asp-Arg-Ile-[D-Ala]-Ala-Gln-Ser-Gly-Leu-Gly-Cys-Asn-Ser-Phe-Arg-Tyr-OH

Example III.A.16: [D-Ala$^{139}$]rANVP(126-150)

H-Arg-Ser-Ser-Cys-Phe-Gly-Gly-Arg-Ile-Asp-Arg-Ile-Gly-[D-Ala]-Gln-Ser-Gly-Leu-Gly-Cys-Asn-Ser-Phe-Arg-Tyr-OH

Example III.A.17: [D-Gln$^{140}$]rANVP(126-150)

H-Arg-Ser-Ser-Cys-Phe-Gly-Gly-Arg-Ile-Asp-Arg-Ile-Gly-Ala-[D-Gln]-Ser-Gly-Leu-Gly-Cys-Asn-Ser-Phe-Arg-Tyr-OH

Example III.A.18: [D-Ser$^{141}$]rANVP(126-150)

H-Arg-Ser-Ser-Cys-Phe-Gly-Gly-Arg-Ile-Asp-Arg-Ile-Gly-Ala-Gln-[D-Ser]-Gly-Leu-Gly-Cys-Asn-Ser-Phe-Arg-Tyr-OH

Example III.A.19: [D-Ala$^{142}$]rANVP(126-150)

H-Arg-Ser-Ser-Cys-Phe-Gly-Gly-Arg-Ile-Asp-Arg-Ile-Gly-
Ala-Gln-Ser-[D-Ala]-Leu-Gly-Cys-Asn-Ser-Phe-Arg-Tyr-OH

Example III.A.20: [D-Leu$^{143}$]rANVP(126-150)

H-Arg-Ser-Ser-Cys-Phe-Gly-Gly-Arg-Ile-Asp-Arg-Ile-Gly-
Ala-Gln-Ser-Gly-[D-Leu]-Gly-Cys-Asn-Ser-Phe-Arg-Tyr-OH

B. Biological Activity of Native and Chemically Produced Atrial Natriuretic/Vasodilator Polypeptides

The biologic activities of native and chemically synthesized ANVP compounds and analogs were determined using in vitro systems, including isolated rat kidneys, isolated rabbit thoracic aortic rings and isolated vascular wall cells. The activities were also measured in intact rats and dogs.

1. In Vitro Biological Assays

The activity of rANVP(126-149) was measured in the isolated rat kidney. Functioning isolated rat kidneys were perfused in a closed-circuit system, as described in Camargo, M.J.F. et al. Am.J.Physiol., 246:F447-F456 (1984). After control clearance periods, 0.1 to 1.0 µg of the compound was added to the perfusate. The effects on multiple parameters were noted. These peak values are expressed in Table I as the experimental data. It is clear that rANVP(126-149) caused urine flow rate, urinary sodium excretion, filtration fraction and glomerular filtration rate to increase.

Table I

| Effects of Synthetic Atrial natriuretic/vasodilator polypeptide rANVP(126-149) on Renal Function in the Isolated Perfused Rat Kidney | | |
|---|---|---|
| | Control | Experimental |
| GFR (ml/min) | 0.43 ± 0.05 | 0.63 ± 0.03* |
| FF | 0.014 ± 0.002 | 0.021 ± 0.001* |
| RVR (mmHg/ml/min) | 2.9 ± 0.1 | 3.9 ± 0.3* |
| $\dot{V}$ (µl/min) | 19.8 ± 4.8 | 97.6 ± 19.4* |
| $FL_{Na}$ (µEq/min) | 60.2 ± 7.9 | 90.1 ± 5.2* |
| $T_{Na}$ (µEq/min) | 60.0 ± 7.6 | 84.2 ± 4.8* |
| $U_{Na}\dot{V}$ (µEq/min) | 0.66 ± 0.35 | 6.01 ± 1.99* |
| $FE_{Na}$ (%) | 0.97 ± 0.38 | 6.6 ± 2.0* |
| $U_K\dot{V}$ (µEq/min) | 0.44 ± 0.19 | 1.46 ± 0.16* |
| $FE_K$ (%) | 19.8 ± 5.9 | 52.1 ± 6.2* |

GFR, glomerular filtration rate; FF, filtration fraction; RVR, renal vascular resistance; $\dot{V}$, urine flow rate; $FL_{Na}$, filtered load of sodium; $T_{Na}$, tubular reabsorption of sodium; $U_{Na}\dot{V}$, urinary sodium excretion rate; $FE_{Na}$, fractional sodium excretion; $U_K\dot{V}$, urinary potassium excretion rate; $FE_K$, fractional potassium excretion. Results are the mean ± SE of 4 kidneys.

*P<0.05 compared to control (Student's t test).

These results also show that in isolated kidneys perfused in the absence of vasoconstrictors, the compounds of the present invention increased renal vascular resistance, filtration fraction and glomerular filtration rate. In contrast, in isolated kidney precontracted with endogenously generated angiotensin, the compounds decreased vascular resistance. These effects with the synthetic compounds show that ANVPs can have both renal vasoconstrictive and vasorelaxant activity depending on the absence or presence of endogenous vasoconstrictors. The natriuresis observed in the isolated kidney can result from a renal vasoconstrictive effect preferentially expressed in the efferent arteriole.

In a similar manner, the effects of other ANVPs were examined in the isolated rat kidney. Table II summarizes the effects of these peptides on urinary flow rate, urinary sodium excretion and glomerular filtration rate. It should be noted that all ANVP's tested in this system increased urine flow rate and urinary sodium excretion. Relatively few unrelated peptides effect flow rate and sodium excretion in this manner in the isolated rat kidney. Thus the observed effects are specific to the ANVPs and related analogs. These effects are presumably mediated by the interaction of the ANVPs with specific receptor sites within the kidney.

Table II

Effects of Atrial Natriuretic/Vasodilator Polypeptides in the Isolated Rat Kidney

| | | (μl/min) $\dot{V}$ | (μEq/min) UNa$\dot{V}$ | (ml/min) GFR |
|---|---|---|---|---|
| (1μg) rANVP(126-150) | C | 11.8 | 0.37 | 0.35 |
| | E | 97.3 | 5.99 | 0.72 |
| (1μg) hANVP(127-151) | C | 25.2 | 0.72 | 0.67 |
| | E | 142.3 | 8.86 | 0.94 |
| (1μg) [D-Ala$^{138}$]rANVP (126-150) | C | 5.9 | 0.11 | 0.18 |
| | E | 57.5 | 2.53 | 0.54 |
| (1μg) [D-Ala$^{139}$]rANVP (126-150) | C | 7.8 | 0.25 | 0.22 |
| | E | 112.5 | 8.14 | 0.59 |
| (1μg) [D-Gln$^{140}$]rANVP (126-150) | C | 10.2 | 0.70 | 0.24 |
| | E | 183.4 | 14.52 | 1.01 |
| (1μg) [D-Ser$^{141}$]rANVP (126-150) | C | 0.42 | 0.10 | 0.20 |
| | E | 29.2 | 2.10 | 0.51 |

| | | | | |
|---|---|---|---|---|
| (0.1μg) [D-Ala[142]]rANVP (126-150) | C | 12.2 | 0.26 | 0.37 |
| | E | 58.5 | 2.50 | 0.75 |
| (1μg) [D-Leu[143]]rANVP (126-150) | C | 14.0 | 0.35 | 0.34 |
| | E | 103.0 | 3.52 | 0.53 |

Isolated rat kidneys were treated as described in Table I. Control periods for each compound are denoted by C and experimental periods following the addition of compounds are denoted by E. $\dot{V}$-urine flow rate $U_{Na}\dot{V}$-urinary sodium excretion rate, GFR-glomerular filtration rate. Data represent the mean of 3-8 experiments.

Since the native ANVPs relax precontracted blood vessels, the effects of these peptides were studied on isolated thoracic aoetic rings from rabbit and on isolated vascular wall cells of bovine origin.

The synthetic ANVPs were compared to partially purified (gel filtration step) and purified (HPLC) native ANVPs for their ability to relax histamine-contracted aortic rings. Rings were suspended in 10 ml aerated Kreb's buffer under 1.5 g passive tension. Rings were precontracted with 5 X $10^{-6}$ M histamine, washed and allowed to return to baseline tension, as described (Kleinert, H. D. (1984), supra). Increasing amounts of purified ANVPs or synthetic compounds were then added in cumulative fashion. The change in tension was shown to be related to the cumulative amount of protein added, as seen in Figure 8.

As shown, both purified native rANVP(126-150) and synthetic rANVP(126-150) relaxed the tissue at the same doses. In addition, hANVP(127-151) is equivalent to rANVP(126-150) in this tissue. However, rANVP-(127-150), the equivalent of atriopeptin III described by Geller, et al., Biochem. Biophys. Res. Comm. 120-(2):333-338 (1984), is significantly less potent in relaxing the precontracted rabbit aorta. This observation has recently been confirmed by Garcia, et al., Biochem. Biophys. Res. Comm. 126(1):178-184 (1985).

In order to relax precontracted aortic vessels, the ANVP compounds must first bind to specific membrane receptors. Associated with this ligand-receptor interaction is an increase in intracellular cyclic GMP (Winquist et al., Proc. Natl. Acad. Sci. USA, 81:7661-7664 (1984)). Since cyclic GMP has been identified as the intracellular mediator of vasorelaxation in responses to ANVP and other compounds, assessment of cyclic GMP levels provides an additional marker for determining the biological actions of ANVP compounds. Therefore, cells of vascular wall origin, e.g. bovine aortic smooth muscle and endothelial cells, were used to determine binding of ANVP compounds to specific receptors and associated cyclic GMP increases were measured. These tests will reflect the relative potency of the ANVPs in relaxing bovine vascular smooth muscle.

Binding assays were performed as follows: ANVPs were labelled with $^{125}$I according to the procedure of Schenk, et al., J. Biol. Chem., 259:14941-14951 (1984). This procedure utilizes glucose oxidase and lactoperoxidase mediated oxidations to transfer $^{125}$I to the tyrosine on the ANVP molecule. $^{125}$I-ANVP was then added to 10 mm confluent cultures of vascular wall cells and incubated at 37°C.

As shown in Figure 9, the $^{125}$I-ANVP binds to the cells in a specific and saturable manner. Specific and saturable binding is a necessary component of a hormone/receptor interaction. Furthermore, $^{125}$I-ANVP binding can be displaced by unlabelled ANVP compounds and not by a variety of hormones (angiotensin, epinephrine, vasopressin, glucagon) that act at other receptor sites. Thus, the displacement of $^{125}$I-ANVP binding by ANVPs and analogs as a function of concentration is a reflection of their potency to bind to the vascular wall and alter the contractile state of the vessel. Concentrations at which ANVPs and related analogs displace $^{125}$I-ANVP from binding sites on vascular smooth muscle cells are outlined on Table III.

Table III

| Binding of Atrial Natriuretic/vasodilator Polypeptides to Cultured Bovine Vascular Smooth Muscle Cells | |
|---|---|
| Peptide | Ki(nM) |
| hANVP(127-151) | 0.5 |
| [D-Ala$^{142}$]rANVP(126-150) | 0.6 |
| rANVP(126-150) | 0.8 |
| rANVP(127-150) | 2.4 |
| hANVP(128-151) | 7.0 |
| [D-Ala$^{138}$]rANVP(126-150) | 20.0 |
| [D-Gln$^{140}$]rANVP(126-150) | 20.0 |
| [D-Ser$^{141}$]rANVP(126-150) | 40.0 |
| rANVP(126-150) was iodinated as described and incubated with cultured smooth muscle cells in the presence of representative peptides listed above. Various concentrations of the peptides described were examined for their ability to displace specific $^{125}$I-rANVP(126-150) binding. The concentration at which half-maximal displacement was observed is reported. | |

These data demonstrate that of the disclosed embodiments [D-Ala$^{142}$]rANVP(126-150), rANVP(126-150) and hANVP(127-151) are most reactive with vascular wall cells.

Since the agonistic action of ANVP in vascular smooth muscle not only results from binding to specific receptors, but is also associated with increases in cyclic GMP, the effects of ANVPs and analogs on cyclic GMP accumulation were examined using the same cultured cells. Figure 10 demonstrates the magnitude of ANVP-mediated cyclic GMP increases in cultured aortic smooth muscle and endothelial cells. In Figure 11A & B, the relative activities of ANVP and analogs on cyclic GMP accumulation in aortic smooth muscle and endothelial cells are shown. The data is plotted in a dose-response relationship (log dose vs % of maximal cyclic GMP increases).

Once again, a distribution in potency over three orders of magnitude can be seen. The rank order of potency in both cell types is [D-Ala$^{142}$]rANVP(126-150) = [D-Ala$^{131}$]rANVP(126-150) = rANVP(126-150) = rANVP(126-149) = hANVP(127-151) > rANVP(127-150) = other D amino acid substitutions. These data will reflect the vasodilatory potency of these peptides in bovine aorta. Thus, the data demonstrate that certain D amino acid substitution increase potency, while others decrease potency. Furthermore, the data imply that removal of the carboxy terminal tyrosine residue has little effect on vascular reactivity. Furthermore, the data confirm the observation that the amino terminal arginine residue is important for maximal reactivity.

2. In Vivo Assays

Synthetic ANVP compounds rANVP(126-149), rANVP(126-150) and hANVP(127-151) were also found to be natriuretic in the intact rat. These compounds were administered as a bolus injection to Inactin anesthetized rats (100 mg/kg, average weight 399 g) which were maintained on a constant infusion of normal saline at 2.2 ml/hr. The results were as shown in Table IV, wherein the change in each parameter was assessed by the difference between the average of three control periods (10 mins. each) and the first experimental period (maximum response). Data are expressed as mean ± SE.

Table IV

| Natriuretic Effect of Synthetic Atrial natriuretic/vasodilator polypeptides Compound in Intact Rats | | | |
|---|---|---|---|
| Dose (µg/kg) | $\dot{V}$ (µl/min) | $U_{Na}\dot{V}$ (µEq/min) | $U_K\dot{V}$ (µEq/min) |
| 1.2 (n=4) | 25.5 ± 9.7 | 2.5 ± 1.1 | 1.6 ± 0.2 |
| 2.6 (n=4) | 41.3 ± 19.5 | 6.7 ± 4.1 | 4.4 ± 1.4 |
| 5.0 (n=4) | 52.8 ± 6.5 | 9.1 ± 1.0 | 3.7 ± 0.5 |
| 7.2 (n=3) | 112.0 ± 12.8 | 18.3 ± 0.5 | 3.1 ± 0.8 |
| $\dot{V}$, urine flow rate; $U_{Na}\dot{V}$, urinary sodium excretion rate; $U_K\dot{V}$, urinary potassium excretion rate. Control values for the 15 animals were: $\dot{V}$, 10.3 ± 2.9 µl/min; $U_{Na}\dot{V}$, 0.93 ± 0.5 µEq/min; and $U_K\dot{V}$, 1.6 ± 0.4 µEq/min. | | | |

Renal and hemodynamic effects have also been measured in anesthetized dogs receiving a constant infusion of the compound rANVP(126-149) (1 µg/kg bolus, followed by 0.1 µg/kg/min for 1 hour). Immediate effects were noted on blood pressure, GFR and urine flow rate and electrolyte excretion which were sustained throughout the infusion. The "experimental" data presented for these parameters in Table IV are the average values obtained during the infusion. Mean arterial pressure (MAP) fell consistently by 10-15% while GFR rose by 25-35%, in association with a sustained diuresis and natriuresis (Table V). All these parameters returned to control (i.e. pre-infusion) levels during the recovery period (i.e. following termination of the infusion).

Table V

Hemodynamic, Renal and Metabolic Effects Of Synthetic Atrial natriuretic/vasodilator polypeptides Compounds in Anesthetized Dogs

| | Control | Experimental | Recovery |
|---|---|---|---|
| MAP (mm Hg) | 134 ± 5 | 122 ± 4*$^+$ | 136 ± 4 |
| GFR (ml/min) | 25.5 ± 2.7 | 32.3 ± 4.1*$^+$ | 25.4 ± 3.3 |
| $\dot{V}$ (ml/min) | 0.21 ± 0.03 | 1.06 ± 0.14*$^+$ | 0.37 ± 0.05 |
| $FE_{H_2O}$ (%) | 0.9 ± 0.2 | 3.4 ± 0.3*$^+$ | 1.5 ± 0.2 |
| $U_{Na}\dot{V}$ (µEq/min) | 38 ± 6 | 187 ± 35*$^+$ | 68 ± 14 |
| $FE_{Na}$ (%) | 1.1 ± 0.2 | 4.1 ± 0.5*$^+$ | 1.9 ± 0.4 |
| $U_K\dot{V}$ (µEq/min) | 15 ± 2 | 36 ± 6*$^+$ | 21 ± 4 |
| $FE_K$ (%) | 18 ± 1 | 34 ± 6*$^+$ | 21 ± 4 |
| PRA (ng/ml/hr) | 13 ± 2.0 | 8.3 ± 1.8*$^+$ | 14 ± 2.5 |
| PA (ng/100 ml) | 8.5 ± 1.9 | 5.4 ± 0.9* | 7.0 ± 1.3 |

MAP, mean arterial pressure (blood pressure); $FE_{H_2O}$, fractional water excretion; PRA, plasma renin activity; PA, plasma aldosterone. For definition of other abbreviations see footnote to Table I. *P<0.05 compared to control; $^+$P<0.05 compared to recovery.

The peptide produced significant decreases in plasma renin activity (PRA) and plasma aldosterone (PA), as shown in Table V. Additional studies demonstrate that this substance also inhibits the ability of angiotensin to stimulate aldosterone production by isolated adrenal cells. Thus, ANVPs are able to block the effects of the renin-angiotensin system at several levels: (1) they antagonize the direct actions of angiotensin on its target organs (blood vessels and the adrenal); and (2) inhibit renin secretion, which leads to a reduced rate of angiotensin formation in the blood.

Based on the above data, it is evident that the synthetic and tissue derived ANVP compounds possess similar activity, desirably after the synthetic ANVP compounds have been allowed to oxidize to promote the formation of disulfide bridges.

It is also evident from the above results that the subject compounds can be used as a potent vasorelaxant, diuretic and natriuretic in mammalian hosts.

IV. Expression of recombinant DNA derived atrial Natriuretic/vasodilatory polypeptides

A. Expression of proatrial natriuretic/vasodilator polypeptides and related fragments in Escherichia coli

In the examples that follow the expression of prorANVP (87-152) prorANVP (25-152), prohANVP(26-151) and prohANVP(102-151) in E. coli is described. In addition, the expression vectors are also described. It should be understood that these are illustrative examples, without implying any limitations and other pre-proANVPs, proANVPs or ANVP's could be expressed in a similar manner.

1. Construction of E. coli expression vector

a. Construction of pKT52 bacterial expression plasmid.

i) Generation of the trc promoter

Plasmid pEA300 (Amman, E. et al., Gene 25:167-178, 1983) was digested with PvuII and ClaI (New England Biolabs, Inc.). The digest was electrophoresed in a 0.8% agarose gel as described by Maniatis, T. et al. supra at p. 157-160. The large fragment containing the -35 nucleotide region of the trp promoter near the ClaI site was detected by UV-shadowing as described by Maniatis et al., supra at p. 167, and eluted from the gel at 37° as described by Maxam, A. and W. Gilbert, Methods in Enzymology, 65:449-560 (1980). The ClaI site of the large fragment was filled in with 50 M dCTP as described in Maniatis et al., supra at p. 394, and the remaining single-stranded 5' overhang removed by digestion with mung bean nuclease (Pharmacia P-L Biochemicals, Inc.) as described by Kroeker, W. et al., Biochemistry 17:3236-3239 (1978). Plasmid pGL101 (Lauer, G. et al., J Mol. Appl. Genet. 1:139-147, 1981) was digested with PvuII and HpaII (New England Biolabs) as described. The digested fragments filled in by the method of Maniatis et al., supra at p. 394, with 5 units of Klenow fragment (Boehringer-Mannheim, Inc., Mannheim, FRG) and the addition of 1 Ci [ - $^{32}P$]-dCTP (Amersham, Chicago, Illinois, 800 Ci/mM) for 15 minutes at 37°C. This was followed by the addition of dCTP and dGTP to 50 M for 30 minutes at 37°C. The labeled, blunt-ended fragments were electrophoresed on a 12% polyacrylamide gel followed by wet gel autoradiography, and the 55 base pair blunted HpaII-PvuII fragment cut out of and eluted from the gel as described. The two isolated fragments were ligated as described in Maniatis et al., supra at p. 392, and used to transform E. coli strain RB791 (R. Brent and M. Ptashne, Proc. Natl. Acad. Sci. USA 78:4204-4208, 1981) as described in Maniatis, et al., supra at p. 250-251. The resulting plasmid, pKK10-0 contains the modified promoter, called the trc promoter, and was isolated by the rapid boiling method as described in Maniatis, et al., supra at pp. 366-367. PKK10-0 was digested with EcoRI (Bethesda Research Labs, Inc.), and used to transform E. coli RB791 as described above. This plasmid, PKK10-1, was isolated as described and digested with PvuII. The PvuII digested plasmid was ligated to the NcoI linker (dACCATGGT, Creative Biomolecules, Inc. Foster City, California), digested with NcoI, filled in with dATP, dCTP, dGTP, and dTTP, and ligated as described to a linker containing PstI and HindIII sites synthesized as two complementary oligonucleotides (5' -dGCTGCAG-CCAAGCTTGG-3' and 5'dCCAAGCTTGGCTGCAGC-3') on a Biosearch SAM I DNA Synthesizer (Biosearch, Inc.). The ligation mixture was digested with BamHI and HindIII (New England Biolabs), electrophoresed on a 5% polyacrylamide gel, and the small BamHI - HindIII fragment eluted as described above. This fragment contains the trc promoter.

ii) Construction of the trc promoter plasmid, pKT52

PKK10-2 (Brosius, J., Gene 27:161-172, 1984) was digested with BamHI and HindIII. The large fragment was isolated from a 0.8% agarose gel and ligated to the trc promoter fragment described above. The ligation was used to transform E. coli RB791 and the new plasmid, pKK233-1 was isolated as described. PKK233-1 was digested to completion with PvuI and partially digested with BglI in accordance with Maniatis et al., supra at p. 381. At the same time, pUC8 (Vieira, J. and J. Messing, Gene 19, supra) was digested with PvuI and BglI and the 360 base pair PvuI-BglI fragment from the ampicillin resistance gene (that no longer contains a PstI site) was isolated from a 5% polyacrylamide gel. This fragment was mixed with the PvuI-BglI partial digestion mix of pKK233-1, ligated and used to transform E. coli RB791. Transformants were screened for the presence of only one PstI site and checked with a EcoRI-PstI digestion such that the remaining PstI site was next to the trc promoter, generating plasmid pKK233-2. Plasmid pKK233-2 was digested with EcoRI and PvuII, filled in with dATP and TTP, ligated, and transformed into E. coli RB791. The resulting vector is pKT52 (Figure 12A).

b. Construction of pTrp-233 bacterial expression plasmid

i) Construction of the synthetic tryptophan operon promoter and operator regulatory sequence.

The ten oligodeoxynucleotides shown in Figure 12E were synthesized and purified as described above. 500 pmole of each oligodeoxynucleotide except 1 and 10 were phosphorylated individually in 20 $\mu$l containing 60 mM Tris-Cl, pH 8, 15 mM DTT, 10 mM $MgCl_2$, 20 $\mu$ci of - $[^{32}P]\gamma$-ATP and 20 units of polynucleotide kinase (P/L Biochemicals) for 30 min. at 37°C. This was followed by the addition of 10 $\mu$l containing 60 mM Tris-HCl, pH 8, 15 mM DTT, 10 mM $MgCl_2$, 1.5 mM ATP and 20 additional units of polynucleotide kinase followed by another 30 min. incubation at 37°C. Following incubation the samples were incubated at 100°C for 5 min. 500 pmole of oligodeoxynucleotides 1 and 10 were diluted to 30 $\mu$l in the above buffer without ATP.

16.7 pmole of each oligodeoxynucleotide constituting a double standard pair (e.g. oligodeoxynucleotides 1 and 2, 3 and 4 etc. Figure 12E) were mixed and incubated at 90°C for 2 min. followed by slow cooling to room temperature. Each pair was then combined with the others in the construction and extracted with phenol/chloroform followed by ethanol precipitation. The oligodeoxynucleotide pairs were reconstituted in 30 $\mu$l containing 5 mM Tris-HCl, pH 8, 10 mM $MgCl_2$, 20 mM DTT, heated to 50°C for 10 min. and allowed to cool to room temperature followed by the addition of ATP to a final concentration of 0.5 mM followed by the addition of 800 units of T4 DNA ligase and incubated at 12.5°C for 12-16 hours.

The ligation mixture was extracted with phenol/chloroform and the DNA ethanol precipitated. The dried DNA was reconstituted in 30 $\mu$l and digested with EcoR1 and PstI for 1 hour at 37°C. The mixture was extracted with phenol/chloroform and ethanol precipitated followed by separation of the various double stranded DNA segments by electrophoresis on an 8% polyacrylamide gel as described previously. The DNA fragments were visualized by wet gel autoradiography and a band corresponding to approximately 100 bp in length was cut out and eluted overnight as described. The excised synthetic DNA fragment was ligated to plasmids M13-mp8 and M13-mp9 (Messing, J. and Vieria, J. supra) similarly digested with EcoRI and PstI and submitted to dideoxynucleotide sequence analysis (Sanger et al. supra) to confirm the designed sequence shown in Figure 12E. This sequence shown in Figure 12E contains the promoter and operator regions of the tryptophan operon (trp) (-35 and -10 regions in Figure A) as well as the ribosome binding region (S.D. region in Figure 12E) of the tryptophan operon leader peptide. Analagous sequences to that shown in Figure 12E have been proven to be useful in the expression of heterologous proteins in E. coli (Hallewell, R.A. and Emtage, S. Gene 9:27-47 (1980), Ikehara, M. et al. Proc. Natl. Acad. Sci. USA 81:5956-5960 (1984)).

ii) Construction of the synthetic trp promoter/operator containing plasmid pTrp-233

Plasmid PKK233-2 (see Section IV.A.1.a.ii.) was digested to completion with NdeI followed by the filling in of the termini by the method of Maniatis et al., supra at p. 394, with 5 units of E. coli polymerase I, Klenow fragment (Boehringer-Mannheim, Inc.) and the addition of dATP, dCTP, dGTP and TTP to 50 $\mu$M. This was incubated at 25°C for 20 min. Following phenol/chloroform extraction and ethanol precipitation, NdeI digested DNA was ligated and transformed into E. coli (Nakamura, K. et al. supra). The resulting plasmid lacking the NdeI site was designated pKK-233-2-Nde.

Twenty nanograms of plasmid pKK-233-2-Nde was digested to completion with EcoRI and PSTI followed by calf intestinal phosphatase treatment (Boehringer Mannheim) in accordance with Maniatis et al., supra at pp. 133-134. Fifty nanograms of the synthetic trp promoter/operator sequence described above with its cohesive 5'-EcoRI and 3'-PstI termini were mixed with ten nanograms of EcoRI - PstI digested pKK-233-2-Nde and ligated with T4-DNA-ligase as described followed by transformation into E. coli JA221 lpp$^-$/I'lacI$^9$. Transformants were screened for the presence of plasmid DNA containing the 100 bp EcoRI - PstI synthetic trp promoter/operator which was isolated and designated pTRP-233 and shown in Figure 12F.

2. Expression of cloned cDNA encoding rat proatrial natriuretic/vasodilator polypeptides

a) Construction of plasmid pRNF-6852

Plasmid pNF1 (see above) was digested to completion with HincII followed by extraction with phenol/chloroform and ethanol precipitation An NcoI decamer linker (dAGCCATGGCT) was synthesized on a SAM I DNA Synthesizer (Biosearch Inc.), purified by preparative gel electrophoresis as described and phosphorylated at it's 5' end with T4-polynucleotide kinase (P-L Biochemicals) using the procedure of Maniatis et al., supra at p. 396. The phosphorylated linker was attached to HincII digested pNFI by blunt-end ligation with T4-DNA ligase at 12.5 °C for 16 hrs.

Following an incubation at 65 °C for 5 min. the ligation mixture was adjusted to 100mM NaCl and incubated for 2 hrs. at 37 °C with NcoI and PstI followed by 5% polyacrylamide gel electrophoresis as described. The separated DNA was visualized by wet gel autoradiography followed by excision of a 316 bp band, elution and ethanol precipitation as described. (Maxam, A. and W. Gilbert, supra).

The expression plasmid, pKT52 was digested to completion with NcoI and PstI followed by treatment with calf intestinal phosphatase (Boehringer Mannheim), in accordance with Maniatis, et al., supra at pp. 133-134. The purified 316 bp NcoI-PstI fragment derived from pNF1, was mixed with NcoI-PstI digested pKT52 and incubated with T4-DNA-ligase for 30 min. at 25 °C and 4 hours at 12.5 °C. E. coli strain JA221 (1pp$^-$, hsd M$^+$, trpE5, leuB6, lacY, recA1/F', lacI$^q$, lacZ$^+$, proA$^+$, proB$^+$, Nakamura, K. et al., J. Mol. Appl. Genet. 1:289-299 (1982)) was made competent for transformation by the $CaCl_2$ method and transformed with the ligation mixture as described in Maniatis et al., supra at pp. 250-251. Resulting ampicillin resistant colonies were grown overnight in 1 ml from which plasmid DNA was prepared by the alkaline lysis method (Maniatis, et al., supra at pp 368-369). Plasmids were screened for the correct insert by digestion with first HindIII followed by KpnI or NcoI. A plasmid having both HindIII-KpnI and HindIII-NcoI fragments of approximately 120 bp and 320 bp respectively, was chosen and designated pRNF-6852 (Figure 12B).

To confirm that the reading frame of the cloned proANVP sequence in pKT-52 was correct, pRNF-6852 was digested with EcoRI and PstI followed by purification of a band of approximately 509 bp by polyacrylamide gel electrophoresis as described above. The EcoRI-PstI fragment was ligated to plasmids M13mp8 and M13mp9 (Messing, J. and J Vieria, supra) and submitted to dideoxynucleotide sequence analysis (Sanger et al., supra).

As shown in Figure 12B, plasmid pRNF-6852 was designed to express a fragment of the rat proANVP cDNA which encodes a protein from amino acids 87 to 152.

Because a synthetic decamer NcoI linker was used to allow cloning of the proANVP cDNA into the expression vector pKT52, the first two amino terminal amino acids of the expressed fragment are $NH_2$- Met-Ala followed by amino acids 87 through 152 of the rat proANVP precursor (Figure 12B).

b) Expression of plasmid pRNF-6852

E. coli JA221 lpp$^-$/F' lacI$^q$ containing pRNF-6852 or pKT52 were grown at 37 °C in media containing M9 minimal salts (Miller, J., Experiments in Molecular Genetics, Cold Spring Harbor Laboratory, Cold Spring Harbor, New York) supplemented with glucose (0.4%), thiamine (2 $\mu$g/ml), $MgS0_4 \cdot 7H_20$ (200 $\mu$g/ml) leucine (20 $\mu$g/ml), tryptophan (20 $\mu$g/ml), ampicillin (100 $\mu$g/ml), and isopropyl-1-thio-$\beta$-D-galactopyranoside (2 mM). At a cell density of approximately 2.5 x $10^8$ cells/ml, L-[$^{35}$S]-cysteine (100$\mu$Ci/ml culture (Amersham Corp., Chicago, Illinois 930 Ci/mmole)), was added. Following 30 sec of incubation, 1 ml of culture was removed and added to 0.34 ml of ice-cold 20% trichloroacetic acid in a 1.5 ml Eppendorf centrifuge tube, vortexed and allowed to stand at 0 °C for 30 min. The mixture was then centrifuged at 4 °C for 15 min in an Eppendorf centrifuge at 15,000 x g. The supernatant was discarded and the pellet washed with 1 ml of ice-cold acetone followed by centrifugation and drying of the resulting pellet in vacuo.

An IgG fraction was prepared from 1 ml of non-immune serum or anti-serum (raised against a chemically synthesized rat ANVP peptide) using Protein A-Sepharose® 4B (Pharmacia Fine Chemicals,

Uppsala, Sweden) chromatography as described in the manufacturer's specifications and collected in a total volume of 4 ml.

The dried TCA pellet was resuspended in 40 μl of 50 mM Tris-Cl, pH 8.0, 1 mM EDTA, and 1% SDS and incubated at 100 °C for 5 min. Ten μl of this mixture (representing total bacterial protein) was diluted to 20 μl with 20 mM Tris-HCl, pH 6.8, 22% glycerol, 2% SDS, 2% 2-mercaptoethanol, and 0.1% bromphenol blue, followed by incubation at 100 °C for 5 min. The remaining 30 μl (used for immunoabsorption) of the mixture was diluted to 1 ml with 50 mM Tris-HCl, pH 8.0, 1 mM EDTA, 0.15 M NaCl, and 2% Triton-X100, followed by the addition of 40 μl of purified IgG derived from non-immune serum or antiserum raised against rat ANVP. The mixture was incubated at room temperature for 30 min and 4 °C overnight.

Following the overnight incubation, 50 ul of Protein A-sepharose® 4B (10% suspension in 50 mM Tris-HCl, pH 7.5, 5 mM EDTA, 0.15 M NaCl, 0.5% Nonidet P-40 (NP-40) and 1 mg/ml ovalbumin) was added to the mixture and incubated at 4 °C for 1 hr with gentle agitation. Following centrifugation at 4 °C, the supernatant was discarded and the Protein A-Sepharose® pellet resuspended in 0.5 ml of 50 mM Tris-HCl, pH7.5, 5 mM EDTA, 0.5 M NaCl, 0.5% NP-40, and 1 mg/ml ovalbumin. The pellet was washed by vigorous vortexing, followed by centrifugation and removal of the supernatant.

This procedure was repeated four additional times. The Protein A-Sepharose® pellet was washed an additional two times with 50 mM Tris-HCl, pH 7.5, 5 mM EDTA, 0.15 M NaCl and 0.5% NP-40, followed by one wash with 10 mM Tris-HCl, pH 7.5. Following drying in vacuo, the pellet was resuspended in 60 μl of 10 mM Tris-HCl, pH 6.8, 1% glycerol, 1% SDS, 1% 2-mercaptoethanol, and 0.05% bromphenol blue, followed by incubation at 100 °C for 10 min.

The total and immunoabsorbed samples were subjected to discontinuous SDS-polyacrylamide gel electrophoresis as described by Anderson, C.W. et al., J. Virol. 12:241-252 (1973) on a 130 x 200 x 0.8 mm polyacrylamide slab gel containing 17.5% acrylamide, 0.0735% bis-acrylamide, 0.335 M Tris-HCl, pH 8.7, 0.04 M NaCl, 0.1% SDS, 0.05% ammonium persulfate, and 0.05% TEMED. The samples were run at 30 mA constant current until the bromphenol blue dye reached the bottom of the gel. The separated proteins were fixed in the gel by shaking in a solution of 25% isopropyl alcohol, 10% acetic acid, and 0.12 mg/ml Coomassie Brilliant Blue (Sigma Chemicals, St. Louis, Missouri) R-250 for 1 hr at room temperature, followed by overnight incubation in a solution of 10% isopropyl alcohol, 10% acetic acid, and 0.12 mg/ml Coomassie Blue. Following destaining with 10% acetic acid, over a period of 3 hours with several changes, the gel was treated with $En^3$Hance (trade name) (New England Nuclear, Boston, Massachusetts) according to the manufacturer's directions, followed by drying and fluorography at -70 °C using Kodak® XAR-5 x-ray film.

A comparison of the polypeptide patterns from cells containing plasmids pKT-52 or pRNF-6852, labeled with L-[$^{35}$S]-cysteine as described above, is shown in Figure 13. A polypeptide with an approximate molecular size of 6200 daltons appears uniquely in lane C, which represents the total polypeptides derived from pRNF-6852. This polypeptide is specifically immunoreactive only to anti-ANVP IgG (lane D). In addition, there was no detectable reaction of immune IgG with any polypeptide derived from pKT-52 (lane B). Thus, it was concluded that the predicted fragment of proANVP was expressed in cells containing the specific plasmid pRNF-6852.

E. coli strain JA221 1pp$^-$F' lacI$^q$ containing pRNF-6852 was deposited with the American Type Culture Collection (ATCC) 12301 Parklawn Drive, Rockville, MD 20852 on May 31, 1984 and accorded the accession number 39720.

3. Expression of cloned cDNA encoding rat proatrial natriuretic/vasodilator polypeptides (26-152)

a. Construction of plasmid pRNF12852

In a manner similar to that described in Section C.2, full length proauriculin was expressed. To accomplish this, plasmid pNFI (see above) was digested to completion with AccI, followed by ethanol precipitation. The AccI-digested DNA was mixed with the synthetic double-stranded DNA linker

```
pCATGAATCCTGT
       TTAGGACATAp
```

which had been synthesized on a SAM I DNA synthesizer (Biosearch Inc.) and purified by preparative gel electrophoresis as described. T4-DNA ligase was added to this mixture to ligate the linker onto the digestion products.

Digestion of the ligation mixture with HinfI yielded a 627 bp DNA fragment which was purified by 5% polyacrylamide gel electrophoresis and eluted. The purified 627 bp HinfI-fragment was treated with Klenow fragment and digested with PstI followed by ethanol precipitation. Plasmid pKT52 was digested with NcoI followed by treatment with Klenow enzyme, PstI digestion and calf intestinal alkaline phosphatase treatment as described (Maniatis, et al., supra at pp. 133-134). The HinfI-PstI digested pNF1 fragment was then mixed NcoI-PstI digested pKT52 and ligated using T4-DNA ligase.

Following transformation of JA221 1pp$^{-}$/F'lacI$^{q}$ with the ligation mixture, mini-preps of plasmids derived from the resulting ampicillin resistant colonies, were screened for the correct insert by digestion with HindIII followed by KpnI or HincII digestion.

A plasmid having both HindIII - KpnI and HindIII - HincII fragments of approximately 120 bp and 312 bp respectively, was chosen and designated pRNF-12852. The reading frame of the cloned full length proauriculin sequence in pKT52 was confirmed by dideoxynucleotide sequence analysis (Sanger, et al., supra).

Plasmid pRNF-12852 (Figure 12D) encodes a protein encompassing residues 25 through 152 of the rat proANVP precursor with the additional methionine codon ATG preceding the codon AAT which corresponds to residue 25.

b. Expression of plasmid pRNF-12852.

E. coli JA221 lpp-/F' lacI$^{q}$ containing pRNF-12852 or pKT52 were grown at 37°C and labeled with L-($^{35}$S]-cysteine as described in Section IV.A.2.b. Following immunoabsorbtion with anti-ANVP IgG, the labeled total and immunoabsorbed polypeptides were separated by SDS-polyacrylamide gel electrophoresis and submitted to autoradiography as described previously

A comparison of the polypeptide patterns from cells containing plasmids pKT-52 or pRNF-12852, labeled with L-[$^{35}$S]-cysteine as described above is shown in Figure 13, lanes A and E, respectively. One major polypeptide species with an approximate molecular size of 18,400 daltons appear uniquely in lane E (marked by arrows) which represents the total polypeptides derived from pRNF-12852. This polypeptide was specifically immunoreactive only to anti-ANVP IgG whereas there was no detectable reaction of immune IgG with any polypeptide derived from pKT-52 (compare lanes B and F in Figure 13). The 18,400 dalton polypeptide observed in Figure 13F is believed to be rat proANVP starting at amino acid 25 following an initiator methionine as shown in Figure 12D.

4. Expression of human proatrial natriuretic/vasodilator polypeptides (102-151)

The plasmid pHGRBI containing the human genomic DNA was digested to completion with ApaI, followed by T4-DNA polymerase treatment (Maniatis et al., supra at p. 395) to blunt the 3'-extended termini. A synthetic HindIII linker (pCAAGCTTG, Collaborative Research Inc., Lexington, MA) was attached to the blunt-ended human genomic DNA through blunt-end ligation as described above. The ligation mixture was then digested with HindIII and NcoI, followed by the isolation of a 272 bp HindIII-NcoI fragment using 5% polyacrylamide gel electrophoresis and elution. The 272bp HindIII-NcoI fragment was mixed with HindIII-NcoI digested pBR329 (Covarrubias, L. and F. Bolivar, Gene 17:79-89 (1982)) and treated wtih T4-DNA ligase. The resulting plasmid pHNF-298 was digested with BamHI and NcoI and the resulting 620 bp NcoI-BamHI fragment purified by agarose gel electrophoresis The 620 bp NcoI-BamHI fragment was digested to completion with MspI followed by repair of the 5'extended termini by E. coli DNA polymerase I (Klenow fragment). The synthetic HindIII linker pTTACTAAGCTTAGTAA was synthesized, purified and phosphorylated and attached to the MspI digested NcoI-BamHI fragment through blunt-end ligation.

The ligation mixture was digested with HindIII, followed by the isolation of an 156 bp HindIII fragment by 5% polyacrylamide gel electrophoresis. The 156 bp HindIII fragment was attached to pKT52, which had been digested with HindIII and treated with calf intestinal alkaline phosphatase using T4-DNA ligase as described.

Following transformation of JA221 1pp$^{-}$/F'lacI$^{q}$ with the ligation mixture, mini-preps of plasmids derived from the resulting ampicillin resistant colonies, were screened for the correct insert by digestion with NcoI followed by ClaI digestion.

A plasmid having an NcoI-ClaI insert of 150 bp was chosen and designated pHNF-5752. The reading frame of the cloned human proANVP sequence in pKT52 was confirmed by DNA sequence analysis as described.

Because a synthetic HindIII 8-mer linker was used to allow cloning of the proANVP cDNA fragment into the HindIII site of pKT52, the amino acids preceding the proANVP sequence are Met-Ala-Ala-Ala-Lys-Leu-Ala. In addition the synthetic HindIII 16-mer linker was used to reconstruct the carboxy terminal amino acid residue Arg and Tyr. Therefore, the sequence of the expressed human proANVP fragment was determined to be:

```
                                                                  NH2 Met
Ala Ala Ala Lys Leu Ala Trp Asp Ser Ser Asp Arg Ser Ala
Leu Leu Lys Ser Lys Leu Arg Ala Leu Leu Thr Ala Pro Arg
Ser Leu Arg Arg Ser Ser Cys Phe Gly Gly Arg Met Asp Arg
Ile Gly Ala Gln Ser Gly Leu Gly Cys Asn Ser Phe Arg Tyr
COOH
```

using dideoxynucleotide sequence analysis as described (Sanger, et al. supra).

5. Expression of cloned cDNA encoding human proatrial natriuretic/vasodilator polypeptide (26-151)

a. Construction of plasmid phNF-233

λ-phage DNA from clone 6 containing the human ANVP cDNA (Section II.C) was digested with EcoRI followed by the isolation of a 713 bp fragment using polyacrylamide gel electrophoresis and elution. The purified EcoRI fragment was ligated to similarly digested plasmid pUC-9 (Vieira, J. and Messing, J., Gene 19, supra) followed by transformation into E. coli as described. A plasmid with an appropriate 713 bp EcoRI fragment was isolated and designated phNF-pUC-1. Plasmid phNF-pUC-1 was digested to completion with RsaI followed by phenol/chloroform extraction and ethanol precipitation. The RsaI digested DNA was mixed with the synthetic double stranded DNA linker

```
5' TATGAATCCCATGT 3'
3'   ACTTAGGGTACA 5'
```

which had been synthesized and purified as described followed by the addition of T4-DNA ligase to ligate the linker onto the RsaI digestion products. Digestion of the ligation mixture with NdeI yielded a 370 bp DNA fragment which was purified by polyacrylamide gel electrophoresis and eluted. Plasmid pUC-19 (Vieria, J. & Messing, J. supra) was digested with NdeI and treated with calf intestinal phosphatase followed by phenol/chloroform extraction and ethanol precipitation. The 370 bp DNA fragment described above was ligated onto the NdeI digested pUC-19 followed by transformation of E. coli. The screening of transformants resulted in plasmid containing the 370 bp NdeI human proANVP cDNA sequence which was chosen and designated phNF-pUC-2. PhNF-pUC-2 was digested with ApaI and EcoRI followed by the isolation of the larger sequence by agarose gel electrophoresis as described. Plasmid phNF-pUC-1 was also digested with ApaI and EcoRI followed by the isolation of a 442 bp sequence by polyacrylamide gel electrophoresis and elution. The 442 bp ApaI-EcoRI fragment derived from phNF-pUC-1 was ligated onto ApaI-EcoRI digested phNF-pUC-2 followed by transformation of E. coli. The resulting transformants were screened by digestion with NdeI and PstI which yielded a plasmid containing a 517 bp NdeI-PstI fragment which was isolated and designated phNF-pUC-3.

The expression plasmid pTRP-233 (Figure 12F) was digested to completion with NdeI and PstI followed by calf-intestinal phosphatase treatment as described. Plasmid phNF-pUC-3 was digested to completion with NdeI and PstI followed by the isolation of 517bp DNA sequence by polyacrylamide gel electrophoresis and elution. The NdeI-PstI fragment derived from phNF-pUC-3 was ligated to NdeI-PstI digested pTRP-233 followed by transformation of E. coli. The resulting plasmid containing the 517bp NdeI-PstI fragment in pTrp233 was isolated and designated phNF-233 (Figure 12G). To confirm the fidelity of the trp promoter/operator sequence as well as the reading frame of cloned human proANVP sequence in pTRP-233, phNF-233 was digested with EcoRI and PstI followed by the isolation of the DNA sequence by polyacrylamide gel electrophoresis and elution. This fragment was ligated to plasmid M13-mp18 (Messing, J. and Vieria, J., supra) and submitted to dideoxynucleotide sequence analysis (Sanger et al., supra).

As shown in Figure 12G, plasmid phNF-233 was designed to express full length human proANVP encompassing residues 26 through 151 of the human pre-proANVP protein with an additional methionine codon, ATG, preceeding the codon AAT which corresponds to residue 26.

b. Expression of cloned full length human proANVP (26-151) cDNA in plasmid phNF-233

E. Coli E103S (Hfr, MetB, $lacI^{ts}$) containing phNF-233 or ptrp-233 were grown overnight at 37°C in Luria-Bertani medium (Maniatis et al., supra at p. 68) supplemented with ampicilin (100 ug/ml). The overnight cultures were diluted 1:100 in media containing M9 minimal salts (Miller, J., Experiments in Molecular Genetics, Cold Spring Harbor Laboratory, Cold Spring Harbor, New York) supplemented with glucose (0.4%), thiamine (2μg/ml), $MgSO4 \cdot 7H_2O$ (200 ug/ml), Casamino acids (0.5%) and ampicillin (100 ug/ml) and grown at 37°C until the cell density reached an $A_{550}$ of 0.1 at which time 3-β-indoleacrylic acid (Sigma Chemical Co.) was added from a 10mg/ml ethanolic solution to a final concentration of 25 μg/ml. At an $A_{550}$ of 0.50, L-[$^{35}$S]-cysteine (200 μCi/ml culture (New England Nuclear)) was added and incubation continued for 1 min. One milliliter of culture was removed at this time and added to 340 μl of ice-cold 20% trichloroacetic acid and treated as described in section IV.A.2.

Immunoabsorption of L-[$^{35}$S]-cysteine labeled polypeptides derived from cells containing phNF-233 or pTRP-233 was accomplished as described in Section IV.A.2. using antisera raised in rabbits injected with synthetic rat ANVP (127-152).

Total and immunoabsorbed L-[$^{35}$S]-cysteine labeled polypeptides were analyzed by SDS-polyacrylamide gel electrophoresis and fluorography as described in Section IV.A.2. A comparison of the polypeptide patterns from cells containing plasmids pTRP-233 or phNF-233 labeled with L-[$^{35}$S)-cysteine is shown in Figure 14. A major polypeptide with an approximate molecular weight of 18,000 daltons appears uniquely in lane B which represents the total polypeptides derived from phNF-233 as compared to lane A which represents total polypeptides derived from pTRP-233. This 18 K dalton polypeptide is specifically immunoreactive to anti-ANVP antisera whereas reaction with polypeptides derived from pTRP-233 was not detected (compare lanes C and D in Figure 14). The predicted polypeptide of human pro-ANVP (26-151) was therefore expressed in cells containing the specific plasmid phNF-233.

E. coli strain K12 E103S Hfr Cavalli, met B, $lacI^{ts}$ containing pRNF-6852 was deposited with the American Type Culture Collection (ATCC) 53085 Parklawn Drive, Rockville, MD 20852 on April 9, 1985 and accorded the accession number 53085.

c. Purification and characterization of human pro-ANVP (26-151) from E. Coli.

E. coli E103S containing phNF-233 were grown in Luria-Bertani media supplemented with ampicillin (100 μg/ml) at 37°C overnight. Ten milliliters of the culture was diluted into 1000 ml of media containing minimal salts and supplements as described in Section IV.5.b and grown at 37°C to a cell density corresponding to 0.1 $A_{550}$. At this time 3-β-indole-acrylic acid was added from a 10 mg/ml ethanolic solution to a final concentration of 25 μg/ml. The cells were allowed to grow until a cell density corresponding to 1.0 $A_{550}$ was reached at which time the cells were collected by centrifugation at 7,000 rpm at 4°C. The cell pellets were resuspended in an ice-cold solution of 10mM Tris-HCl, pH 8, and centrifuged as before. The washed cell pellet was resuspended in 40 ml of an aqueous solution containing 1 M acetic acid and 20 mM HCl. The cells were disrupted using a Heat Systems-Ultrasonics Inc. Model W-225-R sonicater at 0°C. The suspension was then incubated in a closed container in a boiling water bath for 5 min. followed by centrifugation at 12,000 rpm at 4°C for 20 min. The supernatant (termed acid extract) was removed and applied to a 2.5 cm X 100 cm column containing Sephadex® G-10 (Pharmacia) which had been equilibrated with 10mM Tris-HCl, pH 7.5 and 1 mM EDTA. Resulting immunoreactive fractions, as detected by a specific ANVP radioimmunoassay (see Section V), were pooled and dried by lyophilization. The dried fractions were resuspended in 0.5 M acetic acid and applied to a 2.5 X 50 cm column containing Sephadex® G-10 previously equilibrated in 0.5M acetic acid.

Resulting immunoreactive column fractions were pooled and lyophilized. The dried material was brought up in approximately 4 ml of distilled water and submitted to high performance liquid chromatography HPLC purification. The material was applied to a 1 X 25 centimeter Vydac $C_{18}$ column (Vydac 218TP1010) using a Perkin-Elmer Series 4 LC injector and solvent delivery system (Perkin-Elmer). The bound material was washed for 2 minutes with aqueous 15% acetonitrile ($CH_3CN$) and 0.1% trifluoroacetic acid (TFA) which was followed by the development of a linear gradient of $CH_3CN$ and 0.1% TFA from 15:85 to 60:40 over 45 minutes. Aliquots from 1 minute fractions were collected and portions dried and assayed for immunoreactivity using an anti-ANVP radioimmunoassay as described. The peak of immunoreactivity

was subsequently collected and dried in vacuo. The dried material was resuspended in water and dried again by lyophilization.

One hundred pmoles of material was submitted to automated amino acid sequence analysis using a Model 470A protein sequenator (Applied Biosystems Inc.). The first 18 amino acids analyzed were: Met-Asn-Pro-Met-Tyr-Asn-Ala-Val-Ser-Asn-Ala-Asp-Leu-Met-Asp-Phe-Lys-Asn-. No other detectable impurities could be observed from this analysis. SDS-polyacrylamide gel analysis, as described in section IV.2.b, also revealed no other detectable impurities of the purified material which migrated with an apparent molecular size of 18,000 daltons. From the sequence analysis of this polypeptide described above it was concluded that this polypeptide corresponds to full length human (26-151) pro-ANVP with an additional amino-terminal methionine and contains undetectable contamination from bacterial polypeptides.

B. Expression of Proatrial natriuretic/vasodilator polypeptides and Pre-proatrial natriuretic/vasodilator polypeptides in Saccharomyces cerevisiae

In the examples that follow the expression of various proANVP and ANVP polypeptides in S. cerevisiae are described. In a similar manner any proANVP or ANVP sequence can be expressed in S. cerevisiae.

1. Intracellular expression

Two procedures are disclosed for the preparation of vectors for intracellular expression in the yeast Saccharomyces cerevisiae of cDNA encoding pre-proANVPs, proANVPs and fragments of proANVPs containing mature ANVPs. Each utilizes the strong promoter sequence found in front of the yeast phosphoglycerate kinase (PGK) gene. For the first procedure, the plasmid pNFI was digested with HincII (New England Biolabs). BamHI linker oligonucleotides (8 nucleotides in length Collaborative Research, Inc.) were ligated onto the digestion products, and the resulting molecules were digested with BamHI. The 454 bp fragment from this digest containing the mature ANVP sequence was then purified by 5% polyacrylamide gel electrophoresis and ligated into the BamHI site of the yeast - E. coli vector pYPGK2. This vector was constructed by digesting the yeast - E. coli shuttle vector YEp13 (J. Broach et al., Gene 8:121-133 (1979)) with the restriction enzymes BamHI and HindIII, and then ligating the largest of the restriction fragments obtained to a restriction fragment spanning the promoter region from the yeast PGK gene. The PGK-promoter-containing fragment extends from a HindIII restriction site approximately 1500 base pairs upstream from the ATG start codon of PGK, to a BamHI linker oligonucleotide (8 base pairs in length, Collaborative Research,. Inc.) inserted 28 base pairs downstream from the ATG start codon after BAL-31 digestion from within the PGK coding region.

Using this vector, any sequence of DNA in the pre-proANVP sequence can be inserted and used to express a desired fragment of pre-proANVP. For example, insertion of the 454 bp ANVP-containing fragment into the BamHI linker site in this vector in the correct orientation allows the synthesis of a 78-amino acid-long fusion protein from the PGK promoter (consisting of 9 amino acids from the amino terminus of the PKG gene, 3 amino acids coded for by the linker oligonucleotide, 39 amino acids of the pro-ANVP region, the 25 amino acids of the mature ANVP sequence, and the two arginine residues of the carboxy terminus of the ANVP precursor).

A second procedure for intracellular expression of pre-proANVPs also allows extracellular secretion of proANVPs and fragments thereof. In the second procedure, a restriction fragment containing the entire pre-proANVP precursor coding region is isolated from the plasmid pNF4 by first digesting the plasmid with the restriction enzyme SalI (New England Biolabs). The single-stranded regions on the ends of the resulting linear length plasmid molecules are made double-stranded by treatment with DNA polymerase I (Klenow fragment), and BamHI linkers (8 nucleotides in length, Collaborative Research, Inc.) are then ligated on to these blunt ends. The linear-length plasmid molecules are then digested with BamHI and EcoRI, and the approximately 900 bp BamHI (SalI) - EcoRI fragment containing the pre-proANVP sequence is isolated. The fragment is ligated into a vector identical to the pYPGK2 vector described above, except for two modifications: (1) the BamHI linker oligonucleotide lies 23 bp upstream from (5' to) the ATG codon of PGK, and (2) the cloned cDNA fragment is followed by the transcription termination region of the PGK gene (EcoRI - HindIII fragment containing the 3' end of the PGK locus, plus the 346 bp HindIII - BamHI fragment from pBR322 as a 3' linker. Expression of the inserted pre-proANVP cDNA from the PGK promoter results in the synthesis of pre-proANVPs. The pre-proANVP that is expressed will be processed and secreted by the yeast cell if the signal and/or processing sites are recognized as such by the cell and acted upon. The material so secreted will be either proANVPs, fragments thereof or ANVPs alone. If recognition of the signal sequence does not occur, the full-length pre-proANVPs or fragments thereof will be found internally in the

cells.

2. Extracellular Expression

a) Construction of YEp-α-8 expression vector

A yeast library in the E. coli-yeast shuttle vector YEp13 (Nasmyth, K. and K. Tatchell, Cell 19:753-764 (1980)) was screened using a '5-$^{32}$P end labeled oligodeoxynucleotide (5'-CCTGGCCAACCAATG-3'), (see Maniatis et al., supra at pp. 324-325). Plasmids containing inserts of yeast DNA hybridizing to this oligonucleotide were subsequently isolated. One of these plasmids contained an insert of approximately 15kb of yeast DNA, and was shown to contain the 1.7kb EcoRI fragment containing the α-factor gene as described by Kurjan, J. and I. Herskowitz, Cell 30:933-943 (1982). The ends of the 1.7kb EcoRI fragment were made blunt by incubation with DNA polymerase I (Klenow fragment) and BamHI linkers were attached using T4-DNA ligase (Maniatis et al., supra at pp. 113-114, 116, 392-394). The BamHI ends were made cohesive by digestion with BamHI, and subsequently ligated into the BamHI site of the yeast-E. coli shuttle plasmid pCV7-Hin 228. A deletion around the HindIII site of the plasmid CV7 was made by HindIII digestion, treatment with exonuclease III, treatment with S1 nuclease, and religation with T4-DNA ligase to generate the plasmid pCV7-HinΔ228, all using the method described in Broach, J.R. and J.B. Hicks, Cell 21:501-508 1980. This plasmid containing the yeast α-factor gene is diagrammed in Figure 15, and henceforth referred to as YEp-α-8.

b) Insertion of cDNA coding for rat proatrial natriuretic/vasodilator polypeptides into YEp-α-8

Two fragments of DNA from pNFI (Section II.A.3.) encoding pre-proANVP were inserted into the unique HindIII site of YEp-α-8 (Figure 15) by restriction endonuclease cleavage, filling in the ends of DNA with DNA polymerase I (Klenow fragment) as necessary and adding HindIII linkers (Maniatis et al., supra at p. 392). The ends of the DNA fragments were subsequently made cohesive by digestion with HindIII, and ligated into HindIII cleaved YEp-α-8, which had been treated with alkaline phosphatase (see Maniatis et al., supra at pp. 133-134). Recombinant molecules were transformed into E. coli, and colonies analyzed for plasmid DNA (Maniatis et al., supra at pp. 366-369).

A HaeIII fragment was generated as shown and size selected from polyacrylamide gels as described in Maniatis et al, supra at pp. 173-175. This fragment of 266 bp was then cloned into YEp-α-8, as described above to generate expression vector YEp-α-NF-5. This insert, in the correct orientation, encodes a 33 amino acid peptide containing the mature ANVP sequence, corresponding to amino acids 121-152 of the proANVP sequence with an additional phenylalanine at the amino terminus. As a control, the reverse orientation of the insert was cloned into YEp-α-8 and designated YEp-α-NF-7. This insert would encode an unrelated protein having a sequence of different amino acid. Similarly, an AccI fragment of 623 bp was isolated and cloned in its correct orientation into YEp-α-8, yielding expression vector YEp-α-NF-9. This insert encodes a 126 amino acid polypeptide comprising almost the entire prorANVP sequence (amino acids 28-152) with an additional tyrosine at the $NH_2$ terminus. This insert was also cloned in its inverse orientation to generate control plasmid YEp-α-NF12. Insertion of these HaeIII and AccI fragments of rat proANVP, after the addition of the HindIII linkers, yields DNA sequences coding a chimeric protein. This protein codes for the α-factor signal/leader peptide, a spacer fragment and the proper proANVP fragment.

DNA was prepared from E. coli cultures containing these plasmids (Maniatis et al., supra at pp. 366-369) and was used to transform S. cerevisae strain W301-18A (α ade 2-1, trp 1-1, leu 2-3, 112, can 1-100, ura 3-1, his 3-11, 5) to Leu 2 prototrophy. Yeast strains were grown on standard media (Sherman et al., Methods in Yeast Genetics, Cold Spring Harbor Press (Cold Spring Harbor, New York)). Plasmid DNA from E. coli was also re-cloned into M13 for sequencing and confirmation of the α-factor proANVP DNA constructions (Messing J. and J. Vieira, supra).

c) Expression and secretion of rat proatrial natriuretic/vasodilator polypeptides sequences in S. cerevisiae

The α-factor proANVP fragment processing scheme is shown in Figure 15. The mRNA transcript is initiated and terminated from the α-factor sequences in the vector. This is translated into a chimeric protein and initiated through the yeast secretory process. Proteolytic processing of this protein occurs both at the Glu-Ala (QA) residues and the Lys-Arg (KR) residues in the α-factor portion of the molecule (Kurjan J. and I. Herskowitz, supra). The C-terminal portion of this processed protein therefore is the predicted amino acid of rat proANVP. Cultures of yeast containing these plasmids were maintained in synthetic medium lacking

leucine (see Sherman et al., supra). This selection is necessary, as yeast plasmids are relatively unstable and lost at approximately 1.0% per generation. Yeast cultures were labeled with 0.1 to 0.5 mCi/ml $^{35}$S-cysteine (approximately 1000 Ci/mmole) in synthetic medium without leucine for four hours. Bovine serum albumin was added at a final concentration of 100 μg/ml to prevent possible proteolysis. Samples (1.0 ml) were taken, cells removed by centrifugation, the media proteins concentrated by 10% TCA precipitation at 4°C for 15 minutes and subsequent centrifugation in an Eppendorf microfuge (15,000 x g). The resulting pellet was washed with acetone, dried under vacuum and resuspended in SDS sample buffer (Laemlli, U.K., Nature 227:680-685 (1970)). These samples were applied directly to an SDS-PAGE gel (17.5% acrylamide) to examine the pattern of total secreted $^{35}$S-met proteins by autoradiography of the dried gel. As can be seen in Figure 16A, culture supernatants from yeast cultures containing YEp-α-NF-9 showed $^{35}$S-met labeled bands at approximately 11.1 and 9.4 kd (lanes 3 and 4) while media from cultures of YEp-α-NF-12 (the inverse construction) showed a $^{35}$S-met labeled band at approximately 5 kd (lanes 5 and 6). Neither of these bands were detected in media from cultures containing the plasmid vector YEp-α-8 (lanes 1 and 2).

The molecular weights of the proteins whose synthesis and secretion is directed by YEp-α-NF-9 are not inconsistent with the possibility that an endogenous yeast protease cleaves the ANVP peptide from the proANVP precursor encoded by the AccI fragment in this plasmid. To confirm this possibility, yeast cultures harboring this plasmid, its corresponding inverse orientation (YEp-α-NF-12), and yeast cultures harboring YEp-α-NF-5 and YEp-α-NF-7 (the HaeIII fragment encoding the small fragment of proANVP) were labeled as above with both $^{35}$S-Met and $^{35}$S-Cys to determine if they expressed $^{35}$S-labeled proteins which could be specifically immunoprecipitable. The $^{35}$ S-Met will be incorporated into proANVP protein but not ANVP while $^{35}$ S-Cys is selectively incorporated into ANVP. Since control experiments suggested that some yeast media components prohibited direct immunoprecipitation, a novel partial purification scheme was performed as follows.

Cells were removed by centrifugation and the cell free supernatant used either directly or concentrated by lyophilization. Ten volumes of acetone were added to the aqueous solution and the mixture allowed to precipitate on ice for 10-15 minutes. The precipitate was then pelleted by centrifugation, and the acetone removed. A small amount of water (no more than 1 volume) was added to this pellet to facilitate resuspension. Ten volumes of methanol were then added to this mixture, extensively mixed, and the precipitate collected by centrifugation The supernatant was then removed and dried under vacuum. This pellet was resolubilized in 1.0 ml of immunoprecipitation buffer and immunoprecipitated and washed as described in Section IV.A.2.

As shown in Figure 16B, the complexity of proteins as determined after the above extraction procedure is relatively simple compared with the complexity of total secreted protein. Lanes 1 and 2 in Figure 16B show the secreted $^{35}$ S-Cys labeled protein whose synthesis is directed by YEp-α-NF-7 and YEp-α-NF-5 respectively in the methanol soluble fraction. Lanes 3 and 4 in Figure 16B show the same proteins following immunoprecipitation in both cases with anti-ANVP IgG. The antiserum appears to specifically precipitate a 3,000 Dalton protein from YEp-α-NF-5 (lane 4, Figure 16B) while no protein was precipitated from the corresponding inverse orientation (YEp-α-NF-7) (lane 3, Figure 16B). Lanes 5 and 6 show a similar immunoprecipitation of $^{35}$ S-Cysteine labeled proteins appearing in the methanol soluble fraction of media conditioned by yeast cultures harboring plasmids YEp-α -NF-12 and YEp-α-NF-9, respectively. The result is the same as shown for lanes 3 and 4, Figure 16B, and a 3,000 Dalton protein was specifically immunoprecipitated from media conditioned by S. cerevisiae containing YEp-α-NF-9.

These results suggest that both yeast expression plasmids, YEp-α-NF-5 and YEp-α-NF-9, direct the synthesis of a 3 kd $^{35}$ S-Cys labeled protein (approximately 25-30 amino acids in length) which is immunoprecipitated by specific anti-ANVP IgG.

S. cerevisiae strain W301-18A containing YEp-α-NF-9 was deposited with the ATCC on May 31, 1984 and accorded accession number 20710.

d) Expression and Secretion of human proatrial natriuretic/vasodilator polypeptide (128-151) in S. cerevisiae.

i. Construction of PJC1-5 expression vector.

Plasmid YEp-α-8 (Section IV.B.2.b.) was digested to completion with Hind III followed by purification of the largest of the restriction fragments by agarose gel electrophoresis as described. The purified DNA was ligated and transformed in E. coli. Plasmid DNA containing only one Hind III restriction site was purified and designated pJJ-1 (Figure 17A).

Plasmid pJJ-1 was digested to completion with PstI and SalI followed by the purification of a 317 bp DNA fragment by agarose gel electrophoresis. The PstI-SalI fragment was ligated to similarly digested

plasmid M13mp8 (Messing, J. and Vieria, J., supra). Single stranded recombinant phage M13 DNA designated MP-JJ1 was isolated and used as the template for oligodeoxynucleotide mediated-site-directed mutagenesis (Zoller, M.J. and Smith, M., Methods in Enzymology 100:468-500 (1983)) utilizing the mutagenic oligodeoxynucleotide 5'-GAAGAAGGGGTAAGCTTGGATAAAAGAG-3'. The resulting mutagenesis changed the nucleotide codon -TCT- present at position 241-243 in the coding region of the α-factor gene (Kurjan, J. and Herskowitz, I., supra) to AGC w hich introduces a Hind III restriction site at this position but does not change the amino acid Ser encoded by this codon (Figure 17A). The mutagenized recombinant MP-JJ-1 was designated MP-JJ-5. Plasmid JJ-1 was partially digested with PstI in the presence of ethidium bromide followed by phenol/chloroform extraction and ethanol precipitation. The DNA was digested to completion with Hind III followed by calf intestinal phosphatose treatment and the purification of the largest of the restriction fragments by agarose gel electrophoresis. The replicative form of MP-JJ-5 was digested with PstI and Hind III followed by isolation of a 220 bp DNA fragment by polyacrylamide gel electrophoresis. The 220 bp PstI-Hind III DNA fragment was ligated to the similarly digested pJJ-1 and transformed into E. coli. The resulting plasmid containing PstI-Hind III restriction fragments of 5853, 2280, 993, 780 and 220 bp was purified and designated pJC1-5 (Figure 17A).

ii. Construction of plasmid pJC-2 containing a synthetic DNA sequence encoding human AVNP (128-151).

The synthetic DNA sequence shown in Figure 17B was designed with the set of preferred yeast codons derived from the codon usage in the highly expressed yeast genes encoding the glycolytic enzymes.

The eight oligodeoxynucleotides shown in Figure 17B were assembled as described in Section IV.A.1.b. Following ligation, the mixture of DNA was digested with Hind III followed by the purification of a 103 bp fragment by polyacrylamide gel electrophoresis. The purified DNA fragment was ligated into Hind III digested M13-mP18 and M13-mP19 (Vierra, J. and Messing, J., supra) and submitted to dideoxynucleotide sequence analysis as described (Sanger et al., supra) which resulted in the sequence shown in Figure 17B.

Plasmid JCl-5 was digested to completion with Hind III followed by calf intestinal phosphatase treatment, phenol/chloroform extraction and ethanol precipitation. The purified synthetic DNA sequence described above was ligated to Hind III digested JCl-5 followed by transformation in E. coli. Resultant plasmids were screened for the 103 bp synthetic Hind III sequence of which one was purified and designated pJC-2.

Plasmid pJC-2 encodes the α-factor signal/leader peptide (Kurjan, J. and Herskowitz, I., supra) with an inframe fusion of the human AVNP peptide sequence (128-151) starting after amino acid 85 of the α-factor precursor. The presence of the α-factor regulatory and secretory sequences upstream of the AVNP peptide allows the proteolytic processing by the protease encoded by the KEX2 locus (Julius, D. et al. Cell 37:1075-1089 (1984)) following the -Lys-Arg- residues at positions 84 and 85 (Figure 17B), respectively, and the extracellular secretion of human AVNP (128-151) similar to that described in Section IV.b.1.c. for rat pro-AVNP sequences. Plasmid JC-2 was used to transform S. Cerevisae W301-18A (Section IV.B.1.b.) which was maintained on selective media.

iii. Expression and purification of human ANVP (128-151) sequence from S. cerevisae.

S. cerevisae W301-184 containing plasmids JC1-5 or JC-2 were grown at 30°C in selective synthetic medium as described (Section IV.B.1.c.) to stationary phase of growth at which time the cells were removed by centrifugation at 4°C. The resulting supernatant was removed and assayed for the presence of the ANVP peptide by specific radioimmunoassay (Section V). The results shown in Table VI demonstrate that S. cerevisae containing plasmid JC-2 are secreting immunoreactive peptide.

Table VI

| Immunoreactive Peptide (mg/l of culture). | |
|---|---|
| pJC1-5 | 0.0 ± 0.03 |
| pJC-2 | 0.57 ± 0.10 |

To positively identify the immunoreactive peptide, S. cerevisae containing plasmid JC-2 were grown at 30°C in one liter of selective synthetic media to stationary phase. The cells were removed by centrifugation. The resulting supernatent was adjusted to pH 8.0 with ammonium hydroxide followed by centrifugation. The resulting cleared supernatent was applied to a 2.5 cm x 10 cm column containing DEAE® Sephacel®

(Pharmacia) previously equilibrated with 0.01M ammonium acetate followed by the collection of the eluate (approximately 1.1 l) and lyophilization. The dried mixture was resuspended in approximately 12 ml of 0.5M acetic acid and applied to a 2.5 cm x 50 cm column containing Sephadex® G-10 equilibrated with the resuspension solution. Fractions containing immunoactivity as assayed by radioimmunoassay (Section V) were collected, pooled and lypholized.

The dried material was resuspended in 5 ml of acetic acid and submitted to high performance liquid chromatography as described in Section IV.A.5.

The immunoreactive peak fraction was isolated and 200 pmoles submitted to automated amino acid sequence analysis as described (Section IV.A.5.). The resulting amino acid sequence was:

H-Ser-Ser-Cys-Phe-Gly-Gly-Arg-Met-Asp-Arg-Ile-
Gly-Ala-Gln-Ser-Gly-Leu-Gly-Cys-Asn-Ser-Phe-Arg-
Tyr-OH

In addition, contaminants due to yeast polypeptides were not detectable by this method of analysis.

S. cerevisiae strain W301-18A containing pJC2 was deposited with the ATCC on April 9, 1985 and accorded accession number 20754.

C. Expression of pre-proatrial natriuretic/vasodilator polypeptides in cultured Chinese hamster ovary cells

In the examples that follow, the expression of prorANVP(25-152) and prohANVP(26-151) in CHO cells is described. These examples are provided by way of illustration and it will be readily understood that any pre-proANVP, proANVP, or ANVP can be expressed in mammalian cells in the same manner.

1) Expression of rat pre-proatrial natriuretic/vasodilator polypeptides

To facilitate the expression of rat pre-proANVPs in mammalian cells, a hybrid gene was constructed in which the coding segment for rat pre-proANVP was fused to a powerful regulated promoter derived from the human metallothionein II (hMTII) gene. This was performed in two steps. First, an expression vector was prepared. The expression vector, pHSI, carries 840 nucleotide base pairs of hMTII sequence (Karin, M. et al., Nature 299:797-802 (1982)) from a naturally occuring HindIII restriction site at base -765 at the start of transcription to base $^{+}70$, located in the 5' untranslated region adjacent to the coding region. pHSI also carries a region into which coding sequences may be inserted. To construct pHSI the plasmid p84H, which carries the hMTII gene, was digested to completion with restriction endonuclease BamHI followed by treatment with exonuclease Bal-31 to remove terminal nucleotides. Following digestion with HindIII, the products of this reaction were ligated into plasmid pUC8 (Vieira, J. and J. Messing, Gene 19:259-268 (1982) which had been opened with HindIII and HincII digestion. One of the resulting plasmid recombinants had the composition of pHSI as determined by nucleotide sequencing.

To complete the construction of the hybrid gene, the EcoRI-SalI rat pre-proANVP cDNA was isolated from plasmid pNFI (Section II A.) by digestion with EcoRI and SalI followed by polyacrylamide gel purification. pHSI was opened with EcoRI and ligated to the cDNA fragment with T4-DNA ligase. The reaction products were then incubated with the four nucleotide triphosphates and DNA polymerase I (Klenow fragment) in order to create blunt-ended molecules which were subsequently subjected to a second ligation to allow recircularization. The recombinant plasmid molecules were introduced into E. coli MC1061 and screened by restriction endonuclease analysis (Maniatis et al., supra at p. 104). Two recombinants, (Figure 18A) pMT-NFI-10 and pMT-NFI-20, were introduced into the chinese hamster ovary (CH0) line of cultured cells by co-transformation with pSV2:NEO (Southern, P. and P. Berg, J. Mol. Appl. Genet. 1:327-341 (1982)), a plasmid carrying a functional gene conferring resistance to the neomycin analogue G418. 500 ng of pSV2:NEO and 5ug of pMT-NFI-10 or pMT-NFI-20 were applied to a 60 mm dish of cells in a calcium phosphate-DNA coprecipitate according to standard protocols (Wigler, M., et al., Cell 16:777-785 (1979)) with the inclusion of a two minute "shock" with 15% glycerol after 4 hours exposure to the DNA. A day later the cells were subjected to exposure to G418 at lmg/ml. This procedure yielded a pool of G418 resistant colonies most which had also acquired stable inheritance of pMT-NFI-10 or pMT-NF1-20. Previous experience with CHO cells and other cultured cells (McCormick, F. et al., Molecular and Cellular Biology 4-1 p.166 (1984)) indicates that they are able to cleave the signal peptide from mammalian prehormones and are able to secrete the remainder of the polypeptide into the nutrient medium.

Accordingly, the production of pre-proANVPs and related peptides is then examined by incubating the cells with $^{35}$S-met and examining the radio-labeled secreted products by standard protein gel analysis.

Autoradiograms of $^{35}$S-Met-labeled proteins secreted into the media reveal the appearance of a 18,000 dalton protein that is specifically immunoprecipitated by anti-ANVP IgG. This protein is not seen in $^{35}$S-Methionine labelled proteins of cells containing a control plasmid. Thus CHO cells containing pMT-NF-1-10 secrete proANVPs into the media of these cells.

Chinese Hamster Ovary (CHO) cells containing pMT-NF1-10 were deposited with the ATCC on May 31, 1984 and accorded accession number CRL 8569.

2) Expression of human pre-proatrial natriuretic/vasodilator polypeptides in cultured mammalian cells

Human pre-proANVPs was expressed in a similar manner with appropriate modifications to account for the features of the human genomic clone. Briefly, a plasmid, HGRB 1, carrying the BamHI to EcoRI human genomic segment spanning the pre-proANVP gene was constructed, partially digested with restriction endonuclease AccI and completely digested with EcoRI. The resulting AccI-EcoRI fragment was isolated by polyacrylamide gel purification. This fragment, which extends from the 5' untranslated region to a point past the 3' end of the gene, was ligated to the expression plasmid pMT401 which was opened with AccI and EcoRI. Plasmid pMT401 was derived by insertion of the BamHI-bounded polylinker region from M13mp7 (Vieira and Messing, supra) into the BamHI site of pHSI. The resulting recombinant contained the human pre-proANVP gene positioned 3' from the human metallothionein promoter and was designated pHNF-8. The hybrid construction was then introduced into cultured CHO cells for expression in a manner similar to that described above for rat pre-proANVPs.

Cultured CHO cells which were transformed with pHNF-8 were subcloned by plating cells at low dilutions in dishes containing Harris F-12 medium supplemented with 10% fetal calf serum. Individual subclones were subsequently removed and each examined for pro-ANVP production by both radioimmunoassay (Section V) and radiolabelling with $^{35}$S-methionine. The $^{35}$S-methionine-labelled proteins were resolved by SDS-polyacrylamide gel electrophoresis. Shown in Figure 18B are the results of these experiments. Lane 2 shows $^{35}$S-methionine-labelled proteins from control CHO cells. Lane 1 shows $^{35}$S-methionine labelled proteins from a CHO pool transformed with pHNF-8 prior to subcloning. Lanes 3-6 show $^{35}$S-methionine-labelled proteins from four subclones containing pHNF-8 and are designated CHO-8/2-6, CHO-8/2-55, CHO-8/2-81, CHO-8/2-93. In each of the subclones distinct $^{35}$S-methionine-labelled bands appear at 18,000 and 10,000 daltons. The bands were identified as proANVP (18,000 k) and a proANVP fragment comprising the amino terminus of this protein by immunoprecipitation with specific antibodies. The fact that the subclones are producing proANVP was confirmed by radioimmunoassay as described in Section V.

It should be noted that the 10,000 dalton form implies that a substantial portion of proANVP is being cleaved by proteolysis in this system. Such a proteolytic event yields the amino terminal fragment (10,000 k) and smaller carboxy terminal fragments containing shorter ANVP's. Thus, the clones are considered important for producing proANVP and smaller fragments with or without the biologically active ANVP segment.

Chinese Hamster Ovary (CHO) cells containing pHNF-8 and designated CHO-8/2-81 were deposited with the ATCC on April 9, 1985 and accorded accession number CRL-8782.

D. Biological activity of expression products derived from pre-proatrial natriuretic/vasodilator polypeptides and proatrial natriuretic/vasodilator polypeptides

Various rat and human proANVPs and ANVPs whose expression and secretion are directed by the yeast $\alpha$-factor system described in Section D.2 and the E. coli system described in Section C.2 above were tested and shown to possess biological activity. Yeast cultures (100ml) were grown in synthetic media containing 100 $\mu$g/ml HSA for 16 hours at 30 °C. The cells were removed by centrifugation and the media was lyophilized. The lyophilized powder was reconstituted in 2 ml of distilled $H_2O$ and 10 volumes of acetone were added. The solution was thoroughly mixed and then centrifuged at 10,000 x g for 10 minutes in a Sorvall RT6000 centrifuge. (Sorvall Instruments, Wilmington, DE). After removal of supernatant, the pellet was resuspended in 1 ml of distilled $H_2O$ and 10 volumes of methanol added. This solution was thoroughly mixed and again centrifuged at 20,000 x g in a Sorvall RC5B centrifuge for 10 minutes. One-half of the solution was dried by rotary evacuation on a Savant-type evaporator ("methanol soluble" fraction; see Table I). The remaining solution was diluted 1:1 with 0.5 M acetic acid and applied to a 3 ml column of SP-Sephadex® (Sigma Chemical Co.) equilibrated in 0.5 M acetic acid. The column was washed with 15 ml 0.5

M acetic acid and then eluted with 6 ml of 1.0 M ammonium acetate. The eluted material was then dried by lyophilization ("post SP-Sephadex" fraction of Table VII). The dried methanol soluble and ammonium acetate eluted material was resolubilized in 0.5 ml of distilled $H_2O$ and tested for biological activity using the precontracted rabbit thoracic aortic ring model described in Kleinert, et al., Hypertension 6:Suppl. 1:143-146 (1984). Equal volumes of material reconstituted from crude lyophilized media, methanol soluble protein or protein eluted from SP-Sephadex® with 1.0 M ammonium acetate were compared using aortic rings precontracted with 5 μM histamine. Material synthesized by yeast cultures whose plasmids encoded proANVP (amino acids 26-152) (YEp-α-NF-9) and proANVP (amino acids 121-152) (YEp-α-NF-5), as well as the corresponding inverse orientations (YEp-α-NF-12 and YEp-α-NF-7, respectively), were compared. The results are depicted in Table VII. Significant vasodilatory activity was detected in the methanol soluble material as well as the post SP-Sephadex material in extracts from cells containing YEp-α-NF-5 and YEp-α-NF-9. Extracts from cells containing inverse orientation DNA plasmids were not active. This finding, as well as the immunoprecipitation data shown in Figure 16, demonstrates that yeast process full-length and smaller proANVP fragments containing mature ANVP into a form that exhibits potent biological activity.

Table VII

Vasorelaxant Properties of Proatrial natriuretic/vasodilator polypeptides and Proatrial natriuretic/vasodilator polypeptides Fragments Expressed by S. cerevisiae

| Media Sample | % Relaxation |
|---|---|
| YEp-α-NF-5 (methanol soluble) | 100.0 |
| YEp-α-NF-5 (post SP-Sephadex) | 65.5 |
| YEp-α-NF-7 (methanol soluble) | 11.7 |
| YEp-α-NF-7 (post SP-Sephadex) | 3.3 |
| YEp-α-NF-9 (methanol soluble) | 71.2 |
| YEp-α-NF-9 (post SP-Sephadex) | 48.2 |
| YEp-α-NF-12 (methanol soluble) | 13.2 |
| YEp-α-NF-12 (post SP-Sephadex) | 6.5 |

Aortic rings were precontracted with 5μM histamine. The aortic rings were then treated with proteins obtained from S. cerevisiae cultures. The proteins were purified by acetone/methanol treatment of culture media and the indicated fractions were passed over SP-Sephadex. YEp-α-NF-5 and YEp-α-NF-9 contained proANVP cDNA in its correct orientation and YEp-α-NF-7 and YEp-α-NF-12 contained DNA in an inverse orientation. Data are expressed as percent relaxation of the precontracted rings as described in Kleinert, supra.

Samples of the above expressed material were also tested for natriuretic and diuretic activity using the isolated perfused rat kidney model as described by Camargo, M. et al., Am. J. Physiol. 246:F447-F456-(1984). As shown in Table VIII material synthesized and secreted by yeast cultures containing YEp-α-NF-5 and purified as described above through the SP-Sephadex step increased urinary $Na^+$ excretion approximately 3-fold and urinary volume 2-fold as examined during repeated test periods of 10 minutes each. Glomerular filtration also increased in this experiment consistent with the diuretic action. No significant increase occurred in urinary $Na^+$ excretion, urinary volume, glomerular filtration or renal resistance when the same experiment was performed on material synthesized and secreted by yeast cultures containing the reverse orientation control plasmid YEp-α-NF-7 and purified through SP-Sephadex®.

Table VIII

Effects of Atrial natriuretic/vasodilator polypeptides Expressed and Secreted by S. cerevisiae on Renal Function in the Isolated Perfused Rat Kidney

| | Control | YEp-α-NF-5 | YEp-α-NF-7 |
|---|---|---|---|
| Urinary Sodium (μEq/min) | 3.56 | 11.05 | 3.78 |
| Urinary Volume (μl/min) | 6.15 | 14.0 | 7.24 |

Results represent the average of two ten minute control periods followed by the addition of 50 μl of SP-Sephadex purified protein. Experimental measurements represent the average of values obtained during three successive ten minute periods.

The human proANVP(128-151) purified as described in Section IV.B.2.d. was subjected to assays as described above and displayed the full range of biological activities.

In a manner similar to the yeast-expressed active material, rat and human pre-proANVPs, proANVPs and ANVPs expressed by the bacterial and mammalian cell expression systems described in Section IV.A and IV.C, respectively, have been shown to possess biological activity.

The rat proANVP fragments 87-152 and 26-152 whose syntheses were directed by plasmids pRNF-6852 and pRNF-12852, respectively, in E. coli were extracted from 1 liter of bacterial cells as follows. The cells were collected by centrifugation at 5,000 x g for 60 minutes and resuspended in 10 ml of 50 mM Tris, pH 7.5. This suspension was sonicated for 1 minute using a Heat Systems ultrasonic sonicator (Heat Systems, Farmingdale, NY) at setting 4. The sonicate was then centrifuged at 105,000 x g to remove particulate matter and the resulting supernatent was saved and called crude bacterial extract. A fraction of the crude bacteria extract was subsequently boiled for 5 minutes and lowered to pH 2.5 for 1 hour. The pH was then neutralized and both the resulting boiled-acid extract and crude bacterial extract were applied to rabbit thoracic aortic rings as described. As shown in Table IX, both the crude bacterial extracts and boiled-acid extracts from extracts containing pRNF-6852 and pRNF-12852 relaxed the precontracted tissue. Control samples from bacteria containing the pKT52 vector without proANVP DNA were inactive. Thus, in a manner similar to the yeast expression products, the bacterial rat proANVP fragments were vasodilatory. Furthermore, since a fraction of these samples were boiled and acid extracted to prevent subsequent processing, without a loss of biological activity relative to the crude bacterial extract, it was believed that the entire 68 amino acid fragments were biologically active.

Purified human proANVP(26-151) prepared from E. coli as described in Section IV.A.5 was shown to have a profound effect on vascular tissue.

Table IX

| Vasorelaxant Activities of Proatrial Natriuretic/Vasodilator Polypeptide Fragments Expressed in E. coli | |
|---|---|
| Sample | % Relaxation |
| Control (pKT52)<br>prorANVP(87-152)<br>prorANVP(25-152) | 8 ± 2<br>74 ± 8<br>65 ± 9 |
| Rabbit thoracic aortic rings were precontracted with 5 µM histamine. Data are expressed as the % relaxation of the precontracted rings. | |

In the case of mammalian cell expression, both prorANVP(25-152) and prohANVP(126-151) produced in CHO cells exhibited biological activity similar to that observed above.

The proANVP proteins expressed using the above described recombinant DNA techniques in yeast, bacteria and mammalian cells all contain the common ANVP sequence:

```
H-Arg-Ser-Ser-Cys-Phe-Gly-Gly-Arg-
|Met/Ile|-Asp-Arg-Ile-Gly-Ala-Gln-Ser-Gly-
Leu-Gly-Cys-Asn-Ser-Phe-Arg-Tyr-OH
```

where the bracketed amino acid residue is Met in human ANVPs and Ile in rat ANVPs. The proANVP fragments expressed in the systems described above show potent vasorelaxant, natriuretic and diuretic activities (Tables VII, VIII and IX). These same and related activities have also been demonstrated with rANVP)126-150) and hANVP(127-151). Thus, these expressed fragments should share all properties with a synthetic ANVP, both on in vitro preparations and when injected in vivo into humans or animals. As shown in Table V, synthetic ANVPs lowered mean arterial blood pressure, plasma renin activity and plasma aldosterone, and increased urine volume and urine sodium excretion. These are desirable properties for diuretic and antihypertensive agents. Furthermore, proANVPs and ANVPs act at all major foci of volume and blood pressure regulation. Thus, it can be concluded that pre-proANVPs, proANVPs, and ANVPs, when produced by recombinant DNA methods and expressed in yeast, bacteria or mammalian cells in a manner comparable to that described above, will find utility in the acute and chronic treatment of edematous states (i.e. congestive heart failure, nephrotic syndrome, hepatic cirrhosis and ineffective renal perfusion) and in the chronic treatment of renal insufficiencies and hypertension.

As demonstrated in the above examples, the methods and compositions disclosed can find use in expressing pre-proANVPs, proANVPs and fragments thereof, and ANVPs. It is apparent to one having ordinary skill in the art that any sequence or fragment of polypeptide disclosed herein can be expressed by employing minor modifications to this disclosure, while remaining within the scope of the invention.

In particular, any fragment of the pre-proANVPs amino acid sequence disclosed can be expressed. Certain of these fragments will not contain the complete sequence of ANVP, but may contain portions of ANVPs. These fragments can themselves demonstrate biological activity comparable with or complementary to the activities disclosed for the instant compounds.

In addition, human-derived proANVPs, fragments thereof and ANVPs contain a single amino acid replacement compared to the biologically active rat-derived polypeptides. Therefore yeast, bacterial and mammalian cell expressed human-derived compounds, prepared in the manner previously described, will display similar biological activity.

V. Production of Antibody to Atrial Natriuretic/Vasodilator Polypeptides Compounds

Compounds of the present invention were used to provide immunoassays, particularly radioimmunoassays, for the determination of the presence or amount of ANVPs in samples.

Antibody to ANVPs was produced by immunizing New Zealand white rabbits subcutaneously and intramuscularly with 250 µg ANVP conjugated to bovine serum albumin in complete Freund's adjuvant. Rabbits were boosted at three week intervals with an identical quantity of conjugate in incomplete Freund's adjuvant. The rabbits were bled from the ear artery 7-10 days after a boost and the resulting serum was

tested for its ability to bind ANVP. Parallel control non-immune serum samples were also tested. Table V presents data from a representative experiment in which the ability of antisera to interact specifically with ANVPs was examined. ANVP (500 nanograms) was immobilized at individual wells of a polystyrene plate. Varying dilutions of antisera wore then added to these wells and the amount of antibodies specifically bound was quantified by adding $^{125}$I-labeled sheep anti-rabbit IgG antisera. This is a standard method for determining specific antibody titres. As shown in Table X, at a serum dilution of 1:400 significant quantities of antibody were still binding to ANVPs. Specific binding was not observed with non-immune serum.

Table X

| Specific Binding of Anti-Atrial natriuretic/vasodilator polypeptides Antisera to Immobilized Atrial natriuretic/vasodilator polypeptides Compounds | | |
|---|---|---|
| Antiserum Dilution | Antibody Bound(CPM) | |
| | Immune | Non-Immune |
| 1:10 | 7481 | 734 |
| 1:50 | 6977 | 681 |
| 1:100 | 6135 | 685 |
| 1:200 | 5096 | 634 |
| 1:400 | 3898 | 525 |

The experiment depicted in Table XI is identical to that of Table X, however, varying concentrations of non-immobilized ANVPs were added concurrently with a 1:100 dilution of anti-ANVP antiserum. As shown, non-immobilized ANVPs competitively displaced antibody binding from immobilized ANVPs. Thus, this serves as an example of a competitive displacement assay. This assay and similar radioimmunoassays, can be used to quantify ANVP-like immunoreactivity in tissues or serum under a variety of physiological or pathophysiological states. Thus far, the assay has been used to detect ANVP in atrial extracts. ANVP was not detected in ventricular extracts.

Table XI

| Competitive Displacement of Anti-Atrial natriuretic/vasodilator polypeptides Binding To Immobilized Atrial natriuretic/vasodilator polypeptides by Addition of Free Atrial natriuretic/vasodilator polypeptides | |
|---|---|
| Conc. Free ANVP (nmoles) | Antibody Bound (CPM) |
| 0 | 6777 |
| 0.002 | 6343 |
| 0.02 | 5603 |
| 0.2 | 2893 |
| 2.0 | 1223 |

Although the foregoing invention has been described in some detail by way of clarity and for purposes of understanding, it will be understood by those skilled in the art that modifications of the invention may be practiced while remaining within the spirit and scope of the appended claims.

## Claims

**1.** A polypeptide compound useful as a natriuretic, diuretic, and/or vasodilator in mammals, said polypeptide compound having the formula:

1 2 3 4 5 8 10 11

X-Arg-Ser-Ser-Cys-Phe-$AA_6$-Gly-Arg-$AA_9$-Asp-Arg-Ile-$AA_{13}$-

14 15 16 18 19 20

Ala-Gln-Ser-$AA_{17}$-Leu-Gly-Cys-Y

wherein

X is hydrogen, acetyl, or an oligopeptide of up to 125 amino acid residues including the N-acetyl derivatives thereof;

Y is hydroxyl, amido, Asn-OH, Asn-Ser-OH, Asn-Ser-Phe-OH, Asn-Ser-Phe-Arg-OH, Asn-Ser-Phe-Arg-Tyr-OH, or the C-terminal amide derivatives thereof; or an oligopeptide extension of up to 20 amino acid residues from the C-terminus of any of the foregoing including the C-terminal amide derivatives thereof;

each of $AA_6$, $AA_{13}$, and $AA_{17}$ is independently selected from the group consisting of Gly and Ala; $AA_9$ is selected from Ile, Met, and Val;

wherein 0, 1 or 2 of residues 1-20 of formulas (1) are substituted by the D-isomer thereof, and including such compound containing a disulfide bridge joining the cysteine residues and the pharmacologically acceptable salts thereof, with the proviso that when $AA_6$, $AA_7$, $AA_{13}$, and $AA_{17}$ are Gly, $AA_9$ is Ile or Met and Y is Asn-OH, Asn-Ser-OH, Asn-Ser-Phe-OH, Asn-Ser-Phe-Arg-OH, Asn-Ser-Phe-Arg-Tyr-OH, then X is H or acetyl or at least one amino acid residue is a D-isomer.

2. A polypeptide compound of claim 1 wherein the compound is selected from the group consisting of

H-Arg-Ser-Ser-Cys-Phe-Gly-Gly-Arg-(D-Met)-Asp-Arg-Ile-Gly-Ala-Gln-Ser-Gly-Leu-Gly-Cys-Asn-Ser-Phe-Arg-Tyr-OH

H-Arg-Ser-Ser-(D-Cys)-Phe-Gly-Gly-Arg-Ile-Asp-Arg-Ile-Gly-Ala-Gln-Ser-Gly-Leu-Gly-Cys-Asn-Ser-Phe-Arg-Tyr-OH

H-Arg-Ser-Ser-Cys-(D-Phe)-Gly-Gly-Arg-Ile-Asp-Arg-Ile-Gly-Ala-Gln-Ser-Gly-Leu-Gly-Cys-Asn-Ser-Phe-Arg-Tyr-OH

H-Arg-Ser-Ser-Cys-Phe-(D-Ala)-Gly-Arg-Ile-Asp-Arg-Ile-Gly-Ala-Gln-Ser-Gly-Leu-Gly-Cys-Asn-Ser-Phe-Arg-Tyr-OH

H-Arg-Ser-Ser-Cys-Phe-Gly-(D-Ala)-Arg-Ile-Asp-Arg-Ile-Gly-Ala-Gln-Ser-Gly-Leu-Gly-Cys-Asn-Ser-Phe-Arg-Tyr-OH

H-Arg-Ser-Ser-Cys-Phe-Gly-Gly-(D-Arg)-Ile-Asp-Arg-Ile-Gly-Ala-Gln-Ser-Gly-Leu-Gly-Cys-Asn-Ser-Phe-Arg-Tyr-OH

H-Arg-Ser-Ser-Cys-Phe-Gly-Gly-Arg-(D-Val)-Asp-Arg-Ile-Gly-Ala-Gln-Ser-Gly-Leu-Gly-Cys-Asn-Ser-Phe-Arg-Tyr-OH

H-Arg-Ser-Ser-Cys-Phe-Gly-Gly-Arg-Ile-Asp-(D-Arg)-Ile-Gly-Ala-Gln-Ser-Gly-Leu-Gly-Cys-Asn-Ser-Phe-Arg-Tyr-OH

H-Arg-Ser-Ser-Cys-Phe-Gly-Gly-Arg-Ile-Asp-Arg-(D-Val)-Gly-Ala-Gln-Ser-Gly-Leu-Gly-Cys-Asn-Ser-Phe-Arg-Tyr-OH

H-Arg-Ser-Ser-Cys-Phe-Gly-Gly-Arg-Ile-Asp-Arg-Ile-(D-Ala)-Ala-Gln-Ser-Gly-Leu-Gly-Cys-Asn-Ser-Phe-Arg-Tyr-OH

H-Arg-Ser-Ser-Cys-Phe-Gly-Gly-Arg-Ile-Asp-Arg-Ile-Gly-(D-Ala)-Gln-Ser-Gly-Leu-Gly-Cys-Asn-Ser-Phe-Arg-Tyr-OH

H-Arg-Ser-Ser-Cys-Phe-Gly-Gly-Arg-Ile-Asp-Arg-Ile-Gly-Ala-(D-Gln)-Ser-Gly-Leu-Gly-Cys-Asn-Ser-Phe-Arg-Tyr-OH

H-Arg-Ser-Ser-Cys-Phe-Gly-Gly-Arg-Ile-Asp-Arg-Ile-Gly-Ala-Gln-(D-Ser)-[Ser-]Gly-Leu-Gly-Cys-Asn-Ser-Phe-Arg-Tyr-OH

H-Arg-Ser-Ser-Cys-Phe-Gly-Gly-Arg-Ile-Asp-Arg-Ile-Gly-Ala-Gln-Ser-(D-Ala)-Leu-Gly-Cys-Asn-Ser-Phe-Arg-Tyr-OH

H-Arg-Ser-Ser-Cys-Phe-Gly-Gly-Arg-Ile-Asp-Arg-Ile-Gly-Ala-Gln-Ser-Gly-(D-Leu)-Gly-Cys-Asn-Ser-Phe-Arg-Tyr-OH

H-Arg-Ser-Ser-Cys-Phe-Gly-Gly-Arg-Ile-Asp-Arg-Ile-Gly-Ala-Gln-Ser-Gly-Leu-Gly-Cys-Asn-Ser-Phe-Arg-OH;

H-Arg-Ser-Ser-Cys-Phe-Gly-Gly-Arg-Ile-Asp-Arg-Ile-Gly-Ala-Gln-Ser-Gly-Leu-Gly-Cys-Asn-Ser-Phe-Arg-Tyr-OH;

H-Arg-Ser-Ser-Cys-Phe-Gly-Gly-Arg-Met-Asp-Arg-Ile-Gly-Ala-Gln-Ser-Gly-Leu-Gly-Cys-Asn-Ser-Phe-Arg-OH;

H-Arg-Ser-Ser-Cys-Phe-Gly-Gly-Arg-Met-Asp-Arg-Ile-Gly-Ala-Gln-Ser-Gly-Leu-Gly-Cys-Asn-Ser-Phe-Arg-Tyr-OH;

including the C-terminal amides thereof and such compound containing a disulfide bridge joining the cysteine residues, and the pharmacologically acceptable salts thereof.

3. A polypeptide compound of claim 1 which is of the formula

H-Arg-Ser-Ser-Cys-Phe-Gly-Gly-Arg-Met-Asp-Arg-Ile-Gly-
Ala-Gln-Ser-Gly-Leu-Gly-Cys-Asn-Ser-Phe-Arg-Tyr-OH;

including the C-terminal amides thereof and such compound containing a disulfide bridge joining the cysteine residues, and the pharmacologically acceptable salts thereof.

4. A pharmaceutical composition comprising a polypeptide compound in accordance with any of claims 1-3 in a therapeutically effective amount, together with a physiologically suitable carrier.

5. A method for the preparation of any of the peptides according to claims 1 to 3 characterized in that per se known solid-phase techniques are employed, whereby the respective amino acids having protective groups are used.

6. A composition of DNA molecules consisting of DNA molecules which encode the polypeptide of any of claims 1-3.

7. A recombinant expression system capable of expressing, when transformed into a compatible host, a DNA sequence encoding the polypeptide of any of claims 1-3.

8. A recombinant host transformed with the expression system of claim 7.

9. A method to produce the polypeptide of any of claims 1-3, which method comprises culturing a recombinant host transformed with a recombinant expression system capable of expressing, when transformed into a compatible host, a DNA sequence encoding the polypeptide of any of claims 1-3 and recovering the polypeptide.

**Patentansprüche**

1. Polypeptidverbindung, die als ein natriumuretisches, diuretisches oder gefäßerweiterndes Mittel in Säugetieren verwendbar ist, wobei die Polypeptidverbindung die Formel:

```
  1   2   3   4   5             8        10  11
X-Arg-Ser-Ser-Cys-Phe-AA6-Gly-Arg-AA9-Asp-Arg-Ile-AA13-

  14  15  16        18  19  20
  Ala-Gln-Ser-AA17-Leu-Gly-Cys-Y
```

besitzt, worin
X Wasserstoff, Acetyl oder ein Oligopeptid von bis zu 125 Aminosäureresten, einschließlich der N-Acetyl-Derivate davon, ist;
Y für Hydroxyl, Amido, Asn-OH, Asn-Ser-OH, Asn-Ser-Phe-OH, Asn-Ser-Phe-Arg-OH, Asn-Ser-Phe-Arg-Tyr-OH, oder für die C-terminalen Amid-Derivate davon, steht; oder für eine Oligopeptidausdehnung von bis zu 20 Aminosäureresten vom C-Terminus jedes der vorstehend genannten aus, einschließlich der C-terminalen Amid-Derivate davon;
jede unter $AA_6$, $AA_{13}$ und $AA_{17}$ unabhängig aus der aus Gly und Ala bestehenden Gruppe gewählt ist;
$AA_9$ aus Ile, Met und Val gewählt ist;
worin 0, 1 oder 2 der Reste 1-20 der Formeln (1) mit einem D-Isomer davon substituiert sind, und einschließlich einer solchen Verbindung, die eine Disulfidbrücke enthält, welche die Cysteinreste verbindet, und der pharmakologisch annehmbaren Salze davon, mit der Maßgabe, daß, wenn $AA_6$, $AA_7$, $AA_{13}$ und $AA_{17}$ Gly sind, $AA_9$ Ile oder Met ist und Y Asn-OH, Asn-Ser-OH, Asn-Ser-Phe-OH, Asn-Ser-Phe-Arg-OH, Asn-Ser-Phe-Arg-Tyr-OH ist, X dann H oder Acetyl ist, oder mindestens ein Aminosäurerest ein D-Isomer ist.

**2.** Polypeptidverbindung nach Anspruch 1, wobei die Verbindung aus der Gruppe gewählt wird, welche besteht aus:

H-Arg-Ser-Ser-Cys-Phe-Gly-Gly-Arg-(D-Met)-Asp-Arg-Ile-Gly-Ala-Gln-Ser-Gly-Leu-Gly-Cys-Asn-Ser-Phe-Arg-Tyr-OH

H-Arg-Ser-Ser-(D-Cys)-Phe-Gly-Gly-Arg-Ile-Asp-Arg-Ile-Gly-Ala-Gln-Ser-Gly-Leu-Gly-Cys-Asn-Ser-Phe-Arg-Tyr-OH

H-Arg-Ser-Ser-Cys-(D-Phe)-Gly-Gly-Arg-Ile-Asp-Arg-Ile-Gly-Ala-Gln-Ser-Gly-Leu-Gly-Cys-Asn-Ser-Phe-Arg-Tyr-OH

H-Arg-Ser-Ser-Cys-Phe-(D-Ala)-Gly-Arg-Ile-Asp-Arg-Ile-Gly-Ala-Gln-Ser-Gly-Leu-Gly-Cys-Asn-Ser-Phe-Arg-Tyr-OH

H-Arg-Ser-Ser-Cys-Phe-Gly-(D-Ala)-Arg-Ile-Asp-Arg-Ile-Gly-Ala-Gln-Ser-Gly-Leu-Gly-Cys-Asn-Ser-Phe-Arg-Tyr-OH

H-Arg-Ser-Ser-Cys-Phe-Gly-Gly-(D-Arg)-Ile-Asp-Arg-Ile-Gly-Ala-Gln-Ser-Gly-Leu-Gly-Cys-Asn-Ser-Phe-Arg-Tyr-OH

H-Arg-Ser-Ser-Cys-Phe-Gly-Gly-Arg-(D-Val)-Asp-Arg-Ile-Gly-Ala-Gln-Ser-Gly-Leu-Gly-Cys-Asn-Ser-Phe-Arg-Tyr-OH

H-Arg-Ser-Ser-Cys-Phe-Gly-Gly-Arg-Ile-Asp-(D-Arg)-Ile-Gly-Ala-Gln-Ser-Gly-Leu-Gly-Cys-Asn-Ser-Phe-Arg-Tyr-OH

H-Arg-Ser-Ser-Cys-Phe-Gly-Gly-Arg-Ile-Asp-Arg-(D-Val)-Gly-Ala-Gln-Ser-Gly-Leu-Gly-Cys-Asn-Ser-Phe-Arg-Tyr-OH

H-Arg-Ser-Ser-Cys-Phe-Gly-Gly-Arg-Ile-Asp-Arg-Ile-(D-Ala)
-Ala-Gln-Ser-Gly-Leu-Gly-Cys-Asn-Ser-Phe-Arg-Tyr-OH

H-Arg-Ser-Ser-Cys-Phe-Gly-Gly-Arg-Ile-Asp-Arg-Ile-Gly-
(D-Ala)-Gln-Ser-Gly-Leu-Gly-Cys-Asn-Ser-Phe-Arg-Tyr-OH

H-Arg-Ser-Ser-Cys-Phe-Gly-Gly-Arg-Ile-Asp-Arg-Ile-Gly-
Ala-(D-Gln)-Ser-Gly-Leu-Gly-Cys-Asn-Ser-Phe-Arg-Tyr-OH

H-Arg-Ser-Ser-Cys-Phe-Gly-Gly-Arg-Ile-Asp-Arg-Ile-Gly-
Ala-Gln-(D-Ser)-[Ser-]Gly-Leu-Gly-Cys-Asn-Ser-Phe-Arg-Tyr
-OH

H-Arg-Ser-Ser-Cys-Phe-Gly-Gly-Arg-Ile-Asp-Arg-Ile-Gly-Ala
-Gln-Ser-(D-Ala)-Leu-Gly-Cys-Asn-Ser-Phe-Arg-Tyr-OH

H-Arg-Ser-Ser-Cys-Phe-Gly-Gly-Arg-Ile-Asp-Arg-Ile-Gly-
Ala-Gln-Ser-Gly-(D-Leu)-Gly-Cys-Asn-Ser-Phe-Arg-Tyr-OH

H-Arg-Ser-Ser-Cys-Phe-Gly-Gly-Arg-Ile-Asp-Arg-Ile-Gly-
Ala-Gln-Ser-Gly-Leu-Gly-Cys-Asn-Ser-Phe-Arg-OH;

H-Arg-Ser-Ser-Cys-Phe-Gly-Gly-Arg-Ile-Asp-Arg-Ile-Gly-
Ala-Gln-Ser-Gly-Leu-Gly-Cys-Asn-Ser-Phe-Arg-Tyr-OH;

H-Arg-Ser-Ser-Cys-Phe-Gly-Gly-Arg-Met-Asp-Arg-Ile-Gly-
Ala-Gln-Ser-Gly-Leu-Gly-Cys-Asn-Ser-Phe-Arg-OH;

H-Arg-Ser-Ser-Cys-Phe-Gly-Gly-Arg-Met-Asp-Arg-Ile-Gly-
Ala-Gln-Ser-Gly-Leu-Gly-Cys-Asn-Ser-Phe-Arg-Tyr-OH;

einschließlich der C-terminalen Amide davon und einer derartigen Verbindung, die eine Disulfidbrücke enthält, welche die Cysteinreste verbindet, und der pharmakologisch annehmbaren Salze davon.

3. Polypeptidverbindung nach Anspruch 1, die die Formel

H-Arg-Ser-Ser-Cys-Phe-Gly-Gly-Arg-Met-Asp-Arg-Ile-Gly-
Ala-Gln-Ser-Gly-Leu-Gly-Cys-Asn-Ser-Phe-Arg-Tyr-OH

besitzt, einschließlich der C-terminalen Amide davon und einer derartigen Verbindung, die eine Disulfidbrücke enthält, welche die Cysteinreste verbindet, und der pharmakologisch annehmbaren Salze davon.

4. Pharmazeutische Zusammensetzung, umfassend eine Polypeptidverbindung gemäß mindestens einem der Ansprüche 1-3 in einer therapeutisch wirksamen Menge, zusammen mit einem physiologisch geeigneten Träger.

5. Verfahren zur Herstellung eines der Peptide nach Anspruch 1 bis 3**, dadurch gekennzeichnet**, daß per se bekannte Festphasen-Techniken angewandt werden, wobei die jeweiligen Aminosäuren mit Schutzgruppen verwendet werden.

6. Zusammensetzung von DNA-Molekülen, bestehend aus DNA-Molekülen, welche für das Polypeptid aus mindestens einem der Ansprüche 1-3 codieren.

7. Rekombinantes Expressionssystem, das in der Lage ist, wenn in einen kompatiblen Wirt transformiert, eine für das Polypeptid aus mindestens einem der Ansprüche 1-3 codierende DNA-Sequenz zu exprimieren.

8. Rekombinanter Wirt, der mit dem Expressionssystem aus Anspruch 7 transformiert ist.

9. Verfahren, um das Polypeptid aus mindestens einem der Ansprüche 1-3 herzustellen, welches Verfahren die Kultivierung eines rekombinanten Wirtes, der mit einem rekombinanten Expressionssystem, das in der Lage ist, wenn in einen kompatiblen Wirt transformiert, eine für das Polypeptid aus mindestens einem der Ansprüche 1-3 codierende DNA-Sequenz zu exprimieren, transformiert ist, und das Gewinnen des Polypeptids umfaßt.

## Revendications

1. Composé polypeptidique utile en tant que natriurétique, diurétique et/ou vasodilatateur chez les mammifères, ledit composé polypeptidique présentant la formule :

1 2 3 4 5 8 10 11 14
X–Arg–Ser–Ser–Cys–Phe–$AA_6$–Gly–Arg–$AA_9$–Asp–Arg–Ile–$AA_{13}$–Ala–

15 16 18 19 20
Gln–Ser–$AA_{17}$–Leu–Gly–Cys–Y

où
X est l'hydrogène, un groupe acétyle ou un oligopeptide ayant jusqu'à 125 résidus d'acides aminés y compris leurs dérivés N-acétylés,
Y est un groupe hydroxyle, amido, Asn-OH, Asn-Ser-OH, Asn-Ser-Phe-OH, Asn-Ser-Phe-Arg-OH, Asn-Ser-Phe-Arg-Tyr-OH, ou leurs dérivés amidés C-terminaux, ou un prolongement oligopeptidique ayant jusqu'à 20 résidus d'acides aminés depuis l'extrémité C-terminale de l'un quelconque des précédents, y compris leurs dérivés amidés C-terminaux,
chacun des symboles $AA_6$, $AA_{13}$ et $AA_{17}$ est choisi indépendamment dans le groupe consistant en Gly et Ala ; $AA_9$ est choisi parmi Ile, Met et Val,

où 0, 1 ou 2 des résidus 1 à 20 de la formule (1) sont remplacés par leur isomère D, et comprenant un tel composé contenant un pont disulfure reliant les résidus cystéine et ses sels pharmacologiquement acceptables, à condition que lorsque $AA_6$, $AA_7$, $AA_{13}$ et $AA_{17}$ sont Gly, $AA_9$ est Ile ou Met et Y est Asn-OH, Asn-Ser-OH, Asn-Ser-Phe-OH, Asn-Ser-Phe-Arg-OH, Asn-Ser-Phe-Arg-Tyr-OH, X soit H ou un groupe acétyle ou au moins un résidu d'acide aminé soit un isomère D.

**2.** Composé polypeptidique selon la revendication 1, dans lequel le composé est choisi dans le groupe consistant en :

H–Arg–Ser–Ser–Cys–Phe–Gly–Gly–Arg–(D–Met)–Asp–Arg–Ile–Gly–Ala–
Gln–Ser–Gly–Leu–Gly–Cys–Asn–Ser–Phe–Arg–Tyr–OH

H–Arg–Ser–Ser–(D–Cys)–Phe–Gly–Gly–Arg–Ile–Asp–Arg–Ile–Gly–Ala–Gln–Ser–Gly–Leu–Gly–Cys–Asn–Ser–Phe–Arg–Tyr–OH

H–Arg–Ser–Ser–Cys–(D–Phe)–Gly–Gly–Arg–Ile–Asp–Arg–Ile–Gly–Ala–Gln–Ser–Gly–Leu–Gly–Cys–Asn–Ser–Phe–Arg–Tyr–OH

H–Arg–Ser–Ser–Cys–Phe–(D–Ala)–Gly–Arg–Ile–Asp–Arg–Ile–Gly–Ala–Gln–Ser–Gly–Leu–Gly–Cys–Asn–Ser–Phe–Arg–Tyr–OH

H–Arg–Ser–Ser–Cys–Phe–Gly–(D–Ala)–Arg–Ile–Asp–Arg–Ile–Gly–Ala–Gln–Ser–Gly–Leu–Gly–Cys–Asn–Ser–Phe–Arg–Tyr–OH

H–Arg–Ser–Ser–Cys–Phe–Gly–Gly–(D–Arg)–Ile–Asp–Arg–Ile–Gly–Ala–Gln–Ser–Gly–Leu–Gly–Cys–Asn–Ser–Phe–Arg–Tyr–OH

H–Arg–Ser–Ser–Cys–Phe–Gly–Gly–Arg–(D–Val)–Asp–Arg–Ile–Gly–Ala–Gln–Ser–Gly–Leu–Gly–Cys–Asn–Ser–Phe–Arg–Tyr–OH

H–Arg–Ser–Ser–Cys–Phe–Gly–Gly–Arg–Ile–Asp–(D–Arg)–Ile–Gly–Ala–Gln–Ser–Gly–Leu–Gly–Cys–Asn–Ser–Phe–Arg–Tyr–OH

H–Arg–Ser–Ser–Cys–Phe–Gly–Gly–Arg–Ile–Asp–Arg–(D–Val)–Gly–Ala–Gln–Ser–Gly–Leu–Gly–Cys–Asn–Ser–Phe–Arg–Tyr–OH

H–Arg–Ser–Ser–Cys–Phe–Gly–Gly–Arg–Ile–Asp–Arg–Ile–(D–Ala)–Ala–Gln–Ser–Gly–Leu–Gly–Cys–Asn–Ser–Phe–Arg–Tyr–OH

H–Arg–Ser–Ser–Cys–Phe–Gly–Gly–Arg–Ile–Asp–Arg–Ile–Gly–(D–Ala)–Gln–Ser–Gly–Leu–Gly–Cys–Asn–Ser–Phe–Arg–Tyr–OH

H–Arg–Ser–Ser–Cys–Phe–Gly–Gly–Arg–Ile–Asp–Arg–Ile–Gly–Ala–(D–Gln)–Ser–Gly–Leu–Gly–Cys–Asn–Ser–Phe–Arg–Tyr–OH

H–Arg–Ser–Ser–Cys–Phe–Gly–Gly–Arg–Ile–Asp–Arg–Ile–Gly–Ala–Gln–(D–Ser)–[Ser]–Gly–Leu–Gly–Cys–Asn–Ser–Phe–Arg–Tyr–OH

H-Arg-Ser-Ser-Cys-Phe-Gly-Gly-Arg-Ile-Asp-Arg-Ile-Gly-Ala-Gln-Ser-(D-Ala)-Leu-Gly-Cys-Asn-Ser-Phe-Arg-Tyr-OH

H-Arg-Ser-Ser-Cys-Phe-Gly-Gly-Arg-Ile-Asp-Arg-Ile-Gly-Ala-Gln-Ser-Gly-(D-Leu)-Gly-Cys-Asn-Ser-Phe-Arg-Tyr-OH

H-Arg-Ser-Ser-Cys-Phe-Gly-Gly-Arg-Ile-Asp-Arg-Ile-Gly-Ala-Gln-Ser-Gly-Leu-Gly-Cys-Asn-Ser-Phe-Arg-OH

H-Arg-Ser-Ser-Cys-Phe-Gly-Gly-Arg-Ile-Asp-Arg-Ile-Gly-Ala-Gln-Ser-Gly-Leu-Gly-Cys-Asn-Ser-Phe-Arg-Tyr-OH

H-Arg-Ser-Ser-Cys-Phe-Gly-Gly-Arg-Met-Asp-Arg-Ile-Gly-Ala-Gln-Ser-Gly-Leu-Gly-Cys-Asn-Ser-Phe-Arg-OH

H-Arg-Ser-Ser-Cys-Phe-Gly-Gly-Arg-Met-Asp-Arg-Ile-Gly-Ala-Gln-Ser-Gly-Leu-Gly-Cys-Asn-Ser-Phe-Arg-Tyr-OH ;

y compris leurs amides C-terminaux et un tel composé contenant un pont disulfure reliant les résidus cystéine, et ses sels pharmacologiquement acceptables.

**3.** Composé polypeptidique selon la revendication 1, qui est de formule :

H-Arg-Ser-Ser-Cys-Phe-Gly-Gly-Arg-Met-Asp-Arg-Ile-Gly-Ala-Gln-Ser-Gly-Leu-Gly-Cys-Asn-Ser-Phe-Arg-Tyr-OH ;

y compris ses amides C-terminaux et un tel composé contenant un pont disulfure reliant les résidus cystéine, et ses sels pharmacologiquement acceptables.

**4.** Composition pharmaceutique comprenant un composé polypeptidique selon l'une quelconque des revendications 1 à 3 en une quantité thérapeutiquement efficace, ainsi qu'un véhicule physiologiquement approprié.

**5.** Procédé de préparation de l'un quelconque des peptides selon les revendications 1 à 3, caractérisé en ce que l'on emploie des techniques en phase solide connues en soi en utilisant les acides aminés respectifs avec des groupes protecteurs.

**6.** Composition de molécules d'ADN consistant en des molécules d'ADN qui codent le polypeptide selon l'une quelconque des revendications 1 à 3.

**7.** Système d'expression recombinant capable, lorsqu'il est utilisé pour transformer un hôte compatible, d'exprimer une séquence d'ADN codant le polypeptide selon l'une quelconque des revendications 1 à 3.

**8.** Hôte recombinant transformé avec le système d'expression selon la revendication 7.

**9.** Procédé pour produire le polypeptide selon l'une quelconque des revendications 1 à 3, lequel procédé comprend la culture d'un hôte recombinant transformé avec un système d'expression recombinant capable, lorsqu'il est utilisé pour transformer un hôte compatible, d'exprimer une séquence d'ADN codant le polypeptide selon l'une quelconque des revendications 1 à 3 et la récupération du polypeptide.

10 20 30 40 50 60 70
GGATCCATTT GTCTCGGGCT GCTGGCTGCC TGCCATTTCC TCCTCTCCAC CCTTATTTGG AGGCCCTGAC

80 90 100 110 120 130 140
AGCTGAGGCC ACAAACAAAC CAGGGGAGCT GGGCACCAGC CAAGCGTCAC CCTCTGTTTC CCCGCACGGG

150 160 170 180 190 200 210
TACCAGCGTC GAGGAGAAAG AATCCTGAGG CACGGCGGTG AGATAACCAA GGACTCTTTT TTACTCTTCT

220 230 240 250 260 270 280
CACACCTTTG AAGTGGGAGC CTCTTGAGTC AAATCAGTAA GAATGCGGCT CTTGCAGCTG AGGGTCTGGG

290 300 310 320 330 340 350
GGGCTGTTGG GGCTGCCCAA GGCAGAGAGG GGCTGTGACA AGCCCTGCGG GATGATAACT TTAAAAGGGC

360 370 380 390 400 410 420
ATCTCCTGCT GGCTTCTCAC TTGGCAGCTT TATCACTGCA AGTGACAGAA TGGGGAGGGT TCTGTCTCTC

430 440 450 460 470 480 490
CTGCGTGCTT GGAGAGCTGG GGGGCTATAA AAAGAGGCGG CACTGGGCAG CTGGGAGACA GGGACAGACG

500 510 520 530 540 550 560
TAGGCCAAGA GAGGGGAACC AGAGAGGAAC CAGAGGGGAG AGACAGAGCA GCAAGCAGTG GATTGCTCCT

570 588 603
TGACGACGCC AGC ATG AGC TCC TTC TCC ACC ACC ACC GTG AGC TTC CTC CTT TTA
MET Ser Ser Phe Ser Thr Thr Thr Val Ser Phe Leu Leu Leu
1

618 633 648 663
CTG GCA TTC CAG CTC CTA GGT CAG ACC AGA GCT AAT CCC ATG TAC AAT GCC GTG
Leu Ala Phe Gln Leu Leu Gly Gln Thr Arg Ala Asn Pro MET Tyr Asn Ala Val
26

678 693 706 716 726 736
TCC AAC GCA GAC CTG ATG GAT TTC AAG GTAGGGCCAG GAAAGCGGGT GCAGTCTGGG GCCAGGGGGC
Ser Asn Ala Asp Leu MET Asp Phe Lys

746 756 766 776 786 796 806
TTTCTGATGC TGTGCTCACT CCTCTTGATT TCCTCCAAGT CAGTGAGGTT TATCCCTTTC CCTGTATTTT

816 833 848
CCTTTTCTAA AG AAT TTG CTG GAC CAT TTG GAA GAA AAG ATG CCT TTA GAA GAT
Asn Leu Leu Asp His Leu Glu Glu Lys MET Pro Leu Glu Asp
50

863 878 893 908
GAG GTC GTG CCC CCA CAA GTG CTC AGT GAG CCG AAT GAA GAA GCG GGG GCT GCT
Glu Val Val Pro Pro Gln Val Leu Ser Glu Pro Asn Glu Glu Ala Gly Ala Ala

923 938 953 968
CTC AGC CCC CTC CCT GAG GTG CCT CCC TGG ACC GGG GAA GTC AGC CCA GCC CAG
Leu Ser Pro Leu Pro Glu Val Pro Pro Trp Thr Gly Glu Val Ser Pro Ala Gln

983 998 1013
AGA GAT GGA GGT GCC CTC GGG CGG GGC CCC TGG GAC TCC TCT GAT CGA TCT GCC
Arg Asp Gly Gly Ala Leu Gly Arg Gly Pro Trp Asp Ser Ser Asp Arg Ser Ala
100

1028 1043 1058 1073
CTC CTA AAA AGC AAG CTG AGG GCG CTG CTC ACT GCC CCT CGG AGC CTG CGG AGA
Leu Leu Lys Ser Lys Leu Arg Ala Leu Leu Thr Ala Pro Arg Ser Leu Arg Arg
127

FIG.__1A. SHEET I

```
                1088                    1103                    1118
TCC AGC TGC TTC GGG GGC AGG ATG GAC AGG ATT GGA GCC CAG AGC GGA CTG GGC
Ser Ser Cys Phe Gly Gly Arg MET Asp Arg Ile Gly Ala Gln Ser Gly Leu Gly

1133                       1155       1165       1175       1185       1195
TGT AAC AGC TTC CGG GTAAGAGGAA CTGGGGATGG AAATGGGATG GGATGGACAC TACTGGGAGA
Cys Asn Ser Phe Arg
                150
      1205       1215       1225       1235       1245       1255       1265
CACCTTCAGC AGGAAAGGGA CCAATGCAGA AGCTCATTCC CTCTCAAGTT TCTGCCCCAA CACCCAGAGT

      1275       1285       1295       1305       1315       1325       1335
GCCCCATGGG TGTCAGGACA TGCCATCTAT TGTCCTTAGC TAGTCTGCTG AGAAAATGCT TAAAAAAAAA

      1345       1355       1365       1375       1385       1395       1405
AGGGGGGGGG CTGGGCACGG TCGTCACGCC TGTAATCCCA GCACTTTGGG AGGCCAGGCA GCGGATCATG

      1415       1425       1435       1445       1455       1465       1475
AGGTCAAGAG ATCAAGACTA TCCTGGCCAA CATGGTGAAA CCCCAGCTCT ACTAAAAATA CAAAAATTAG

      1485       1495       1505       1515       1525       1535       1545
CTGGGTGTGT GGCGGGCACC TGTACTCTCA GCTACTTGGG AGGCTGAGGC AGGAGAATCA CTTGAACCCA

      1555       1565       1575       1585       1595       1605       1615
GGAGGCAGAG GTTGCAGTGA GCAGAGATCA CGCCACTGCA GTCCAGCCTA GGTGATAGAG CGAGACTGTC

      1625       1635       1645       1655       1665       1675       1685
TCAAAAAAAA AAAAAAAAGG CCAGGCGCGG TGGCTCACGC CTGTAATCCC AGCGCTTTGG GAGGCCAAGG

      1695       1705       1715       1725       1735       1745       1755
CGGGTGGATC ACGAGGTCAG GAGATGGAGA CCATCCTGGC TAACACGGTG AAACCCCGTC TCTACTAAAA

      1765       1775       1785       1795       1805       1815       1825
ATACAAAAAA TTAGCCAGGC GTGGTGGCCA GGCGCCTGTA AGTCCTAGCT ACTCCGGAGC TGAGGCAGGA

      1835       1845       1855       1865       1875       1885       1895
GAATGGCCGT GAACCCGGGA GGCGGAGCTT GCAGTGAGCA GAGATGGCAC CACTGCACTC CAGCCTGGGC

      1905       1915       1925       1935       1945       1955       1965
GACAGAGCAA GACTCCGTCT CAAAAAAAAA AAAAAAAAAA GCAACTGCCA CTAGCACTGG GAAATTAAAA

      1975       1985       1995       2005       2015       2025       2035
TATTCATAGA GCCAAGTTAT CTTTGCATGG CTGATTAGCA GTTCATATTC CTCCCCAGAA TTGCAAGATC

      2045       2055       2065       2075       2085       2095       2105
CTGAAGGGCT TAAGTGAAAT TTACTCTGAT GAGTAACTTG CTTATCAATT CATGAAGCTC AGAGGGTCAT

      2115       2125       2135       2145       2155       2165       2175
CAGGCTGGGG TGGGGGCCGG TGGGAAGCAG GTGGTCAGTA ATCAAGTTCA GAGGATGGGC ACACTCATAC

      2185       2195       2205       2215       2225       2235
ATGAAGCTGA CTTTTCCCAG GACAGCCAGG TCACCAAGCC AGATATGTCT GTGTTCTCTT TGCAG

                             2262       2272       2282       2292       2302
TAC TGA AGA TAA CAGCCAGGGA GGACAAGCAG GGCTGGGCCT AGGGACAGAC TGCAAGAGGC
Tyr     Arg

151
```

FIG.__1A. SHEET II

```
      2312       2322       2332       2342       2352       2362       2372
TCCTGTCCCC TGGGGTCTCT GCTGCATTTG TGTCATCTTG TTGCCATGGA GTTGTGATCA TCCCATCTAA

      2382       2392       2402       2412       2422       2432       2442
GCTGCAGCTT CCTGTCAACA CTTCTCACAT CTTATGCTAA CTGTAGATAA AGTGGTTTGA TGGTGACTTC

      2452       2462       2472       2482       2492       2502       2512
CTCGCCTCTC CCACCCCATG CATTAAATTT TAAGGTAGAA CCTCACCTGT TACTGAAAGT GGTTTGAAAG

      2522       2532       2542       2552       2562       2572       2582
TGAATAAACT TCAGCACCAT GGACAGAAGA CAAATGCCTG CGTTGGTGTG CTTTCTTTCT TCTTGGGAAG

AGAATTC
```

FIG._1A. SHEET III

```
1                                                                                   50
G GCA TTC CAG CTC CTA GGT CAG ACC AGA GCT AAT CCC ATG TAC AAT GCC GTG TCC AAC GCA GAC CTG ATG GAT
  Ala Phe Gln Leu Leu Gly Gln Thr Arg Ala Asn Pro Met Tyr Asn Ala Val Ser Asn Ala Asp Leu Met Asp
  16                                      26

                            100
TTC AAG AAT TTG CTG GAC CAT TTG GAA GAA AAG ATG CCT TTA GAA GAT GAG GTC GTG CCC CCA CAA GTG CTC
Phe Lys Asn Leu Leu Asp His Leu Glu Glu Lys Met Pro Leu Glu Asp Glu Val Val Pro Pro Gln Val Leu
                                        50

    150                                                             200
AGT GAG CCG AAT GAA GAA GCG GGG GCT GCT CTC AGC CCC CTC CCT GAG GTG CCT CCC TGG ACC GGG GAA GTC
Ser Glu Pro Asn Glu Glu Ala Gly Ala Ala Leu Ser Pro Leu Pro Glu Val Pro Pro Trp Thr Gly Glu Val

                                        250
AGC CCA GCC CAG AGA GAT GGA GGT GCC CTC GGG CGG GGC CCC TGG GAC TCC TCT GAT CGA TCT GCC CTC CTA
Ser Pro Ala Gln Arg Asp Gly Gly Ala Leu Gly Arg Gly Pro Trp Asp Ser Ser Asp Arg Ser Ala Leu Leu
                                                100
        300                                                                 350
AAA AGC AAG CTG AGG GCG CTG CTC ACT GCC CCT CGG AGC CTG CGG AGA TCC AGC TGC TTC GGG.GGC AGG ATG
Lys Ser Lys Leu Arg Ala Leu Leu Thr Ala Pro Arg Ser Leu Arg Arg Ser Ser Cys Phe Gly Gly Arg Met

                                                400         127
GAC AGG ATT GGA GCC CAG AGC GGA CTG GGC TGT AAC AGC TTC CGG TAC TGA agataacagc caggggaggac
Asp Arg Ile Gly Ala Gln Ser Gly Leu Gly Cys Asn Ser Phe Arg Tyr

                 450                                        151        500
aagcagggct gggcctaggg acagactgca agaggctcct gtcccctggg gtctctgctg catttgtgtc atcttgttgc catggagttg

                          550                                                    600
tgatcatccc atctaagctg cagcttcctg tcaacacttc tcacatctta tgctaactgt agataaagtg gtttgatggt gacttcctcg

                                      650                                                700
cctctcccac cccatgcatt aaattttaag gtagaacctc acctgttact gaaagtggtt tgaaagtgaa taaacttcag caccatggac
```

FIG.__Ib.

10 20 30 40 50 60 70
GAGCAAGAAG CCCTTGGTGA TGGAGAGAAA CCAGAGAGTG AGCCGAGACA GCAAACATCA GATCGTGCCC

80 99 114
CGACCCACGC CAGC ATG GGC TCC TTC TCC ATC ACC AAG GGC TTC TTC CTC TTC CTG
MET Gly Ser Phe Ser Ile Thr Lys Gly Phe Phe Leu Phe Leu
1

129 144 159 174
GCC TTT TGG CTC CCA GGC CAT ATT GGA GCA AAT CCC GTA TAC AGT GCG GTG TCC
Ala Phe Trp Leu Pro Gly His Ile Gly Ala Asn Pro Val Tyr Ser Ala Val Ser
25

189 204 219 234
AAC ACA GAT CTG ATG GAT TTC AAG AAC CTG CTA GAC CAC CTG GAG GAG AAG ATG
Asn Thr Asp Leu MET Asp Phe Lys Asn Leu Leu Asp His Leu Glu Glu Lys MET
50

249 264 279
CCG GTA GAA GAT GAG GTC ATG CCT CCG CAG GCC CTG AGC GAG CAG ACC GAT GAA
Pro Val Glu Asp Glu Val MET Pro Pro Gln Ala Leu Ser Glu Gln Thr Asp Glu

294 309 324 339
GCG GGG GCG GCA CTT AGC TCC CTC TCT GAG GTG CCT CCC TGG ACT GGG GAA GTC
Ala Gly Ala Ala Leu Ser Ser Leu Ser Glu Val Pro Pro Trp Thr Gly Glu Val

354 369 384
AAC CCG TCT CAG AGA GAT GGA GGT GCT CTC GGG CGC GGC CCC TGG GAC CCC TCC
Asn Pro Ser Gln Arg Asp Gly Gly Ala Leu Gly Arg Gly Pro Trp Asp Pro Ser
100

399 414 429 444
GAT AGA TCT GCC CTC TTG AAA AGC AAA CTG AGG GCT CTG CTC GCT GGC CCT CGG
Asp Arg Ser Ala Leu Leu Lys Ser Lys Leu Arg Ala Leu Leu Ala Gly Pro Arg

459 474 489 504
AGC CTG CGA AGG TCA AGC TGC TTC GGG GGT AGG ATT GAC AGG ATT GGA GCC CAG
Ser Leu Arg Arg Ser Ser Cys Phe Gly Gly Arg Ile Asp Arg Ile Gly Ala Gln

126 519 534 553 563
AGC GGA CTA GGC TGC AAC AGC TTC CGG TAC CGA AGA TAA CAGCCAAATC TGCTCGAGCA
Ser Gly Leu Gly Cys Asn Ser Phe Arg Tyr Arg Arg

573 583 593 150 603 152 613 623 633
GATCGCAAAA GATCCCAAGC CTTGCGGTGT GTCACACAGC TTGGTCGCAT TGCCACTGAG AGGTGGTGAA

643 653 663 673 683 693 703
TACCCTCCTG GAGCTGCAGC TTCCTGTCTT CATCTATCAC GATCGATGTT AAGTGTAGAT GAGTGGTTTA

713 723 733 743 753 763 773
GTGAGGCCTT ACCTCTCCCA CTCTGCATAT TAAGGTAGAT CCTCACCCCT TTCAGAAAGC AGTTGGAAAA

783 793 803
AAATAAATCC GAATAAACTT CAGCACCACG GAC

FIG._2.

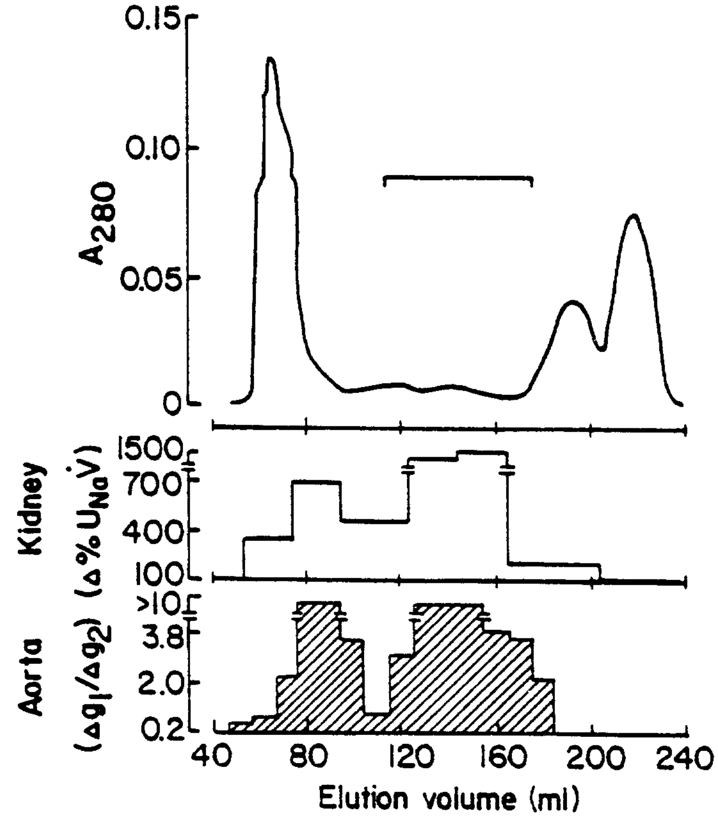
0.15
0.10
A280
0.05
0
Kidney
(Δ% U_Na V̇)
1500
700
400
100
Aorta
(Δg1/Δg2)
>10
3.8
2.0
0.2
40
80
120
160
200
240
Elution volume (ml)

FIG._3A.

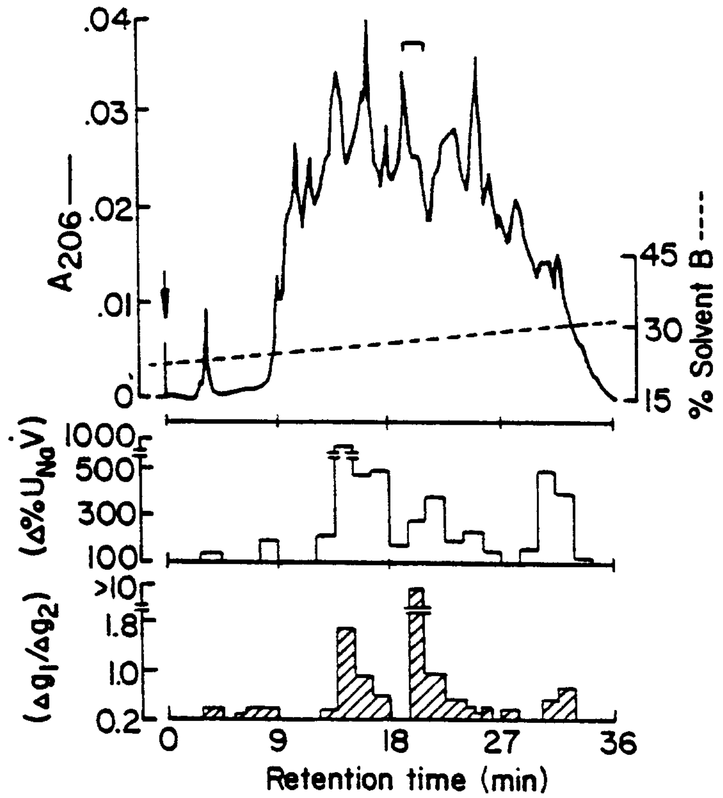
.04
.03
A206
.02
.01
0
45
30
15
% Solvent B
(Δ% U_Na V̇)
1000
500
300
100
(Δg1/Δg2)
>10
1.8
1.0
0.2
0
9
18
27
36
Retention time (min)

FIG._3B.

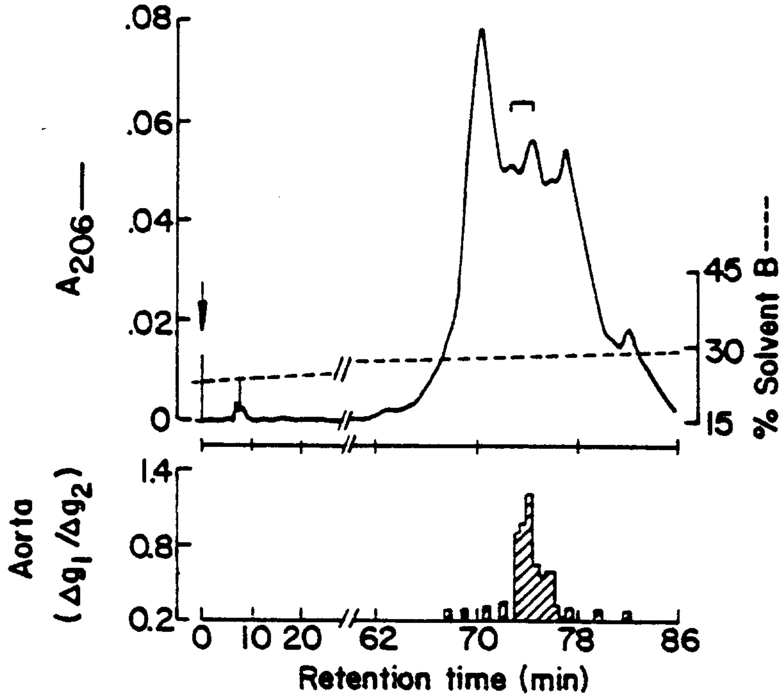
.08
.06
.04
.02
0
A206
45
30
15
% Solvent B
1.4
0.8
0.2
Aorta
(Δg1/Δg2)
0
10
20
62
70
78
86
Retention time (min)

FIG._3C.

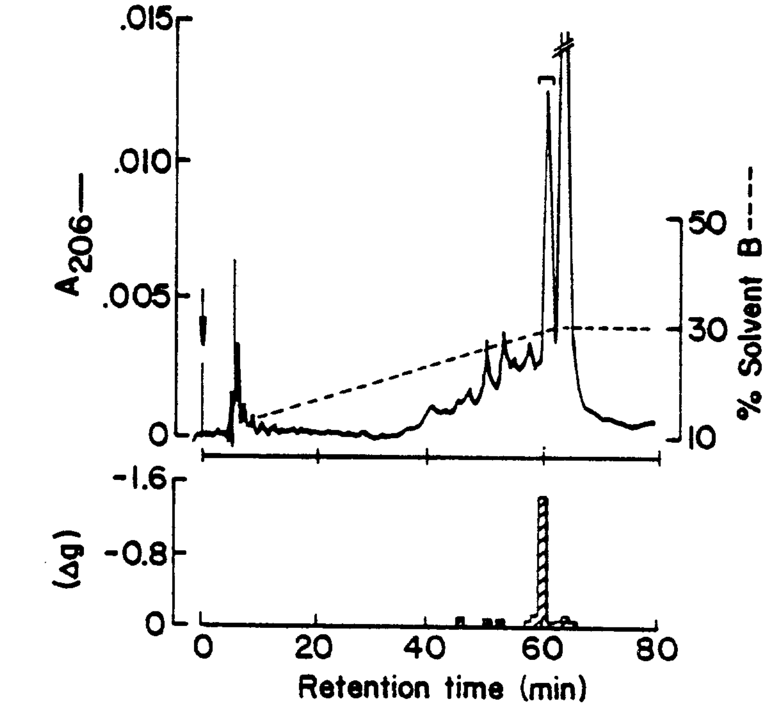
.015
.010
.005
0
A206
50
30
10
% Solvent B
-1.6
-0.8
0
(Δg)
0
20
40
60
80
Retention time (min)

FIG._3D.

| | | 4 | 5 | 6 | 7 | 8 | 9 | 10 | 11 | 12 | 13 | |
|---|---|---|---|---|---|---|---|---|---|---|---|---|
| Amino Acids | | Cys | Phe | Gly | Gly | Arg | Ile | Asp | Arg | Ile | Gly | |
| Possible Codons | 5' | TGY | TTY | GGN | GGN | CGN | ATY | GAY | CGN | ATY | GGN | 3' |
| | | | | | | AGR | ATA | | AGR | ATA | | |

REGION 1
probe a 3' ACR AAR CCN CCN GC 5'
probe b 3' ACR AAR CCN CCN TC 3'

REGION 2
probe c 3' TA$_{T}^{R}$ CTR GCN TA$_{T}^{R}$ CC 5'
probe d 3' TA$_{T}^{R}$ CTR TCN TA$_{T}^{R}$ CC 5'

FIG.—4.

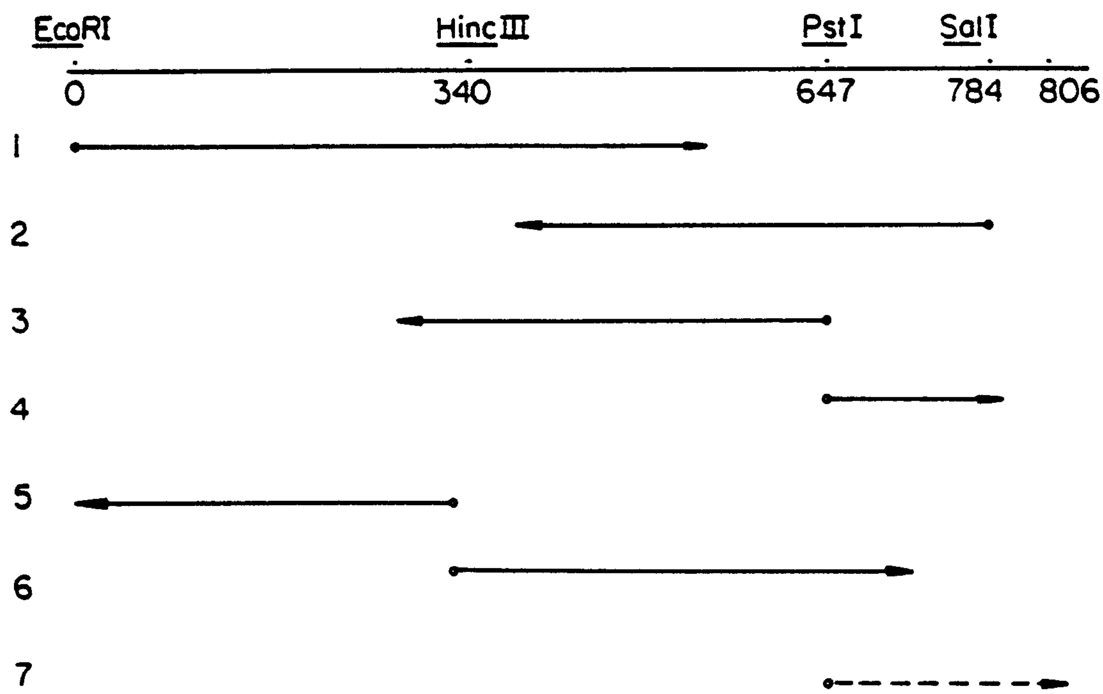


FIG.—5.

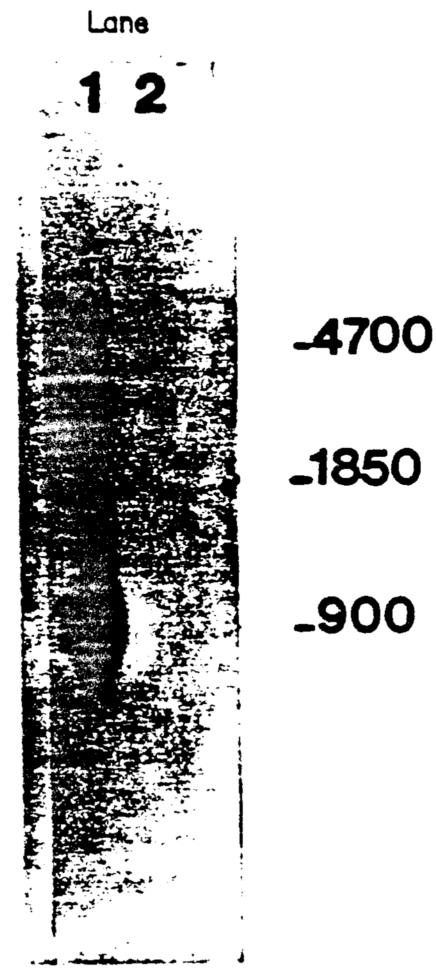


FIG.—6A.

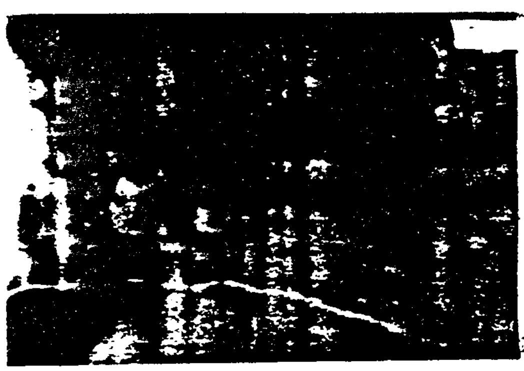

FIG.—6B.

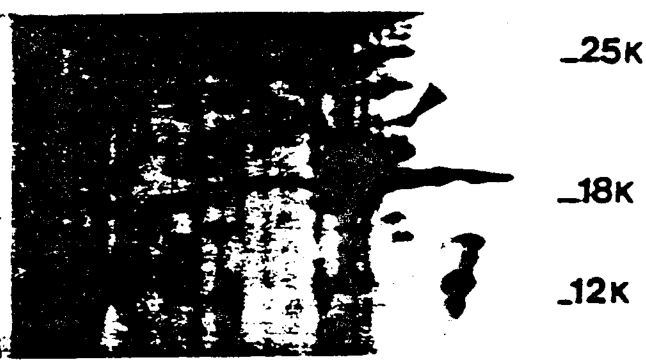


FIG.—6C.

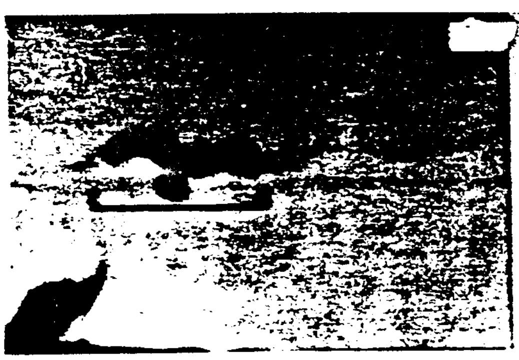

FIG.—6D.

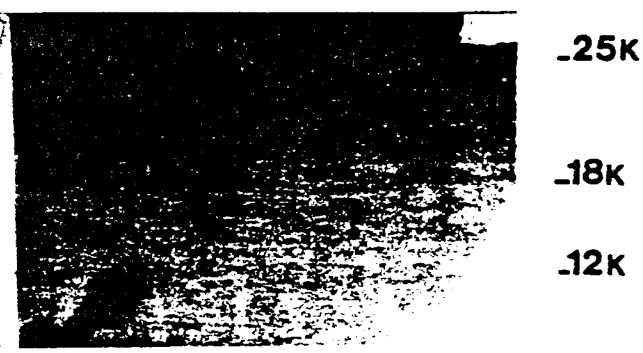


FIG.—6E.

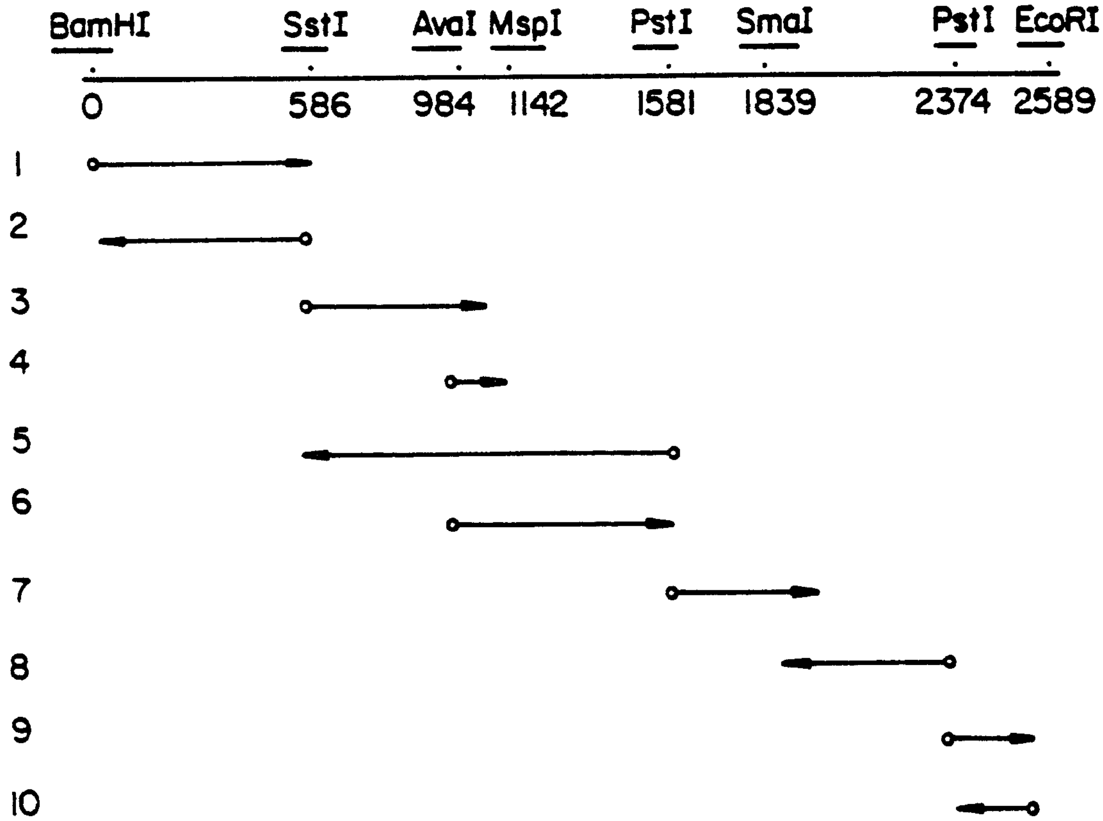
BamHI
SstI
AvaI
MspI
PstI
SmaI
PstI
EcoRI
0
586
984
1142
1581
1839
2374
2589
1
2
3
4
5
6
7
8
9
10

FIG._7.

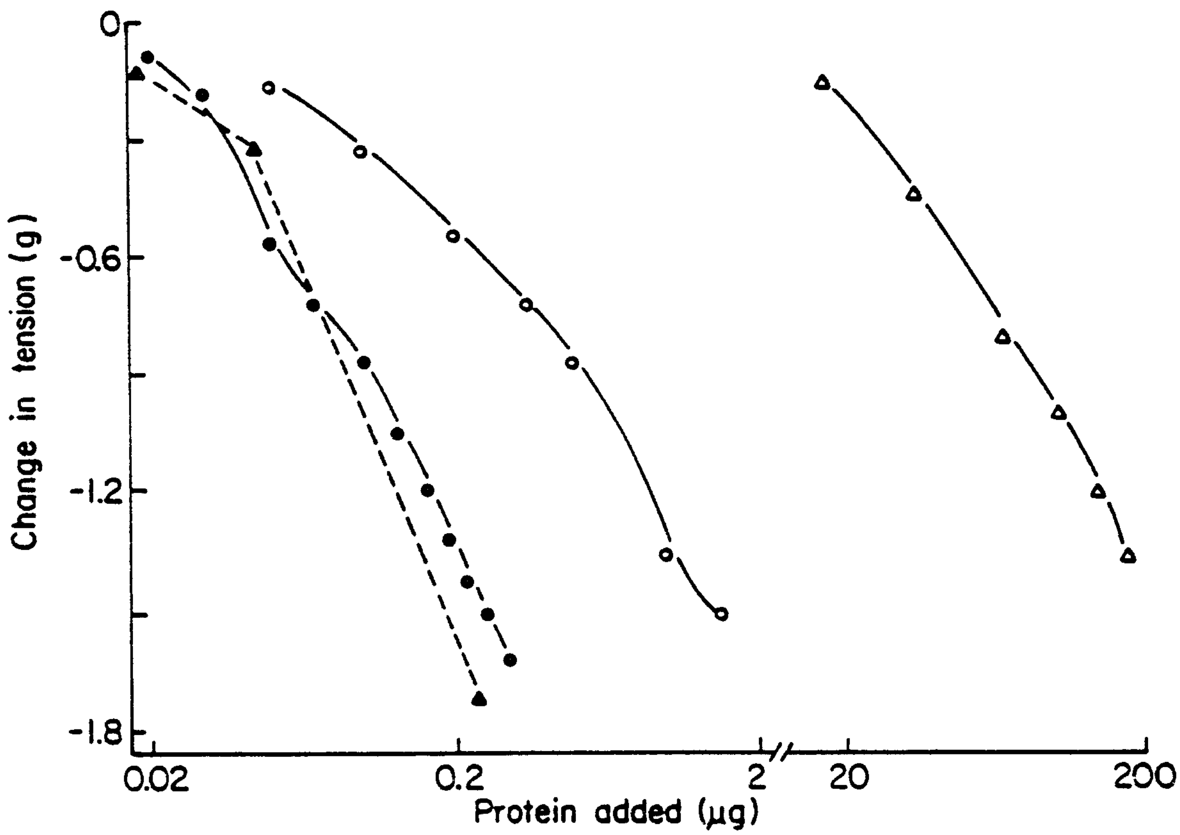
0
-0.6
-1.2
-1.8
Change in tension (g)
0.02
0.2
2
20
200
Protein added (μg)

FIG._8.

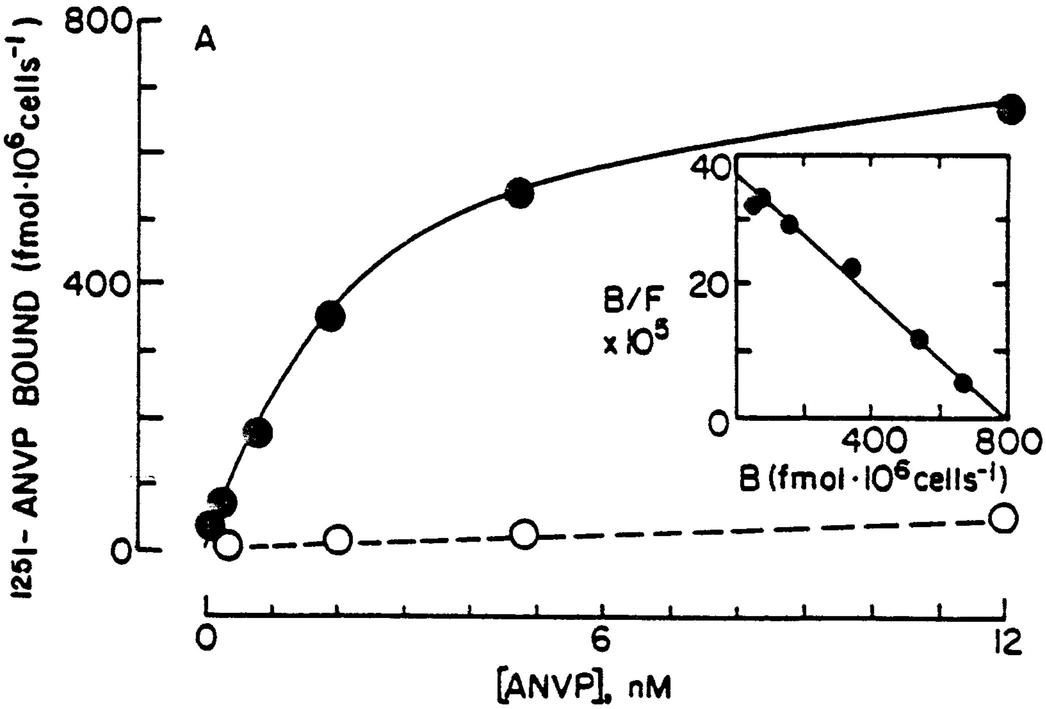
A
125I- ANVP BOUND (fmol·10^6 cells^-1)
800
400
0
B/F x 10^5
40
20
0
400
800
B (fmol·10^6 cells^-1)
0
6
12
[ANVP], nM

FIG._9A.

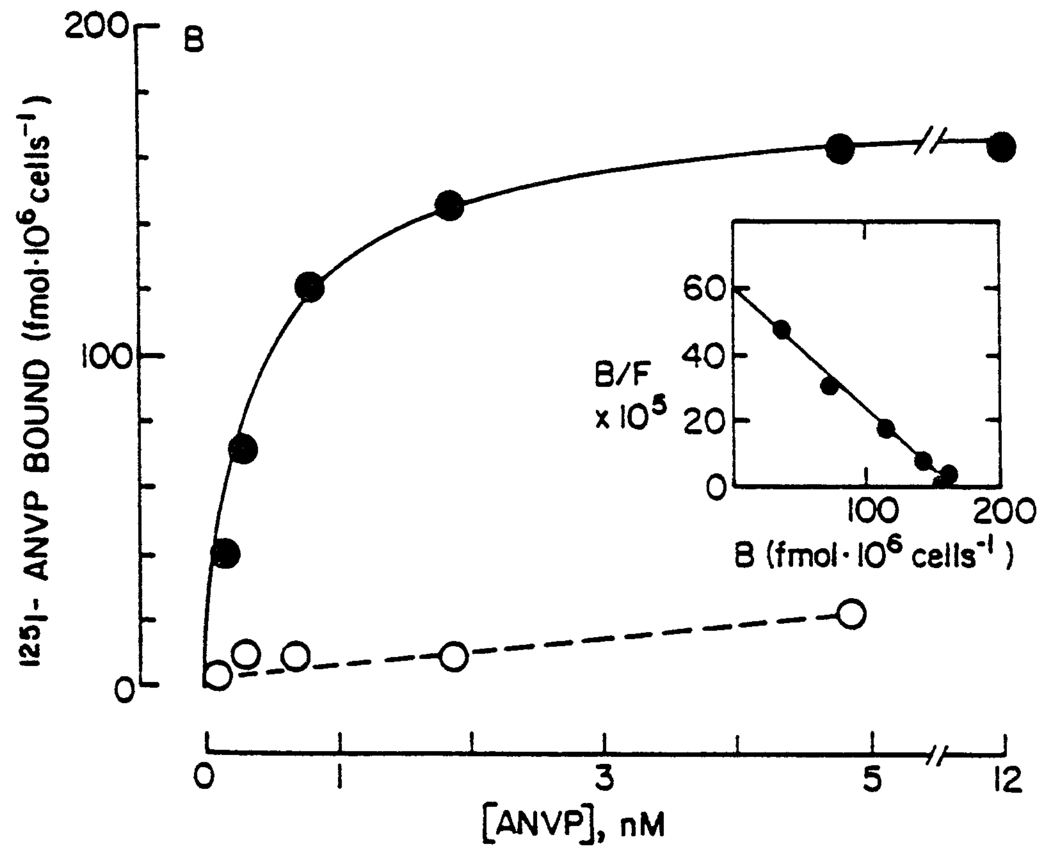
B
125I- ANVP BOUND (fmol·10^6 cells^-1)
200
100
0
B/F x 10^5
60
40
20
0
100
200
B (fmol·10^6 cells^-1)
0
1
3
5
12
[ANVP], nM

FIG._9B.

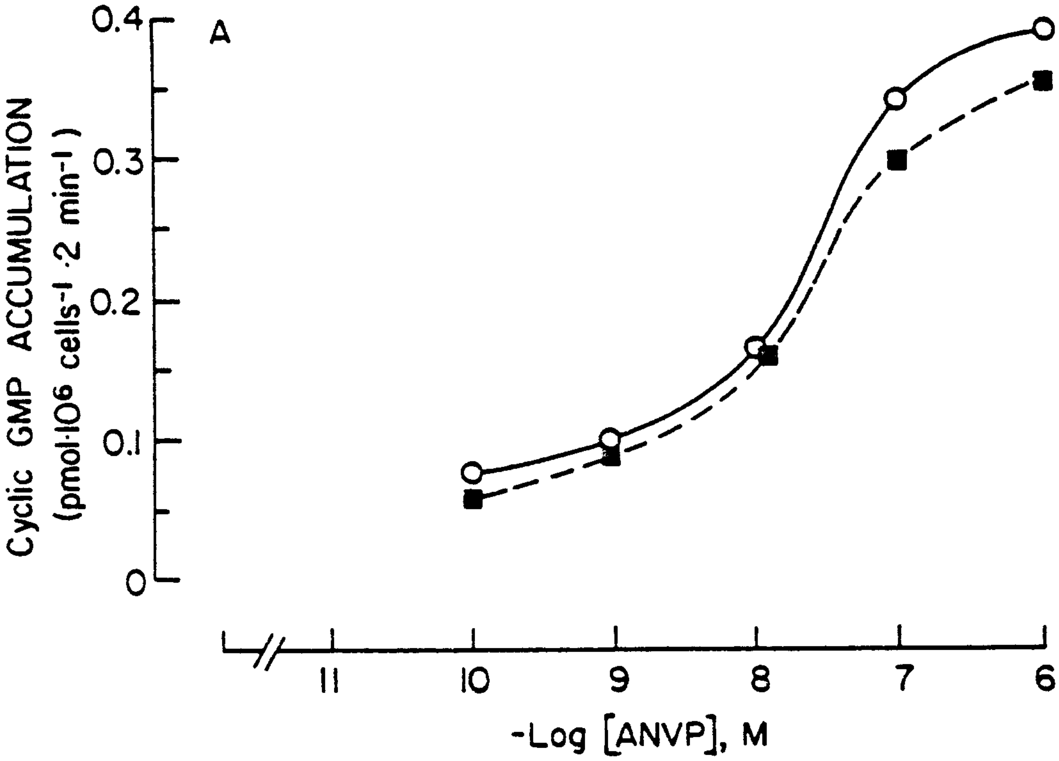


FIG._10A.

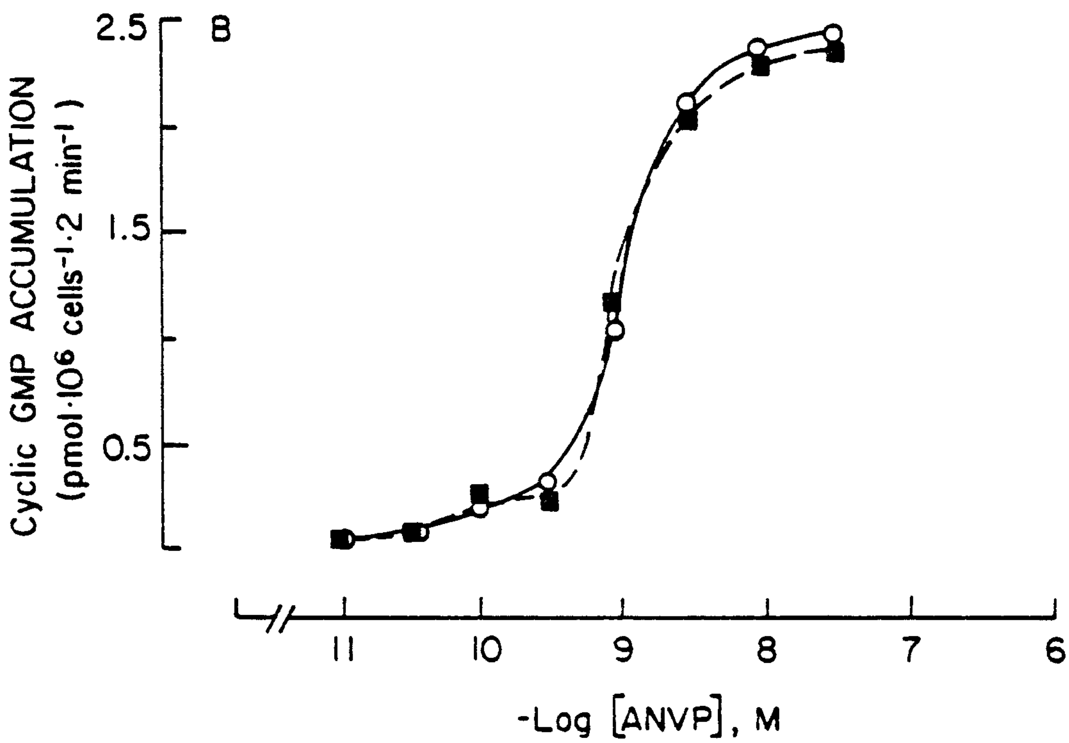


FIG._10B.

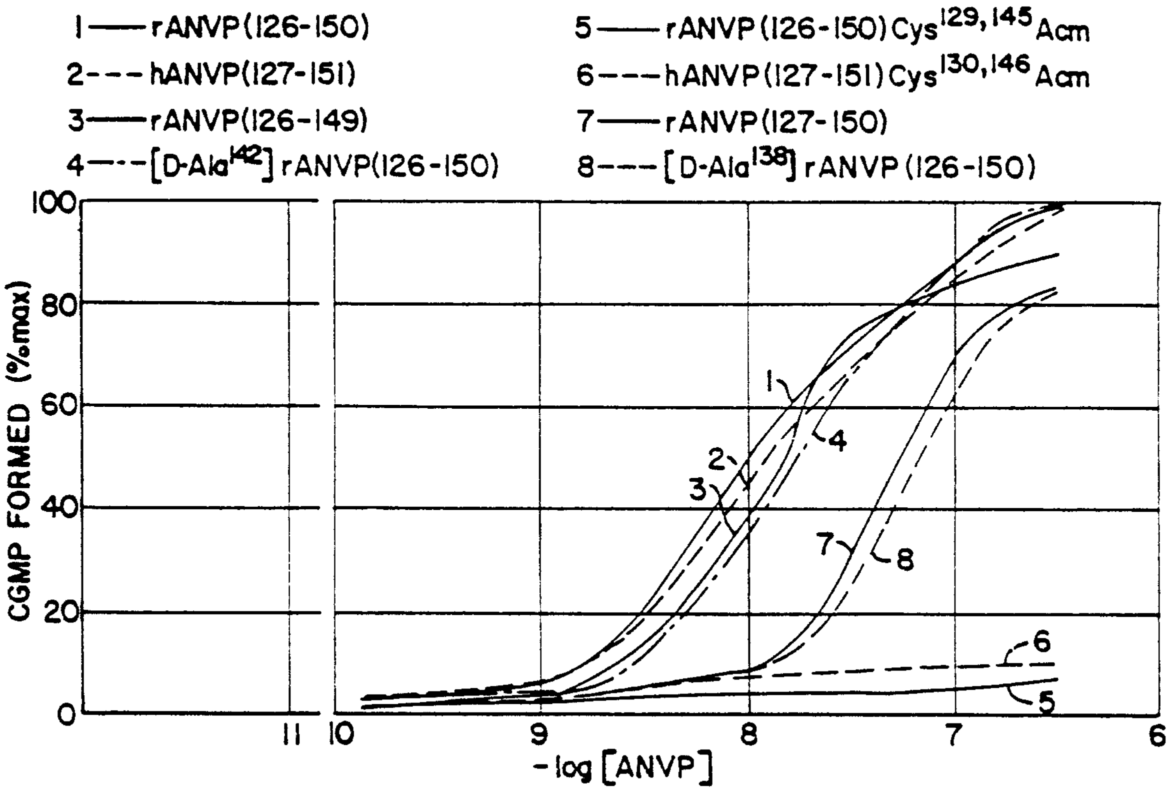


FIG._11A.

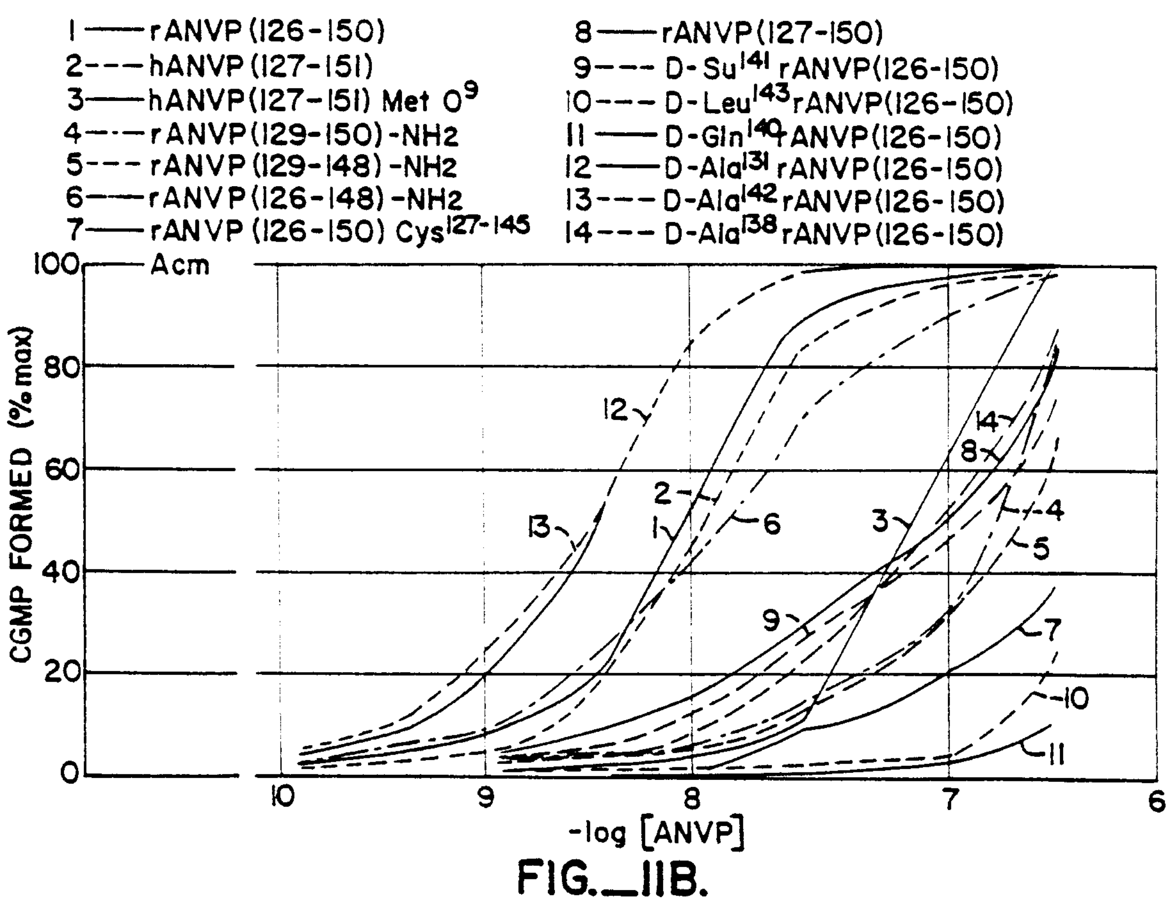


FIG._11B.

FIG._12A.

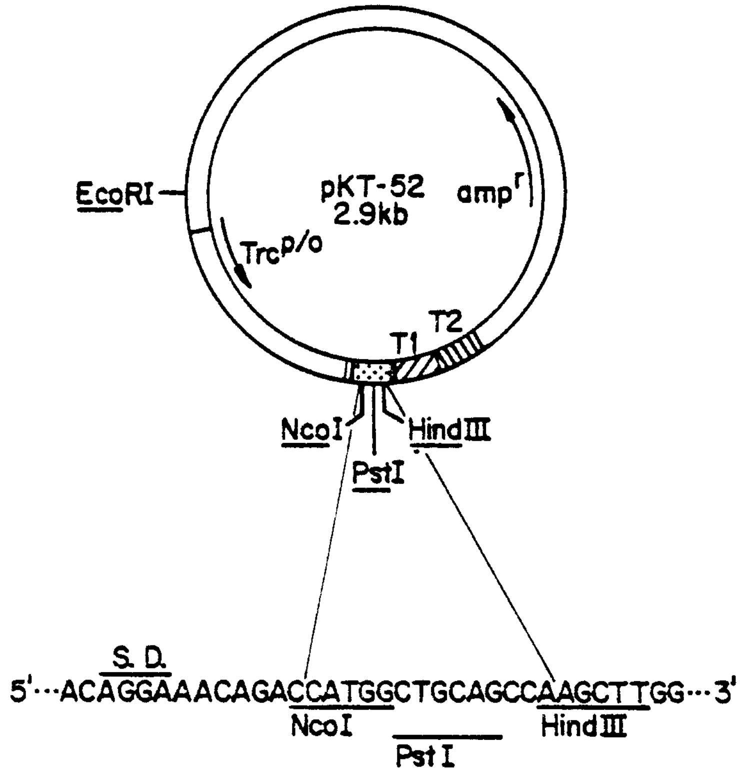


FIG._12B.

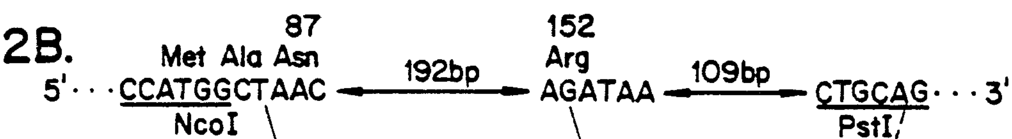


FIG._12C.

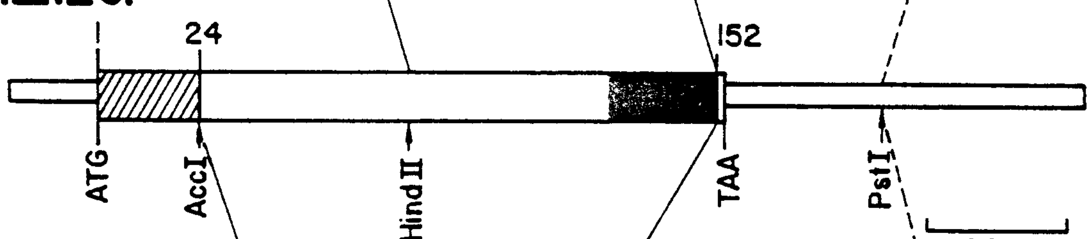


FIG._12D.

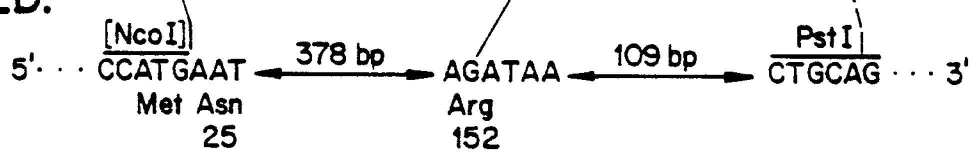

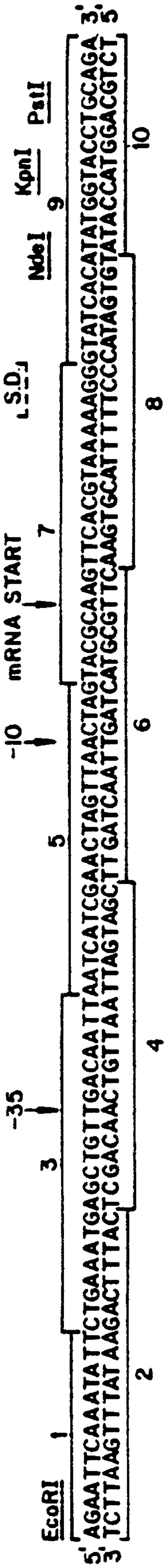


FIG.__12E.

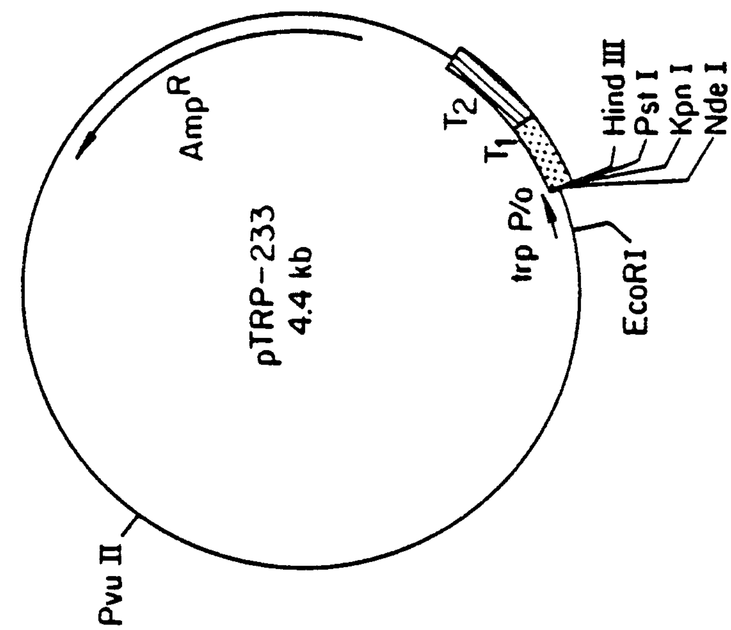


FIG.__12F.

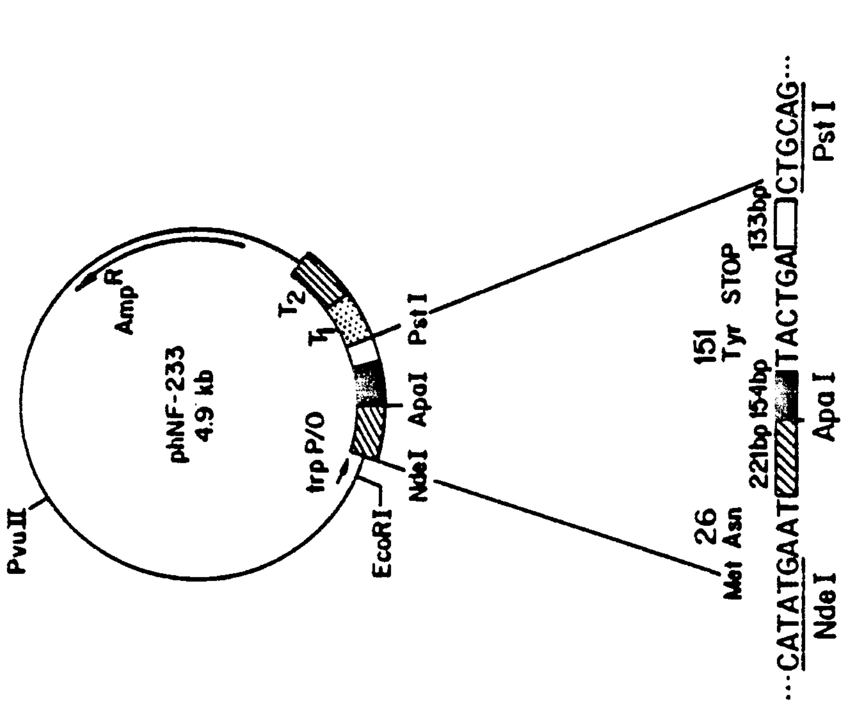


FIG.__12G.

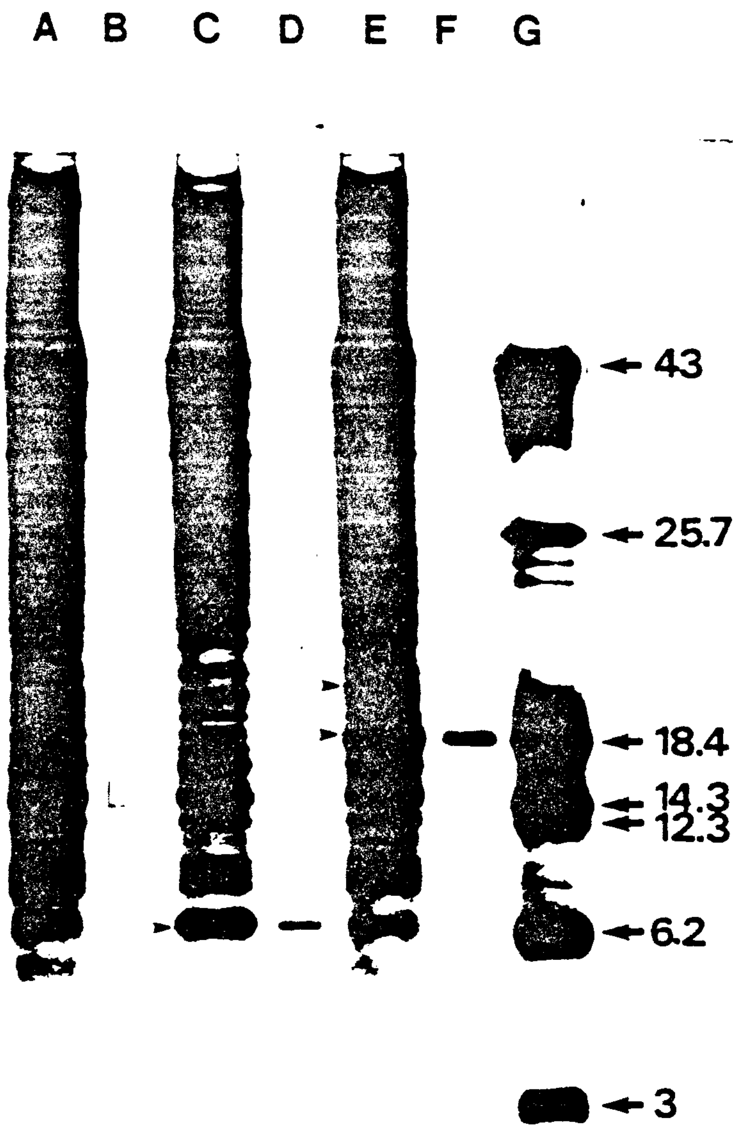


FIG._13.

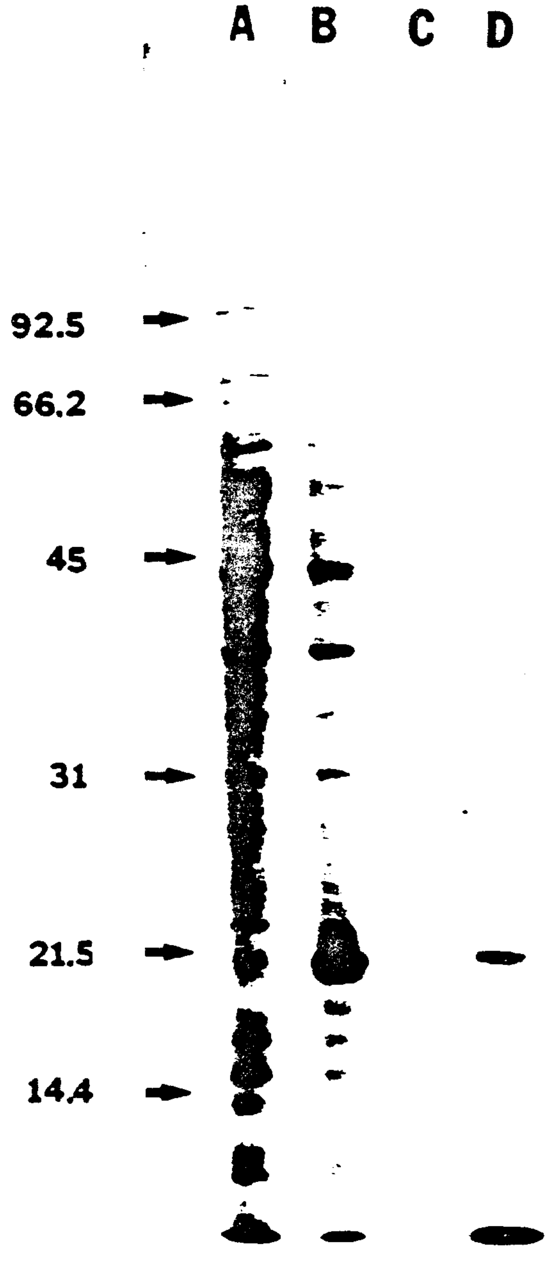


FIG._14.

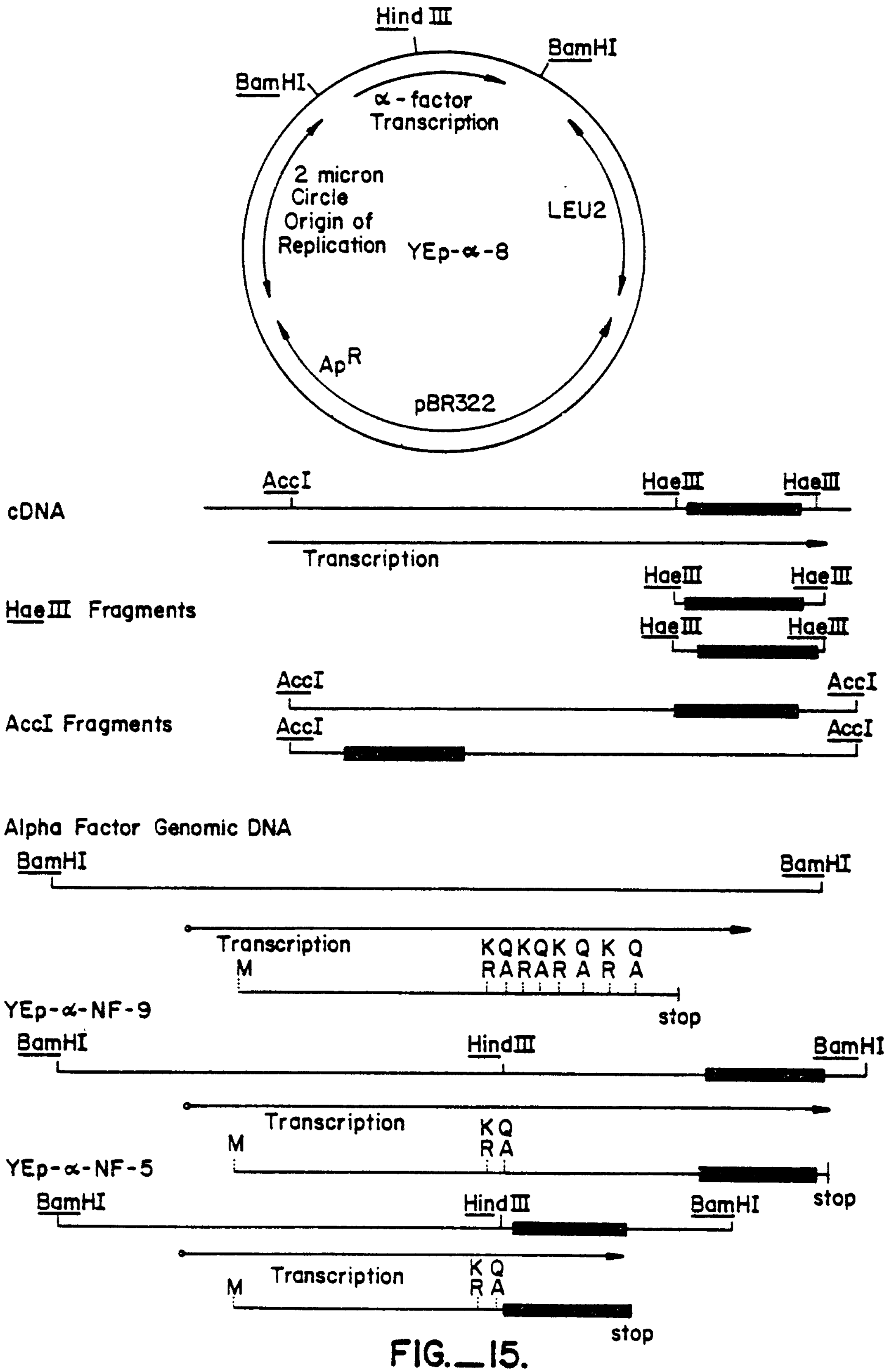
Hind III
BamHI
BamHI
α - factor Transcription
2 micron Circle Origin of Replication
LEU2
YEp-α-8
ApR
pBR322
AccI
HaeIII
HaeIII
cDNA
Transcription
HaeIII
HaeIII
HaeIII Fragments
HaeIII
HaeIII
AccI
AccI
AccI Fragments
AccI
AccI
Alpha Factor Genomic DNA
BamHI
BamHI
Transcription
KQKQK Q K Q
M
RARAR A R A
stop
YEp-α-NF-9
BamHI
HindIII
BamHI
Transcription
KQ
M
RA
YEp-α-NF-5
stop
BamHI
HindIII
BamHI
Transcription
KQ
M
RA
stop

FIG._15.

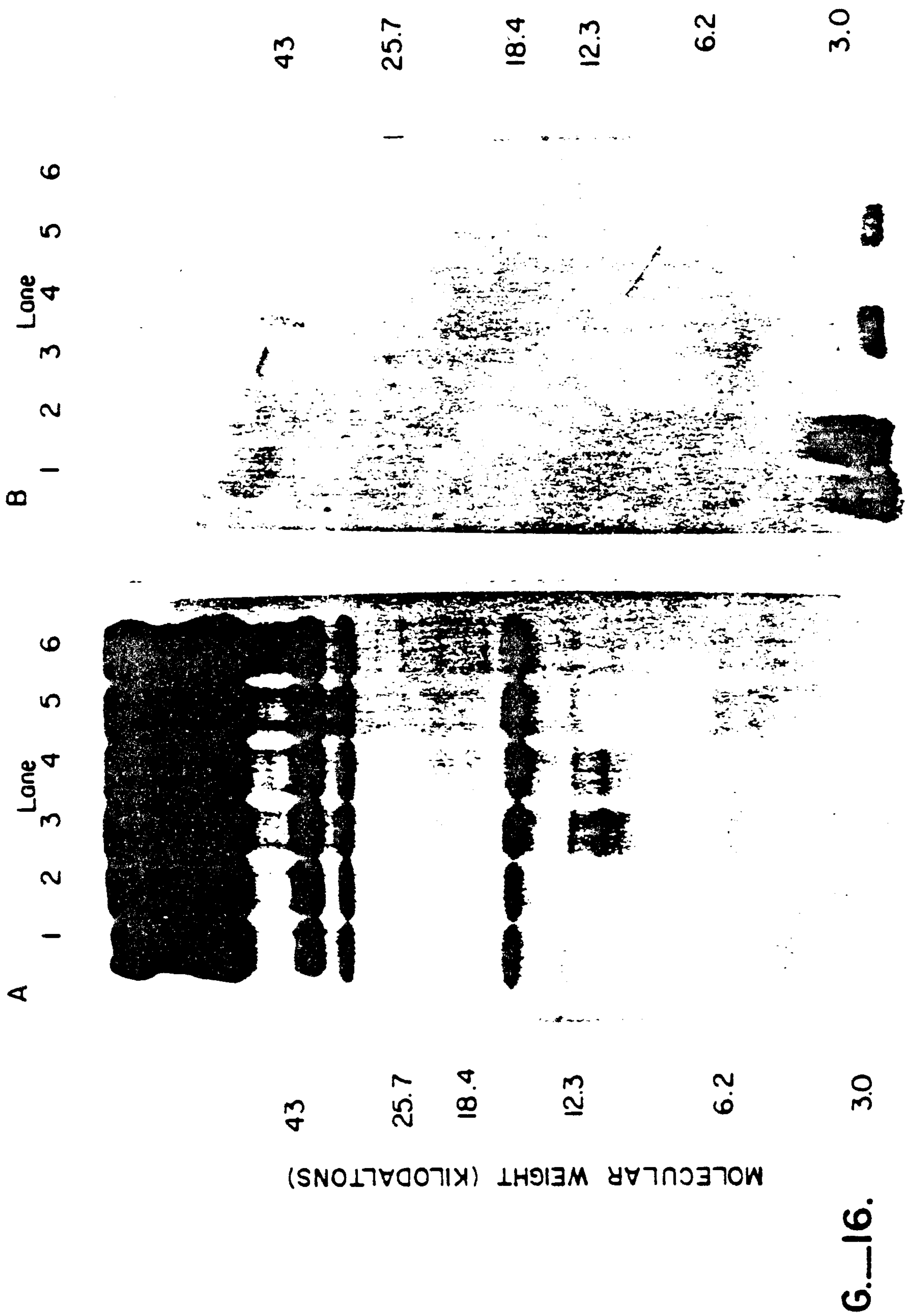
A
B
Lane
1 2 3 4 5 6
Lane
1 2 3 4 5 6
43
25.7
18.4
12.3
6.2
3.0
43
25.7
18.4
12.3
6.2
3.0
MOLECULAR WEIGHT (KILODALTONS)

FIG._16.

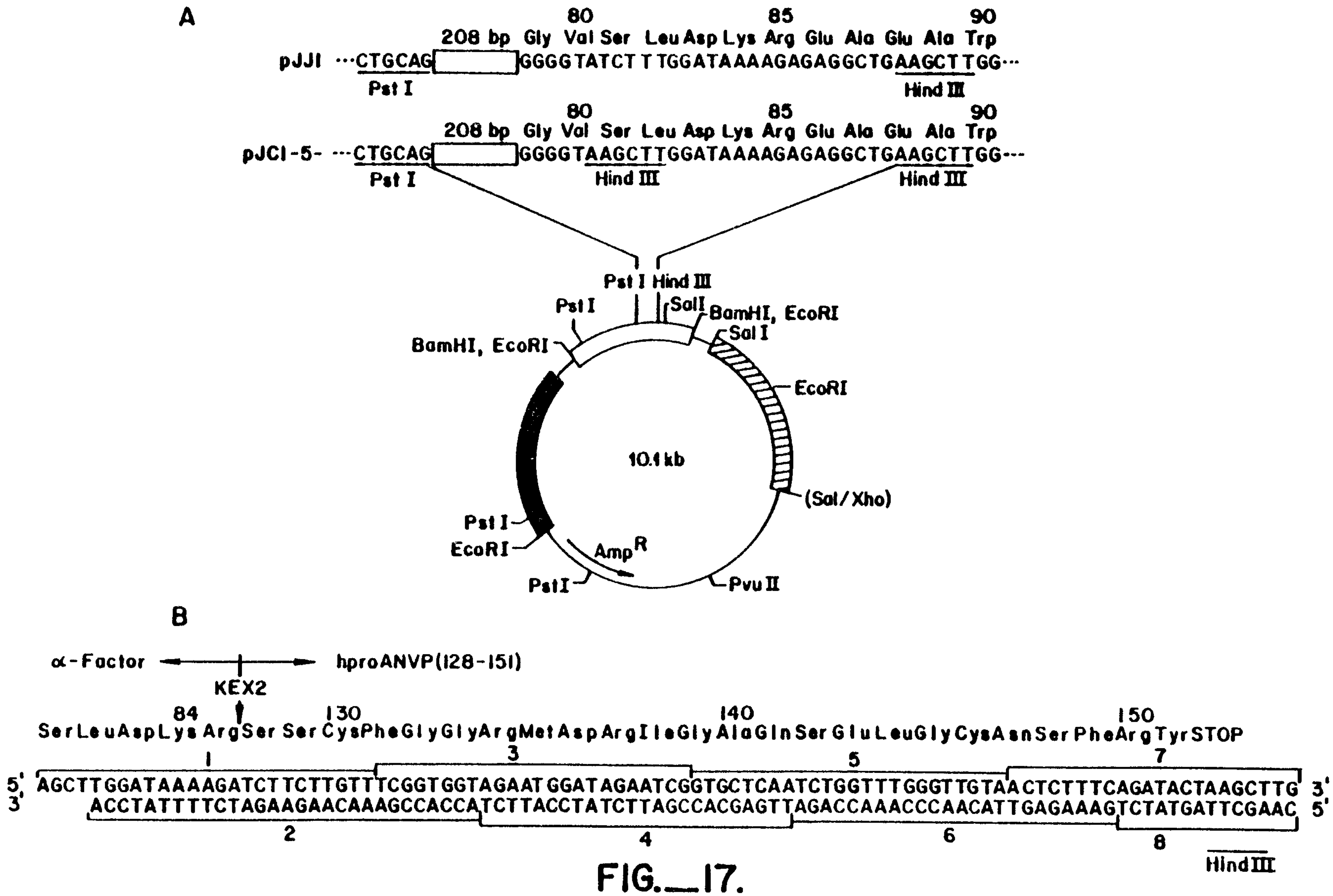
A
80
85
90
208 bp Gly Val Ser Leu Asp Lys Arg Glu Ala Glu Ala Trp
pJJ1 ···CTGCAG GGGGTATCTTTGGATAAAAGAGAGGCTGAAGCTTGG···
Pst I
Hind III
80
85
90
208 bp Gly Val Ser Leu Asp Lys Arg Glu Ala Glu Ala Trp
pJCI-5- ···CTGCAG GGGGTAAGCTTGGATAAAAGAGAGGCTGAAGCTTGG···
Pst I
Hind III
Hind III
Pst I Hind III
Pst I
Sal I
BamHI, EcoRI
BamHI, EcoRI
Sal I
EcoRI
10.1 kb
(Sal/Xho)
Pst I
EcoRI
Amp^R
Pst I
Pvu II
B
α-Factor
hproANVP(128-151)
KEX2
84
130
140
150
SerLeuAspLysArgSer SerCysPheGlyGlyArgMetAspArgIleGlyAlaGlnSerGluLeuGlyCysAsnSerPheArgTyrSTOP
1
3
5
7
5' AGCTTGGATAAAAGATCTTCTTGTTTCGGTGGTAGAATGGATAGAATCGGTGCTCAATCTGGTTTGGGTTGTAACTCTTTCAGATACTAAGCTTG 3'
3' ACCTATTTTCTAGAAGAACAAAGCCACCATCTTACCTATCTTAGCCACGAGTTAGACCAAACCCAACATTGAGAAAGTCTATGATTCGAAC 5'
2
4
6
8
HindIII

FIG._17.

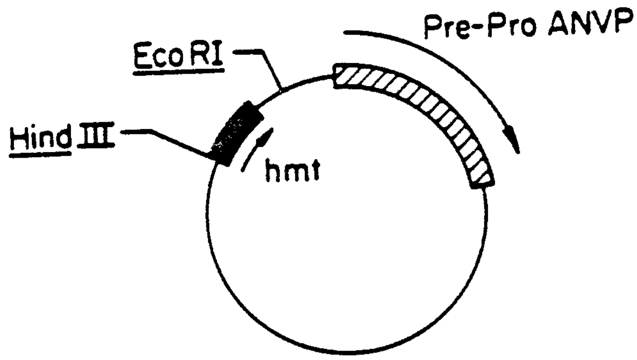


FIG._18A.

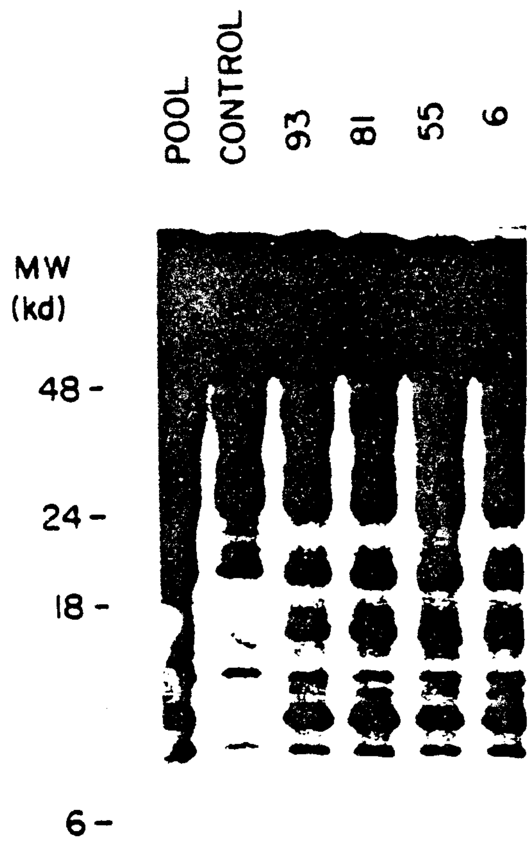


FIG._18B.